(19) Europäisches Patentamt / European Patent Office / Office européen des brevets

(11) **EP 3 141 300 A1**

(12) **EUROPEAN PATENT APPLICATION**
published in accordance with Art. 153(4) EPC

(43) Date of publication:
**15.03.2017 Bulletin 2017/11**

(51) Int Cl.:
**B01J 19/00** (2006.01)          **C12M 1/26** (2006.01)
**C12M 1/34** (2006.01)          **G01N 15/00** (2006.01)
**G01N 15/14** (2006.01)

(21) Application number: **15788799.3**

(22) Date of filing: **08.05.2015**

(86) International application number:
**PCT/JP2015/063364**

(87) International publication number:
**WO 2015/170758 (12.11.2015 Gazette 2015/45)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(30) Priority: **08.05.2014 JP 2014097055**

(71) Applicants:
• **Osaka Prefecture University Public Corporation
Osaka 599-8531 (JP)**
• **Sharp Kabushiki Kaisha
Sakai City, Osaka 590-8522 (JP)**

(72) Inventors:
• **IIDA, Takuya
Sakai-shi
Osaka 599-8531 (JP)**

• **TOKONAMI, Shiho
Sakai-shi
Osaka 599-8531 (JP)**
• **NAKASE, Ikuhiko
Sakai-shi
Osaka 599-8531 (JP)**
• **NISHIMURA, Yushi
Sakai-shi
Osaka 599-8531 (JP)**
• **YAMAMOTO, Yasuyuki
Sakai-shi
Osaka 599-8531 (JP)**

(74) Representative: **Müller Hoffmann & Partner
Patentanwälte mbB
St.-Martin-Strasse 58
81541 München (DE)**

(54) **ACCUMULATION DEVICE AND ACCUMULATION METHOD, MANUFACTURING DEVICE FOR MICROSCOPIC OBJECT ACCUMULATION STRUCTURAL BODY, MICROSCOPIC ORGANISM ACCUMULATION AND ELIMINATION DEVICE, DETECTION-SUBSTANCE DETECTION DEVICE, SEPARATION-SUBSTANCE SEPARATION DEVICE, AND INTRODUCTION-SUBSTANCE INTRODUCTION DEVICE**

(57)      An assembling apparatus (100) assembles beads (1,2) different in particle size from each other. The assembling apparatus (100) includes a substrate (10) and a photothermal light source (20). The substrate is constructed to be able to hold a sample (13) in which the beads (1, 2) are dispersed. The photothermal light source (20) irradiates the substrate (10) or the sample (13) with laser beams (201) to thereby produce a temperature difference in the sample (13).

FIG.1

EP 3 141 300 A1

## EP 3 141 300 A1

**Description**

TECHNICAL FIELD

The present invention relates to an assembling apparatus and an assembling method, an apparatus for manufacturing a microscopic object assembly structure, an apparatus for assembling and removing a microorganism, an apparatus for detecting a detection target substance, an apparatus for separating a separation target substance, and an apparatus for introducing an introduction target substance. More particularly, the present invention relates to an assembling apparatus for assembling a plurality of types of microscopic objects, an apparatus for manufacturing a microscopic object assembly structure, an apparatus for assembling and removing a microorganism, an apparatus for detecting a detection target substance, an apparatus for separating a separation target substance, and an apparatus for introducing an introduction target substance which include the assembling apparatus, as well as an assembling method for assembling a plurality of types of microscopic objects.

BACKGROUND ART

A technology for assembling microparticles at an aimed location by irradiation with light has recently been proposed. For example, WO2006/104048 (PTD 1) discloses a method of arranging microparticles in a liquid phase. A source substrate in which microparticles are fixed to a microparticle fixation surface and a target substrate having a microparticle arrangement surface are prepared. The source substrate and the target substrate are arranged with a liquid phase being interposed while the microparticle fixation surface and the microparticle arrangement surface face each other. In this state, at least one of physical force and chemical force induced by irradiation with laser separates microparticles from the microparticle fixation surface. The separated microparticles fall down through the liquid phase under the gravity and are arranged on the microparticle arrangement surface.

CITATION LIST

PATENT DOCUMENT

PTD 1: WO2006/104048

SUMMARY OF INVENTION

TECHNICAL PROBLEM

The arrangement method disclosed in WO2006/104048 (PTD 1) is a technology in which microparticles fixed to the microparticle fixation surface of the source substrate are irradiated with light. From a point of view of expansion of the scope of application of the technology to assemble a microscopic object by irradiation with light, a technology allowing assembly of a microscopic object dispersed in a liquid or a gas is demanded.
The present invention was made to solve the problem above, and an object thereof is to provide a technology allowing assembly of a microscopic object dispersed in a liquid or a gas with irradiation with light.

SOLUTION TO PROBLEM

An assembling apparatus according to one aspect of the present invention assembles a plurality of types of microscopic objects different in at least one of physical characteristics, chemical characteristics, and biological characteristics. The assembling apparatus includes a holding member and a light source. The holding member is configured to hold a space in which the plurality of types of microscopic objects are dispersed. The light source irradiates the holding member or the space with light and produces a temperature difference in the space.
Preferably, the space contains a liquid in which the plurality of types of microscopic objects are dispersible. The holding member includes a substrate configured to hold a liquid in which the plurality of types of microscopic objects are dispersed.
Preferably, the substrate includes a holding region for holding the liquid. The holding region is formed of a material which converts light from the light source into heat.
Preferably, the holding region includes a film formed through vapor deposition or sputtering. Preferably, the holding region includes a film formed through self-assembly of a plurality of metallic nanoparticles.
Preferably, the light source emits laser beams at a wavelength included in a light absorption region of the material.
Preferably, the holding region has a thickness along a direction of emission of the laser beams. The thickness is determined based on intensity of the laser beams and light absorbency of the material.

Preferably, the light source heats the holding region by irradiating the holding region with light so as to produce the temperature difference to thereby produce convection and a bubble in the vicinity of the holding region.

Preferably, the plurality of types of microscopic objects include a plurality of first particles and a plurality of second particles different in particle size from each other. At least one of the plurality of first particles and the plurality of second particles includes a plurality of photothermal conversion particles each of which converts light from the light source into heat. The light source assembles the plurality of first particles and the plurality of second particles at (at least one of) a position of irradiation with light (and a portion around the same) in the liquid by irradiating the liquid with light so as to have the plurality of photothermal conversion particles generate heat at the position of irradiation, produce the temperature difference and convection in the liquid, and move the plurality of first particles and the plurality of second particles over the convection.

Preferably, the assembling apparatus further includes an objective lens. The objective lens gathers light from the light source and introduces gathered light into the holding member or the space.

Preferably, the assembling apparatus further includes an adjustment mechanism. The adjustment mechanism adjusts a distance between the objective lens and a gas-liquid interface which is an interface between a gas around the liquid and the liquid such that a focal point of the objective lens is located in the liquid and located in the vicinity of the gas-liquid interface.

Preferably, the plurality of types of microscopic objects include two or more types of microscopic objects different in at least one of a particle size and a shape representing the physical characteristics.

With an assembling method according to another aspect of the present invention, a plurality of types of microscopic objects different in at least one of physical characteristics, chemical characteristics, and biological characteristics are assembled. The assembling method includes dispersing the plurality of types of microscopic objects in a space defined by a holding member and irradiating the holding member or the space with light for producing a temperature difference in the space.

Preferably, the space contains a liquid in which the plurality of types of microscopic objects are dispersible. The holding member includes a substrate configured to hold the liquid. The dispersing the plurality of types of microscopic objects includes preparing the liquid in which the plurality of types of microscopic objects are dispersed prior to the irradiating the holding member or the space.

Preferably, the substrate includes a holding region in which the liquid is held. In the irradiating the holding member or the space, light with which the holding region is irradiated is converted to heat.

Preferably, in the irradiating the holding member or the space, laser beams at a wavelength included in a light absorption region of a material for the holding region are emitted.

Preferably, in the irradiating the holding member or the space, the holding region is heated by irradiation of the holding region with light such that convection and bubble is produced in the vicinity of the holding region.

Preferably, the assembling method further includes vacuum drying the holding region after heating the holding region.

Preferably, the plurality of types of microscopic objects include a plurality of first particles and a plurality of second particles different in particle size from each other. At least one of the plurality of first particles and the plurality of second particles includes a plurality of photothermal conversion particles each of which converts light from the light source into heat. In the irradiating the holding member or the space, the plurality of photothermal conversion particles produce convection in the liquid by generating heat at a position of irradiation with light in the liquid so as to produce the temperature difference in the liquid. The plurality of first particles and the plurality of second particles are assembled at (at least one of) the position of irradiation (and a portion around the same) by being moved over the convection.

Preferably, the plurality of types of microscopic objects include two or more types of microscopic objects different in at least one of a particle size and a shape representing the physical characteristics.

An apparatus for manufacturing a microscopic object assembly structure according to yet another aspect of the present invention includes the assembling apparatus described above.

An apparatus for assembling and removing a microorganism according to yet another aspect of the present invention includes the assembling apparatus described above. The plurality of types of microscopic objects include a microorganism. The light source heats the holding member by irradiating the holding member or the space with light so as to remove the microorganism in the space.

A detection apparatus according to yet another aspect of the present invention detects a detection target substance which may be included in a plurality of types of microscopic objects. The detection apparatus includes the assembling apparatus described above, an imaging device, and a detector. The imaging device obtains an image of the space irradiated with light from the light source. The detector detects the detection target substance based on an image obtained by the shooting instrument.

A separation apparatus according to yet another aspect of the present invention separates a separation target substance which may be included in a plurality of types of microscopic objects. The separation apparatus includes the assembling apparatus described above which assembles the plurality of types of microscopic objects so as to form an assembly, a spectroscopic light source, a spectroscope, and a separation portion. The spectroscopic light source emits light for measuring a spectrum of the assembly assembled by the assembling apparatus. The spectroscope measures a spectrum of the assembly. The separation portion separates the separation target substance based on a result of measurement of the spectrum by the spectroscope.

An apparatus for introducing an introduction target substance according to yet another aspect of the present invention includes the assembling apparatus described above. The plurality of types of microscopic objects include cells and an introduction target substance to be introduced into the cells by an uptake action of the cells. The light source promotes introduction of the introduction target substance into the cells by irradiating the holding member or the space with light so as to heat the holding member and assemble the introduction target substance.

Preferably, the apparatus for introducing an introduction target substance further includes cells adhering to the holding member.

ADVANTAGEOUS EFFECTS OF INVENTION

According to the present invention, a microscopic object dispersed in a liquid or a gas can be assembled through irradiation of light.

BRIEF DESCRIPTION OF DRAWINGS

Fig. 1 is a diagram showing a schematic construction of an assembling apparatus according to a first embodiment of the present invention.

Fig. 2 is an enlarged perspective view of a construction of the assembling apparatus shown in Fig. 1 around a substrate.

Fig. 3 is a diagram for illustrating beads dispersed in first to third samples.

Fig. 4 is a flowchart for illustrating a method of assembling beads according to the first embodiment of the present invention.

Fig. 5 shows successive photographs showing change over time of a region in the vicinity of a laser spot after start of irradiation with light in the third sample.

Fig. 6 is a schematic diagram for illustrating a mechanism of formation of an assembly of beads in the third sample.

Fig. 7 is a diagram for illustrating conditions for assembling beads in Figs. 8 to 17.

Fig. 8 is a diagram showing an SEM image of an assembly formed with a 100x lens and a result of determination of a cross-sectional shape of the assembly, in the first sample.

Fig. 9 is a diagram showing an SEM image of an assembly formed with a 100x lens and a result of determination of a cross-sectional shape of the assembly, in the second sample.

Fig. 10 is a diagram showing an SEM image of an assembly formed with a 100x lens and a result of determination of a cross-sectional shape of the assembly, in the third sample.

Fig. 11 is a diagram showing an SEM image of an assembly formed with a 10x lens and a result of determination of a cross-sectional shape of the assembly, in the first sample.

Fig. 12 is a diagram showing an SEM image of an assembly formed with a 10x lens and a result of determination of a cross-sectional shape of the assembly, in the second sample.

Fig. 13 is a diagram showing an SEM image of an assembly formed with a 10x lens and a result of determination of a cross-sectional shape of the assembly, in the third sample.

Fig. 14 is a diagram showing an SEM image of an assembly formed with a 100x lens and a three-dimensional shape of the assembly when a ratio between the number of beads 1 and the number of beads 2 is 1:43.

Fig. 15 is a diagram showing an SEM image of an assembly formed with a 100x lens and a three-dimensional shape of the assembly when a ratio between the number of beads 1 and the number of beads 2 is 1:9.

Fig. 16 is a diagram showing an SEM image of an assembly formed with a 10x lens and a three-dimensional shape of the assembly when a ratio between the number of beads 1 and the number of beads 2 is 1:43.

Fig. 17 is a diagram showing an SEM image of an assembly formed with a 10x lens and a three-dimensional shape of the assembly when a ratio between the number of beads 1 and the number of beads 2 is 1:9.

Fig. 18 is a diagram showing a schematic construction of an assembling apparatus according to a modification of the first embodiment of the present invention.

Fig. 19 shows optical photographs of assemblies at gas-liquid interfaces of respective fourth to sixth samples.

Fig. 20 shows an SEM image showing the assembly of the fourth sample as being enlarged.

Fig. 21 shows an SEM image of an assembly formed when the third sample is vacuum dried.

Fig. 22 is a diagram showing a three-dimensional shape and a cross-sectional shape of the assembly shown in Fig. 21.

Fig. 23 is a diagram showing an extinction spectrum when the third sample is naturally dried without irradiation with light and an extinction spectrum when the third sample is vacuum dried after irradiation with light.

Fig. 24 is a schematic diagram for comparison between a mechanism in naturally drying after irradiation with light and a mechanism in vacuum drying after irradiation with light.

Fig. 25 is a diagram showing a result of measurement of a diameter of a microbubble.

Fig. 26 shows an SEM image of an assembly formed in a region at an edge of a droplet of the first sample.

Fig. 27 shows an SEM image of an assembly formed in a region at an edge of a droplet of the second sample.

Fig. 28 shows an SEM image of an assembly formed in a region at an edge of a droplet of the third sample.

Fig. 29 is a diagram showing extinction spectra of the first to third samples.

Fig. 30 shows successive photographs showing change over time of a region in the vicinity of a laser spot after start of irradiation with light in a seventh sample.

Fig. 31 shows successive photographs for comparison of change after irradiation with light is stopped, between the third sample and the seventh sample.

Fig. 32 is a diagram showing an SEM image of an assembly formed with a 100x lens and a result of determination of a cross-sectional shape of the assembly, in the seventh sample.

Fig. 33 is a diagram showing an SEM image of an assembly formed with a 10x lens and a result of determination of a cross-sectional shape of the assembly, in the seventh sample.

Fig. 34 is a diagram showing a schematic construction of an apparatus for assembling and removing a microorganism according to a fourth embodiment of the present invention.

Fig. 35 is a diagram showing one example of a construction of a collection portion shown in Fig. 34.

Fig. 36 is a diagram showing a schematic construction of a detection apparatus according to a fifth embodiment of the present invention.

Fig. 37 is an enlarged perspective view of a construction of the detection apparatus shown in Fig. 36 around a substrate.

Fig. 38 is a flowchart for illustrating a method of detecting PM 2.5 according to the fifth embodiment of the present invention.

Fig. 39 is a diagram showing a schematic construction of a separation apparatus according to a sixth embodiment of the present invention.

Fig. 40 is a flowchart for illustrating a method of separating cells representing a separation target substance according to the sixth embodiment of the present invention.

Fig. 41 is a schematic diagram for illustrating a mechanism of capture of a plurality of beads.

Fig. 42 is a diagram for illustrating a 2-particle model of beads.

Fig. 43 is a diagram showing a schematic construction of an assembling apparatus according to a seventh embodiment of the present invention.

Fig. 44 is an enlarged perspective view of a construction of the assembling apparatus shown in Fig. 43 around a substrate.

Fig. 45 is a diagram for illustrating samples used in Figs. 46 to 49.

Fig. 46 shows successive photographs showing change over time of a region in the vicinity of a laser spot resulting from irradiation with light in an eighth sample.

Fig. 47 shows an optical photograph of an assembly formed in a tenth sample which is naturally dried after irradiation with light is stopped.

Fig. 48 shows an SEM image of a region A (a region in the vicinity of a laser spot) shown in Fig. 47.

Fig. 49 is a schematic diagram for illustrating a mechanism of formation of an assembly in the seventh embodiment.

Fig. 50 shows successive photographs showing change over time of a portion in the vicinity of a laser spot resulting from irradiation with light in the tenth sample.

Fig. 51 is a diagram for comparison of change over time in size of assemblies after irradiation with light is started, among eighth to tenth samples.

Fig. 52 is a diagram showing extinction spectra in the vicinity of a gas-liquid interface before start of irradiation with light, of eighth and eleventh samples.

Fig. 53 shows successive photographs showing change over time of a portion in the vicinity of a laser spot resulting from irradiation with light in a twelfth sample.

Fig. 54 shows an optical photograph of an assembly formed in the twelfth sample which is naturally dried after irradiation with light.

Fig. 55 shows an SEM image of a laser spot region (a region R2 shown in Fig. 54) of the twelfth sample.

Fig. 56 shows an SEM image of a region (a region R3 shown in Fig. 54) in a region distant from the laser spot in the twelfth sample.

Fig. 57 is a conceptual diagram for illustrating introduction of peptides as a result of endocytosis by cells.

Fig. 58 is an enlarged perspective view of a construction around a substrate.

Fig. 59 is a top view of the substrate shown in Fig. 57.

Fig. 60 is a diagram showing a peptide employed in an eighth embodiment.

Fig. 61 is a flowchart for illustrating a method of introducing peptides according to the eighth embodiment of the present invention.

Fig. 62 is a schematic diagram for illustrating a mechanism of assembly of peptides in the eighth embodiment.

Fig. 63 shows a confocal micrograph after a thirteenth sample is irradiated with light.

Fig. 64 shows an optical photograph and a fluorescent image in the vicinity of a laser spot resulting from irradiation with light in a fourteenth sample.

DESCRIPTION OF EMBODIMENTS

An embodiment of the present invention will be described hereinafter in detail with reference to the drawings. The same or corresponding elements in the drawings have the same reference characters allotted and description thereof will not be repeated.

In the present invention and embodiments thereof, a holding region in a substrate is heated by irradiating the holding region with light to thereby produce a temperature difference and hence convection and air bubbles in the vicinity of the holding region. "In the vicinity of" preferably refers to a space at a distance smaller than, for example, twice as large as a diameter of an air bubble. "Air bubbles being produced in the vicinity of the holding region" includes a case where air bubbles are produced in contact with the holding region.

In the present invention and embodiments thereof, the term "microscopic object" means an object having a size from a sub nanometer order to a micrometer order. A shape of a microscopic object is not particularly limited, and a microscopic object may be in a shape, for example, of a sphere, an oval sphere, and a rod. When a microscopic object is in a shape of an oval sphere, at least one of a length in a direction of the minor axis and a length in a direction of the major axis of the oval sphere should only be from the sub nanometer order to the micrometer order. When a microscopic object is in a shape of a rod, at least one of a width and a length of the rod should only be from the sub nanometer order to the micrometer order.

Examples of the microscopic object include metallic nanoparticles, a metallic nanoparticle assembly, a metallic nanoparticle assembly structure, semiconductor nanoparticles, organic nanoparticles, and resin beads. The "metallic nanoparticles" refer to metallic particles having a size of the nanometer order. The "metallic nanoparticle assembly" refers to an assembly formed by aggregation of a plurality of metallic nanoparticles. The "metallic nanoparticle assembly structure" refers to a structure, for example, in which a plurality of metallic nanoparticles are fixed to a surface of a bead with an interacting site being interposed and arranged at an interval not greater than a diameter of the metallic nanoparticle with a gap lying therebetween. The "semiconductor nanoparticles" refer to semiconductor particles having a size of the nanometer order. The "organic nanoparticles" refer to particles composed of an organic compound having a size of the nanometer order. The "resin beads" refer to particles composed of a resin having a size from the nanometer order to the micrometer order.

The microscopic object may be an object derived from a living body (such as cells, tissues, and organs) or an object interacting with a living body, and may include, for example, cells and biological materials. The biological material may include biopolymers such as nucleic acid, protein, and polysaccharide. The microscopic object may include microorganisms (such as bacteria and fungi), antigens such as allergens, and viruses.

In the present invention and embodiments thereof, an "introduction target substance" may be a biological material so long as it is a substance which can be introduced in cells, or may be an inorganic molecule or an organic molecule (such as drugs), without being limited to the biological material. The "introduction target substance" may include microorganisms, antigens, viruses, and ions.

In the present invention and embodiments thereof, the term "microscopic object assembly structure" means a structure formed by assembly of a plurality of microscopic objects.

In the present invention and embodiments thereof, the "particulate matter (PM)" refers to a particulate substance having a size of the micrometer order. Examples of PM include PM 2.5, PM 10, and suspended particulate matter (SPM). PM 2.5 refers to particles having a particle size not greater than 2.5 $\mu$m. PM 10 and SPM are both particles having a particle size not greater than 10 $\mu$m. PM 10 and SPM are different from each other in that PM 10 may include particles having a particle size exceeding 10 $\mu$m whereas SPM does not include particles having a particle size exceeding 10 $\mu$m.

In the present invention and embodiments thereof, "microbubbles" refer to air bubbles of the micrometer order.

In the present invention and embodiments thereof, the "sub nanometer order" covers a range from 0.1 nm to 1 nm. The "nanometer order" covers a range from 1 nm to 1000 nm (1 $\mu$m). The "micrometer order" covers a range from 1 $\mu$m to 1000 $\mu$m (1 mm). Therefore, the term "from the sub nanometer order to the micrometer order" covers a range from 0.1 nm to 1000 $\mu$m. The term "from the sub nanometer order to the micrometer order" may typically represent a range from several nanometers to several hundred micrometers, preferably a range from 0.1 $\mu$m to 100 $\mu$m, and more

preferably a range from 0.1 μm to several ten micrometers.

In the present invention and embodiments thereof, the term "type" means a group of microscopic objects when microscopic objects are categorized based on common properties. The common properties include physical characteristics or chemical characteristics of a microscopic object.

In the present invention and embodiments thereof, the term "physical characteristics" means mechanical characteristics (dynamic characteristics), electrical characteristics, magnetic characteristics, optical characteristics, or thermal characteristics of a microscopic object. Specific examples of mechanical characteristics include a size, a shape, an internal structure, and a density of a microscopic object. Specific examples of electrical characteristics include surface charges and a dielectric constant of a microscopic object. Specific examples of magnetic characteristics include permeability of a microscopic object. Specific examples of optical characteristics include a resonance absorption wavelength (an excitation wavelength), an index of refraction (a square root of a high-frequency dielectric constant), and non-linear susceptibility of a microscopic object. Specific examples of thermal characteristics include a specific heat, a thermal conductivity, a coefficient of thermal expansion, and resistance to heat of a microscopic object.

In the present invention and embodiments thereof, the term "chemical characteristics" means characteristics relating to a material for a microscopic object and affinity of a microscopic object to a liquid (a dispersion medium) in which the microscopic object is dispersed. Specific examples of characteristics relating to a material for a microscopic object include a composition and a molecular structure of the microscopic object. Specific examples of affinity of a microscopic object to a dispersion medium include a degree of hydrophilicity or hydrophobicity of the microscopic object.

In the present invention and embodiments thereof, the term "biological characteristics" means characteristics of a microscopic object as cells. Specific examples of the characteristics as cells include cell division, production and secretion of protein, lipid, and sugar, degradation of a substance, and apoptosis.

In the present invention and embodiments thereof, the term "absorbing light" or "having light absorbency" means such a property that energy of light absorbed by a substance is greater than zero. A wavelength region of light may any of any region of an ultraviolet region, a visible region, and a near infrared region, a region extending over two regions of these three regions, and a region extending over all of these three regions. Light may be laser beams, and may be light great in spectrum width such as white light or pulsed light, without being limited to the former.

Light absorbency can be defined, for example, by a range of absorptance. In this case, the lower limit of the range of the absorptance should only be greater than zero, without being particularly limited. The upper limit of the range of the absorptance is 100%.

In the present invention and embodiments thereof, the term "dispersion" means floating of a microscopic object in a liquid. The term "monodisperse" means high uniformity in particle size and shape. By way of example, a coefficient of variation (CV) defined by a ratio of a standard deviation to an average particle size can typically be not higher than 15%, preferably not higher than 10%, and more preferably not higher than 5%.

[First Embodiment]

Resin beads composed of polystyrene are adopted as one exemplary form of a microscopic object in a first embodiment below. A material for the resin beads is not limited as such, and the resin beads may be composed, for example, of an acrylic, polyolefin, polyethylene, or polypropylene.

<Construction of Assembling Apparatus>

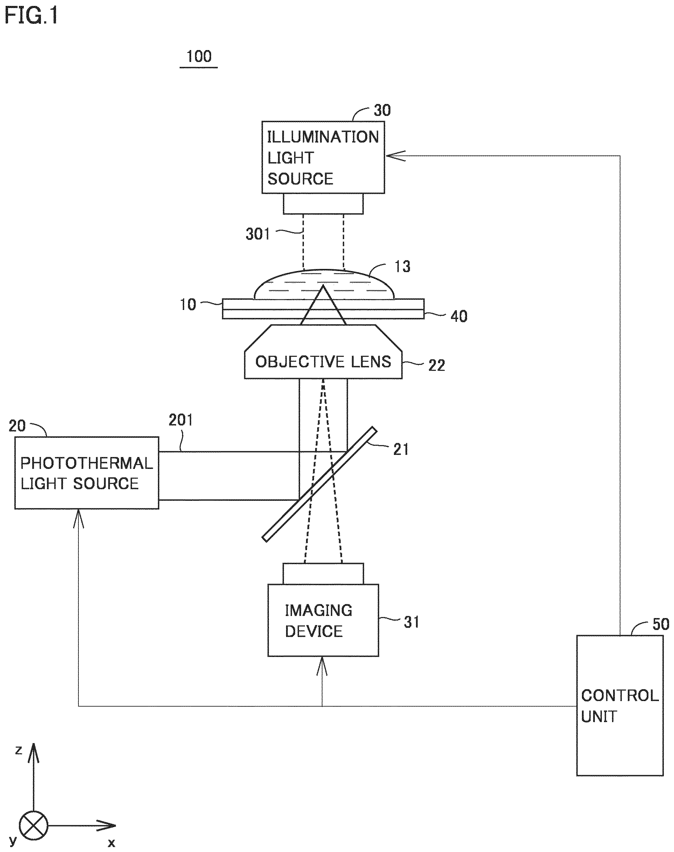

Fig. 1 is a diagram showing a schematic construction of an assembling apparatus according to the first embodiment of the present invention. Referring to Fig. 1, an assembling apparatus 100 includes a substrate 10, a photothermal light source 20, a dichroic mirror 21, an objective lens 22, an illumination light source 30, a shooting instrument 31, an XYZ-axis stage 40, and a control unit 50. The x direction and the y direction represent a horizontal direction. The x direction and the y direction are orthogonal to each other. The z direction represents a vertical direction. A direction of gravity is downward in the z direction.

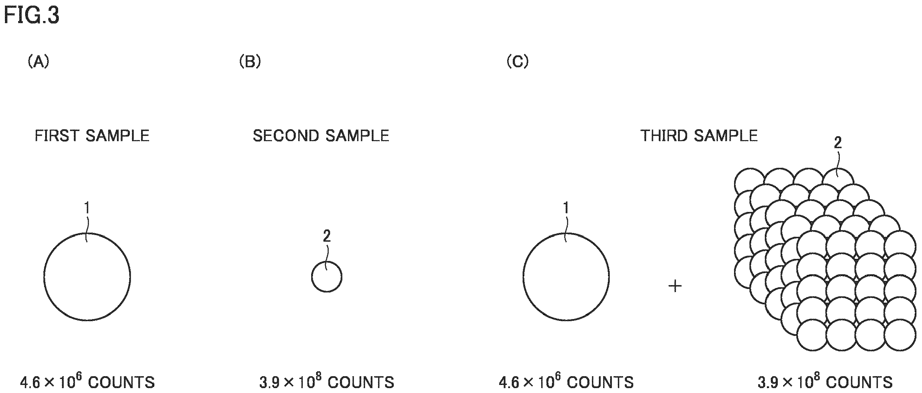

Substrate 10 is arranged on XYZ-axis stage 40. Substrate 10 holds a sample 13. In the present embodiment, sample 13 is a liquid in which at least one of beads 1 and beads 2 (see Fig. 3) are dispersed. Details of sample 13 will be described later.

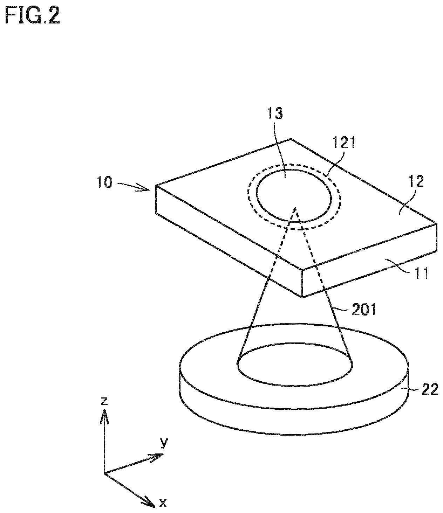

Photothermal light source 20 generates, for example, near infrared laser beams 201 (for example, at a wavelength of 1064 nm). Laser beams 201 are preferably continuous light. A specific construction of photothermal light source 20 is not limited as such. Various light sources which emit light in a light absorption band of a material for a thin film 12 (see Fig. 2) which will be described later can be employed as photothermal light source 20.

Dichroic mirror 21 reflects near infrared light whereas it allows passage of white light therethrough. Dichroic mirror 21 reflects near infrared laser beams 201 from photothermal light source 20 and guides the reflected light to objective lens 22.

Objective lens 22 gathers laser beams 201 from photothermal light source 20. Objective lens 22 can form, at a focal position thereof, a laser spot (or a beam waist) at which a diameter of a cross-section of laser beams 201 in a direction perpendicular to an optical axis is minimal. Substrate 10 or sample 13 is irradiated with light gathered by objective lens 22. Here, "to irradiate" includes passage of laser beams 201 through substrate 10 or sample 13, that is, it is not limited to a case where a laser spot of light gathered by objective lens 22 is located in substrate 10 or sample 13.

In the present embodiment, two types of objective lenses 22 different in magnification are prepared. A magnification of the objective lens is set to 10x or 100x. Dichroic mirror 21 and objective lens 22 are incorporated, for example, in a main body of an inverted microscope (not shown).

When a 100x objective lens is employed, power of laser beams 201 after passage through objective lens 22 is, for example, approximately 20% of power of laser beams 201 emitted from photothermal light source 20. In the present embodiment, power of laser beams 201 is 0.5W, and power of laser beams 201 after passage through objective lens 22 and substrate 10 is 100 mW in the absence of thin film 12 and sample 13. The laser spot has a diameter of approximately 1 $\mu$m. When a 10x objective lens is employed, power of laser beams 201 after passage through objective lens 22 and substrate 10 is approximately 60% of power of laser beams 201 emitted from photothermal light source 20. The laser spot has a diameter of approximately 10 $\mu$m. In each embodiment and a modification thereof which will be described below, power of laser beams 201 after passage through objective lens 22 and substrate 10 is set to 100 mW in the absence of thin film 12 and sample 13, unless otherwise indicated.

Illumination light source 30 emits light for illuminating sample 13 on substrate 10. Illumination light source 30 is a light source emitting, for example, white light 301. In one example, a halogen lamp can be employed for illumination light source 30.

Objective lens 22 is used also for taking in white light 301 from sample 13. White light 301 which has passed through objective lens 22 passes through dichroic mirror 21 and reaches shooting instrument 31.

Shooting instrument 31 shoots a region in the vicinity of a laser spot of laser beams 201 in sample 13. Shooting instrument 31 is implemented, for example, by a video camera including an image sensor. An image shot by shooting instrument 31 may be moving images or still images.

Control unit 50 controls photothermal light source 20, illumination light source 30, and shooting instrument 31. Control unit 50 is implemented, for example, by a microcomputer or a personal computer.

An optical system of assembling apparatus 100 is not limited to a system including a dichroic mirror so long as the system can irradiate substrate 10 with photothermal laser beams 201 and can take white light 301 from sample 13 into shooting instrument 31. The optical system of assembling apparatus 100 may include optical components such as optical fibers in addition to or instead of the dichroic mirror. In assembling apparatus 100, illumination light source 30 and shooting instrument 31 are not essential constituent elements.

Fig. 2 is an enlarged perspective view of a construction of assembling apparatus 100 shown in Fig. 1 around substrate 10. Referring to Figs. 1 and 2, substrate 10 includes a cover glass 11 and thin film 12 formed on cover glass 11.

Thin film 12 is formed to absorb laser beams 201 from photothermal light source 20 and to convert light energy to thermal energy. A material for thin film 12 is preferably high in absorbency of light (for example, photothermal conversion efficiency) in a wavelength band (near infrared in the present embodiment) of laser beams 201. A thickness of thin film 12 is preferably determined in design or experimentally, in consideration of intensity of laser beams 201 and light absorbency of a material for thin film 12.

In the present embodiment, a thin gold film having a thickness of the nanometer order is formed as thin film 12. Free electrons at a surface of the thin gold film form surface plasmon and are oscillated by laser beams 201. Polarization is thus caused. Consequently, the thin gold film generates heat. This effect is referred to as a "photothermal effect."

Though a photothermal effect is produced by using light at a wavelength of 1064 nm as laser beams 201 in the present embodiment, light at a wavelength close to a surface plasmon resonant wavelength (a wavelength in a wavelength region of visible light from 400 nm to 800 nm in air or water) of the thin gold film may be employed as laser beams 201. Thus, more heat can be generated with the same laser power.

Thin film 12 can be formed with a known technique such as vapor deposition, sputtering, or self-assembly. In the present embodiment, thin film 12 is formed by using an ion sputtering apparatus E-1010 manufactured by Hitachi High-Technologies Corporation. A thickness of thin film 12 is set to 10 nm.

A material for thin film 12 is not limited to gold, and may be a metal element other than gold (such as silver) which can achieve the photothermal effect or a metallic nanoparticle assembly structure (for example, a structure containing gold nanoparticles or silver nanoparticles). Alternatively, a material high in light absorptance in a wavelength band of laser beams 201 may be employed. Examples of such a material include a material close to a blackbody (for example, a carbon nanotube blackbody).

A position of substrate 10 relative to a focal point of objective lens 22 is adjusted by adjusting a position of XYZ-axis stage 40. A laser spot of laser beams 201 is formed at a position of the focal point of objective lens 22. The laser spot of laser beams 201 is preferably adjusted to be located in the vicinity of thin film 12.

A holding region in which sample 13 is held is formed in substrate 10. In the example shown in Fig. 2, thin film 12 is formed over the entire cover glass 11, and a region (shown with a dashed line) in thin film 12 where sample 13 is held corresponds to a holding region 121. A thin film may be formed only in holding region 121. Though not shown in Fig. 2, a droplet guide indicating a position onto which sample 13 should be dropped may be provided. As a droplet guide, an annular step along an outer periphery of holding region 121 can be formed. A size and a shape of the droplet guide are determined as appropriate in accordance with a volume of sample 13 to be held.

Sample 13 is a liquid in which polystyrene beads are dispersed. Though a type of a liquid (a dispersion medium) is not particularly limited, water (specifically, ultrapure water) is employed in the present embodiment. Three types of samples are prepared in the present embodiment. First and second samples described below are prepared as comparative examples for a third sample. A volume of each liquid is set to 10 $\mu$L.

Fig. 3 is a diagram for illustrating beads dispersed in the first to third samples. Referring to Fig. 3(A) to (C), each of beads 1 and 2 is a hydrophobic particle. Beads 1 and 2 are in a substantially spherical shape. In order to prevent aggregation of beads 1 and 2 dispersed in the liquid, surfaces of all beads 1 and 2 are charged to the same sign (negatively charged in the present embodiment).

The first sample is a monodisperse liquid containing only beads 1 (see Fig. 3(A)). Beads 1 have a particle size of 1.0 $\mu$m. The number N1 of beads 1 is set to $4.6 \times 10^6$.

The second sample is a monodisperse liquid containing only beads 2 (see Fig. 3(B)). Beads 2 have a particle size of 0.2 $\mu$m. The number N2 of beads 2 is set to $3.9 \times 10^8$.

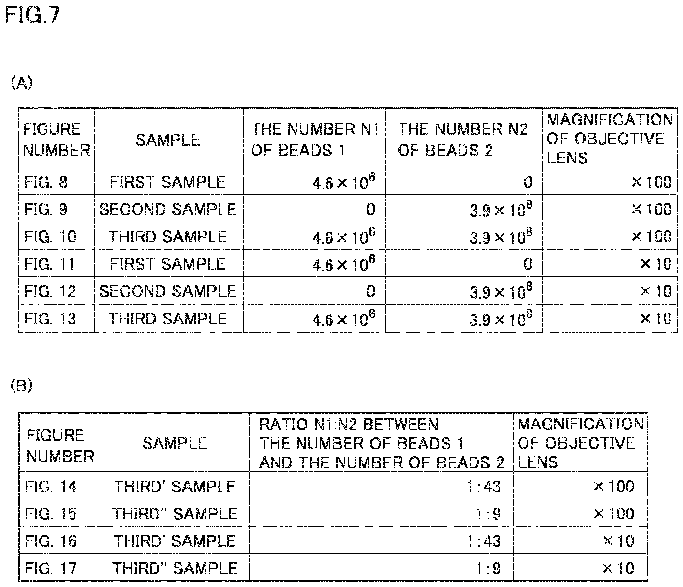

The third sample is a liquid containing both of beads 1 and 2 (see Fig. 3 (C)). The number N1 of beads 1 and the number N2 of beads 2 are set to $4.6 \times 10^6$ and $3.9 \times 10^8$, respectively. A ratio between the number of beads 1 and the number of beads 2 in the third sample is N1:N2 = 1:85. Ratios between the number of beads 1 and the number of beads 2 in a third' sample and a third" sample which will be described later are N1:N2 = 1:43 and 1:9, respectively (see Fig. 7).

In the present embodiment, Fluoresbrite carboxylate microspheres (2.5% Solids-Latex), 1.0 $\mu$m BB manufactured by Polysciences, Inc. are employed as beads 1. Fluoresbrite carboxylate microspheres (2.5% Solids-Latex), 0.20 $\mu$m NYO manufactured by the same company are employed as beads 2.

<Assembling Method>

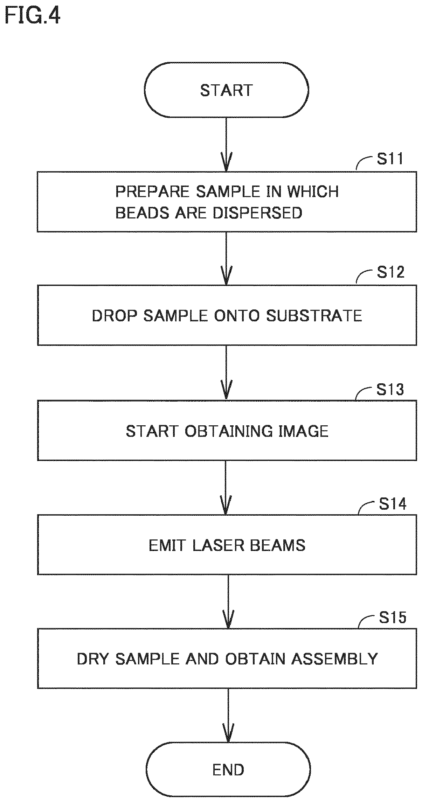

Fig. 4 is a flowchart for illustrating a method of assembling beads according to the first embodiment of the present invention. Referring to Figs. 1 to 4, in step S11, sample 13 in which beads are dispersed (any of the first to third samples) is prepared. Sample 13 is then dropped onto substrate 10 (step S12). After sample 13 is dropped, sample 13 may be rested for a prescribed period of time (for example, approximately from 5 to 10 minutes). By resting sample 13 until movement of beads in sample 13 becomes less, conditions for assembling beads are unified.

Control unit 50 controls shooting instrument 31 so as to start shooting of sample 13 (step S13). Control unit 50 controls photothermal light source 20 so as to irradiate substrate 10 with laser beams 201 (step S14). In step S14, laser beams 201 for producing a temperature difference in sample 13 are gathered by objective lens 22 and gathered light is introduced into holding region 121 of thin film 12 or sample 13. Beads dispersed in sample 13 are thus assembled. Details of assembly of beads and a mechanism thereof will be described later.

Thereafter, the liquid representing sample 13 is vaporized (step S 15). It thus becomes easy to obtain an assembly of beads formed on substrate 10. A technique for vaporizing the liquid is not particularly limited. For example, the liquid may be vaporized by continued irradiation with laser beams 201 and resultant heat generation by thin film 12. In the present embodiment, irradiation with laser beams 201 is stopped and sample 13 is left until the liquid disappears as a result of natural evaporation in air. Sample 13 is naturally dried.

Since operations in steps S 13 and S 15 are operations for observing sample 13 or obtaining an assembly, they are not essential in assembly of beads. Beads can be assembled even when a flowchart including only operations in steps S11, S12, and S14 is executed.

<Assembly of Beads and Mechanism of Assembly>

In succession, one example of assembly of beads will be described with reference to photographs taken by shooting instrument 31. Photographs of the third sample containing both of beads 1 and 2 will be shown here.

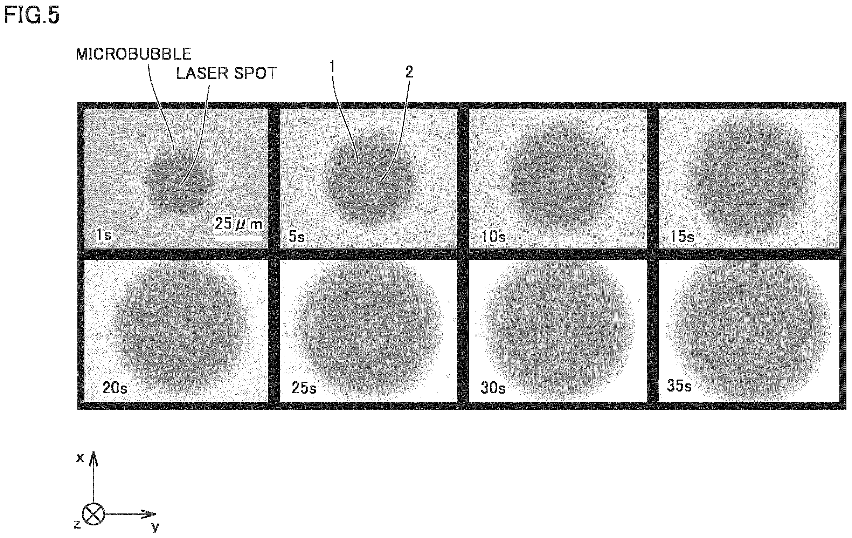

Fig. 5 shows successive photographs showing change over time of a region in the vicinity of a laser spot after start of irradiation with light in the third sample. Referring to Fig. 5, each photograph is obtained by shooting the third sample from below in the vertical direction (the z direction). Time shown in each photograph (such as 1 s and 5 s) represents lapse of time (unit: second) with the timing of start of irradiation with light being defined as the reference. Objective lens 22 has a magnification of 100x.

When irradiation with laser beams 201 is started, a microbubble MB is produced around a laser spot. When irradiation with laser beams 201 is continued, microbubble MB grows with lapse of time. In the example shown in Fig. 5, a size of microbubble MB is substantially saturated after lapse of approximately 25 seconds since start of irradiation with light.

The photographs show assembly of beads 1 and 2 vertically below microbubble MB around the laser spot. Assembly of beads 1 and 2 can be observed in approximately several seconds (for example, 1 second to 5 seconds) after start of irradiation with light. Thereafter, with lapse of time, an amount of assembly of beads 1 and 2 increases.

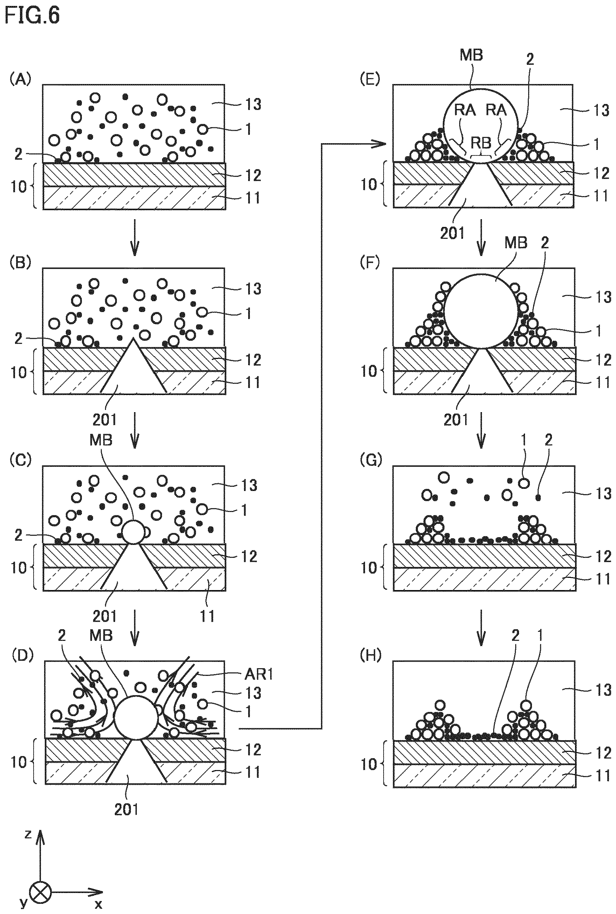

Fig. 6 is a schematic diagram for illustrating a mechanism of formation of an assembly of beads 1 and 2 in the third sample. Referring to Fig. 6, before irradiation with light is started, beads 1 and 2 are dispersed in the liquid representing sample 13 (see Fig. 6 (A)).

When irradiation with laser beams 201 is started, thin film 12 absorbs laser beams 201. Light energy absorbed in thin film 12 is converted to thermal energy as a result of the photothermal effect (Fig. 6 (B)). Thus, a region in thin film 12 in the vicinity of the laser spot is locally heated. With increase in temperature of the liquid around the heated region, microbubble MB is produced (see Fig. 6 (C)). Microbubble MB grows in the vicinity of thin film 12.

A temperature of the liquid is higher toward the laser spot. In other words, a temperature difference is produced in the liquid as a result of irradiation with laser beams 201. Convection is thus produced in the liquid. Convection produced here is thermal convection. Convection is once directed to microbubble MB and thereafter directed away from microbubble MB as shown with an arrow AR1 (see Fig. 6 (D)).

A stagnation region where a current velocity of convection is zero is produced between microbubble MB and thin film 12 on substrate 10. Beads 1 and 2 which have moved over convection are initially pressed against a surface of microbubble MB Many of beads 1 and 2 stay in the vicinity of the ring-shaped stagnation region formed between microbubble MB and substrate 10. Microbubble MB functions as a fluid stopper of the micrometer order which holds back convection which can be controlled by irradiation with light. Since beads 2 are smaller than beads 1, beads 2 tend to enter a region RA between microbubble MB and substrate 10 (see Fig. 6 (E) and (F)). Some of beads 2 may enter also a region RB vertically directly under microbubble MB Since beads 1 are less likely to enter regions RA and RB, beads 1 tend to assemble at a position more distant from the laser spot than beads 2.

When assembly of beads 1 and 2 is completed, irradiation with laser beams 201 is stopped. In a subsequent process for vaporizing the liquid representing sample 13, microbubble MB is dissociated from thin film 12. Accordingly, some of beads 1 and 2 assembled around microbubble MB are raised in the liquid (see Fig. 6 (G)). Raised beads 1 and 2 fall down through the liquid under the gravity and assembled on a surface of thin film 12 on substrate 10 (see Fig. 6 (H)).

<Influence of Particle Size of Beads on Structure of Assembly>

A structure of an assembly in each of the first to third samples will now be described. An image of an assembly taken by a scanning electron microscope (SEM) and a result of determination of a cross-sectional shape of the assembly by a laser microscope are shown below. The result shown below is obtained through measurement of sample 13 after it is naturally dried.

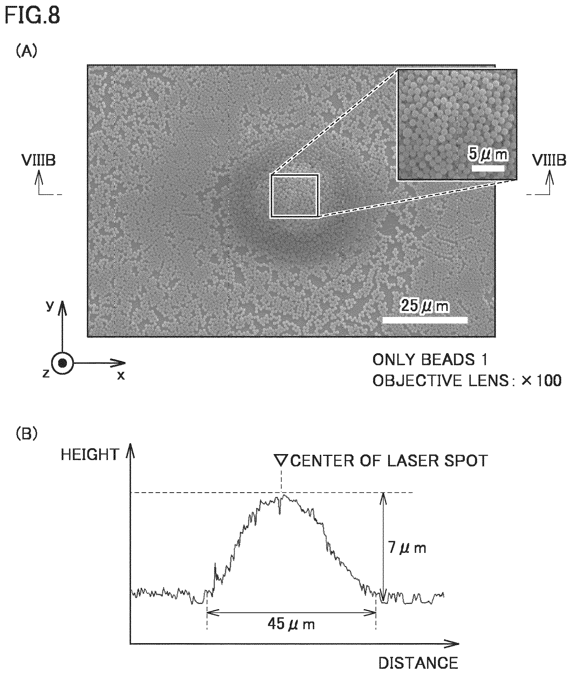

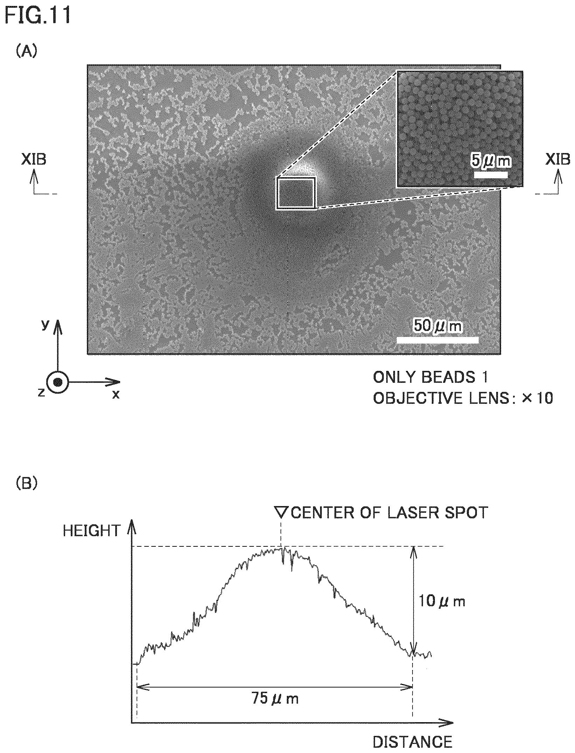

Fig. 7 is a diagram for illustrating conditions for assembling beads in Figs. 8 to 17. Referring to Fig. 7 (A), Figs. 8 to 10 are diagrams showing results of assembly of beads when 100x objective lens 22 (hereinafter simply also denoted as a "100x lens") is employed for the first to third samples, respectively. Figs. 11 to 13 are diagrams showing results of assembly of beads when 10x objective lens 22 (hereinafter simply also denoted as a "10x lens") is employed for the first to third samples, respectively. Referring to Fig. 7 (B), Figs. 14 to 17 are diagrams showing results of assembly of beads when a ratio between the number of beads 1 and the number of beads 2 (N1:N2) is varied between the third' and third" samples.

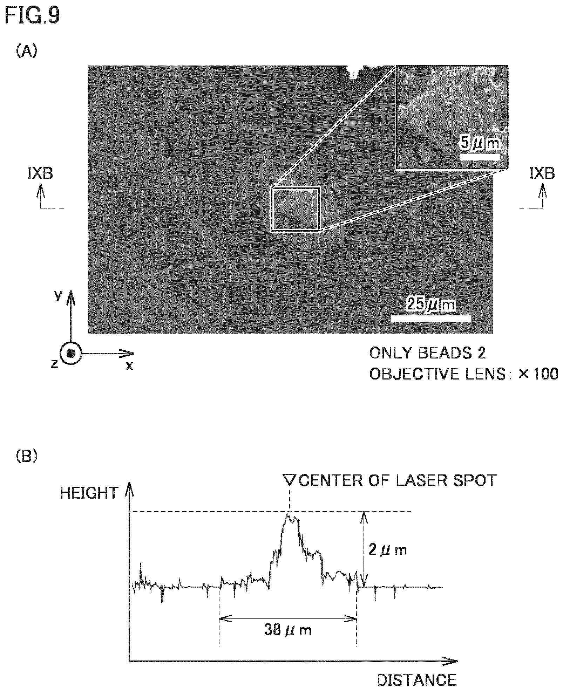

Figs. 8 and 9 are diagrams showing SEM images of assemblies formed with the 100x lens and results of determination of cross-sectional shapes of the assemblies, in the first and the second samples, respectively. Figs. 8 (A) and 9 (A) are SEM images of the respective assemblies. Fig. 8 (B) is a diagram showing a result of determination of the cross-sectional shape of the assembly along the line VIIIB-VIIIB. The abscissa in Fig. 8 (B) represents a distance along the line VIIIB-VIIIB. The ordinate in Fig. 8(B) represents a height in a vertically upward direction (the z direction). Explanation of a result of measurement shown in Fig. 9 (B) is the same as that for Fig. 8 (B).

Referring to Fig. 8 (A) and (B), in connection with the first sample, a height of the assembly formed from beads 1 is highest at the center of the laser spot and the height decreases with a distance from the center of the laser spot is greater. Such a shape can be expressed as a dome shape.

Referring to Fig. 9 (A) and (B), in connection with the second sample, the assembly formed from beads 2 is smaller in height and diameter than the assembly formed from beads 1. The assembly formed from beads 2, however, is common to the assembly formed from beads 1 in that a height thereof is greatest at the center of the laser spot.

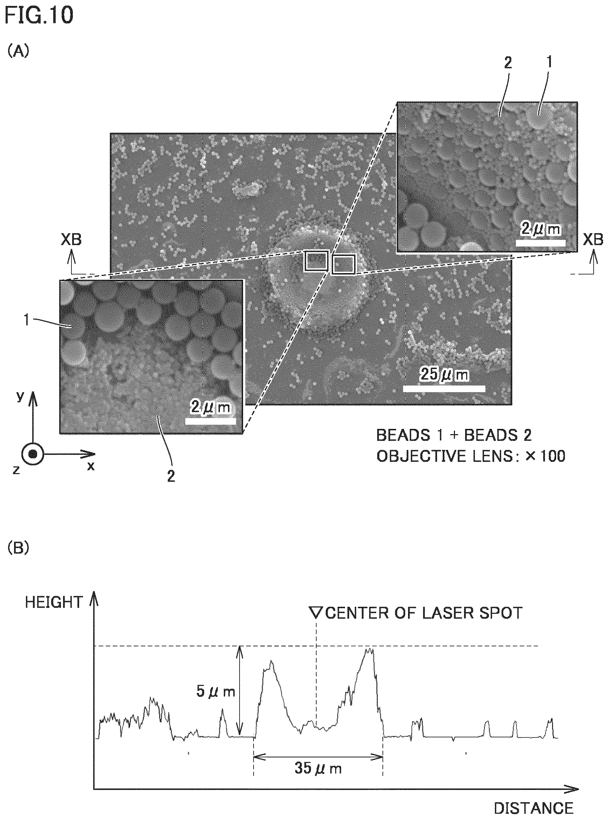

Fig. 10 is a diagram showing an SEM image of the assembly formed with the 100x lens and a result of determination of a cross-sectional shape of the assembly, in the third sample. Fig. 10 is compared with Figs. 8 and 9.

Referring to Fig. 10 (B), a height of the assembly at the center of the laser spot is smaller than the height of the assembly around the laser spot. Thus, in the sample containing both of beads 1 and beads 2, an assembly in what is called a ring shape is formed.

Referring further to Fig. 10 (A), as shown in an enlarged view in a lower left portion, it can be seen that the central portion of the assembly is mainly formed from beads 2. A structure mainly formed from beads 1 is formed to surround the central portion.

As set forth above, when the sample containing only one type of beads (the first sample) and the sample containing two types of beads (the third sample) are compared with each other, they are different in structure of the assembly. A factor for such a difference may be a difference in structure stability of an assembly structure (see Fig. 6(E) and (F)) formed at a gas-liquid interface between microbubble MB and a liquid around the same.

More specifically, in connection with the first sample, in the presence of microbubble MB, beads 1 stay along the gas-liquid interface between microbubble MB and a liquid around the same, so that the assembly formed from beads 1 is in a shape of a ring. In this assembly, a certain type of structure is formed by beads 1. This structure is considered as a hexagonal closest packing structure.

Microbubble MB disappears in a process of vaporization of the liquid representing sample 13. Therefore, the assembly formed from beads 1 can no longer maintain a ring shape by making use of the gas-liquid interface.

In addition, capillary force acts between beads 1 before the liquid is completely vaporized. Capillary force acts as attraction force between hydrophilic microscopic objects or between hydrophobic microscopic objects, whereas it acts as repulsion force between a hydrophilic microscopic object and a hydrophobic microscopic object. Since beads 1 are hydrophobic, capillary force acts as attraction force to thereby aggregate beads 1.

As set forth above, when the liquid representing sample 13 is vaporized, the gas-liquid interface can no longer be made use of for maintaining a ring shape and capillary force acts in a direction of aggregation of beads 1. Thus, in the case of the first sample, the assembly collapses in a direction toward the center of the ring shape. It is considered that as a result of deposition of collapsed beads 1 in the central portion of the ring shape, the shape of the assembly varies from the ring shape to the dome shape.

In contrast, in the case of the third sample, the hexagonal closest packing structure is formed by beads 1 as in the first sample. Since the third sample contains beads 2, beads 2 enter a gap between adjacent beads 1. Consequently, an interparticle distance between bead 1 and bead 2 is smaller than an interparticle distance between beads 1 in the hexagonal closest packing structure formed only from beads 1. By thus containing two types of beads different in particle size, an assembly structure high in density and structure stability is formed. Therefore, the assembly of the third sample is considered to be able to maintain the ring shape even after microbubble MB disappears.

<Influence of Magnification of Objective Lens on Structure of Assembly>

In succession, an influence of a magnification of objective lens 22 on a structure of an assembly will be described. Specifically, an assembly formed with a 10x lens and an assembly formed with a 100x lens are compared with each other.

Figs. 11 to 13 are diagrams showing SEM images of assemblies formed with a 10x lens and results of determination of cross-sectional shapes of the assemblies, in the first to third samples, respectively. Conditions for assembly in the results of assembly shown in Figs. 11 to 13 are similar to the conditions for assembly in the results of assembly shown in Figs. 8 to 10, respectively, except for a magnification of objective lens 22.

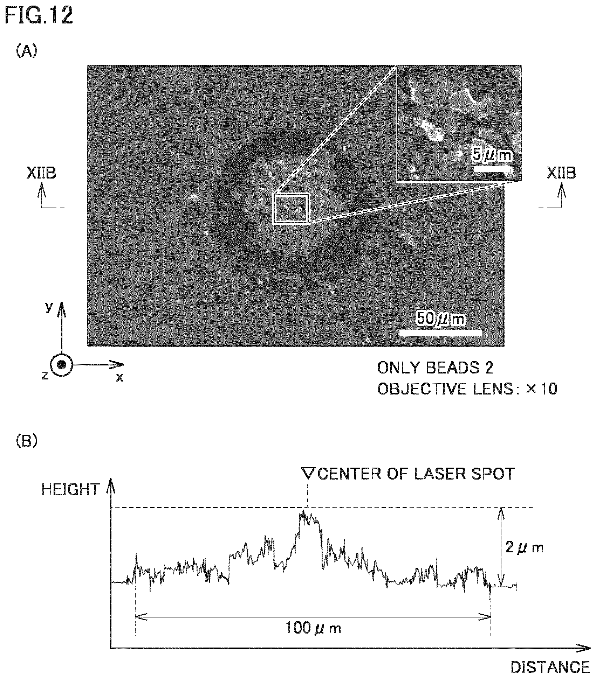

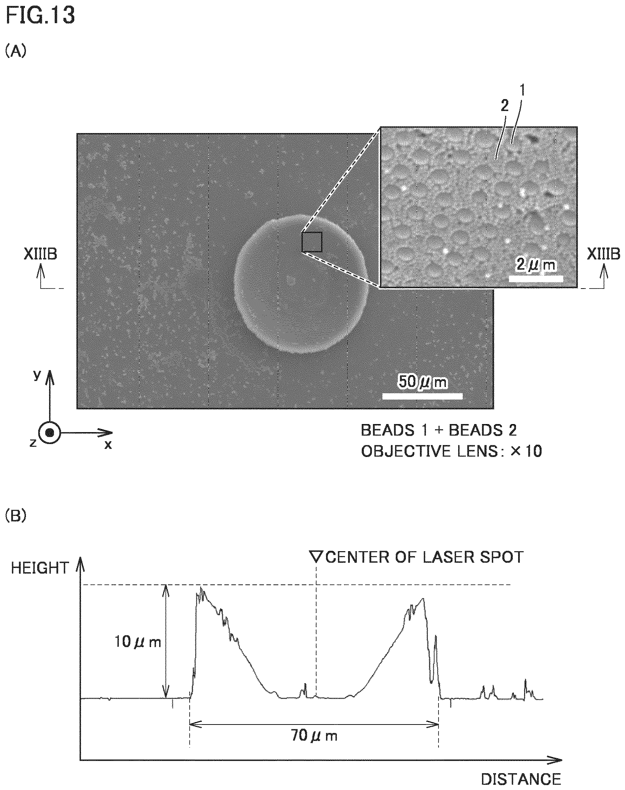

Referring to Figs. 8 and 11, a diameter of the assembly in the first sample is 45 $\mu$m in the case of the 100x lens, whereas the diameter thereof is 75 $\mu$m in the case of the 10x lens. Similarly, referring to Figs. 9 and 12, a diameter of the assembly in the second sample is 38 $\mu$m in the case of the 100x lens, whereas the diameter thereof is 100 $\mu$m in the case of the 10x lens. Referring to Figs. 10 and 13, a diameter of the assembly in the third sample is 35 $\mu$m in the case of the 100x lens, whereas the diameter thereof is 70 $\mu$m in the case of the 10x lens.

Thus, a size of the assembly is greater when the assembly is formed with the 10x lens than with the 100x lens. A size of the assembly can be controlled by using objective lenses different in magnification. The reason will be described below.

Larger microbubble MB is produced when laser beams 201 are gathered by using the 10x lens than by using the 100x lens. Referring to one example of an actually measured value, when light is gathered with the 100x lens, maximum values for diameters of microbubbles MB in the first to third samples are 93.8 $\mu$m, 91.1 $\mu$m, and 61.0 $\mu$m, respectively. When light is gathered with the 10x lens, maximum values for diameters of microbubbles MB in the first to third samples are 213 $\mu$m, 202 $\mu$m, and 177 $\mu$m, respectively. As described with reference to Fig. 6, an assembly is formed at the gas-liquid interface between microbubble MB and a liquid around the same. Therefore, as microbubble MB is greater, the assembly can be greater.

Three factors below may be reasons why microbubble MB is greater when light is gathered with the 10x lens than with the 100x lens.

The first factor may be an area of a laser spot. An area of a laser spot is greater when light is gathered with the 10x lens than with the 100x lens. Since an area of a region where the photothermal effect is produced is great, a volume of a liquid in contact with that region is also great. Consequently, microbubble MB is considered to become greater.

The second factor may be a ratio of laser beams 201 absorbed in thin film 12 (= light energy absorbed in thin film 12/light energy emitted to thin film 12). When intensity of laser beams 201 ([W/m²], that is, energy density [J/(m²·s)] per unit time) with respect to a thickness of thin film 12 is relatively high, some of laser beams 201 can pass through without being absorbed in thin film 12. This may be because when intensity of laser is not lower than a certain value, intensity of laser exceeds an allowable value of light absorbable in a substance due to a non-linear optical phenomenon called absorption saturation. Since an area of a laser spot is greater when light is gathered with the 10x lens than with the 100x lens even though power of laser beams is equal, energy density of light at the laser spot is low. Therefore, energy of light which passes through thin film 12 due to absorption saturation decreases and a ratio of light absorbed in thin film 12 becomes higher. Therefore, more light energy is absorbed in thin film 12 when light is gathered with the 10x lens, which can contribute to production of microbubble MB.

The third factor may be change (including peeling off) of thin film 12. When energy density of laser beams 201 is high, a thin film in a region of a laser spot may change. Since energy density at the laser spot is lower when the 10x lens is used than when the 100x lens is used, change of thin film 12 is less likely.

A thickness of thin film 12 is preferably determined based on intensity of laser beams and light absorbency of a material for the thin film as described above. As thin film 12 is smaller in thickness, heat generated by the photothermal effect tends to conduct through thin film 12. Since a region in thin film 12 where a temperature has increased is thus greater, a volume of a liquid in contact with the region increases. Consequently, greater microbubble MB can be produced. When thin film 12 is too thin with respect to intensity of laser beams 201, some of laser beams 201 is not absorbed in thin film 12. Consequently, thermal energy produced by the photothermal effect becomes less. Since such tradeoff relation exists, there is an optimal value for a thickness of thin film 12.

In the present embodiment, a thickness of thin film 12 is set to 10 nm because, as a result of formation of thin films having three types of thicknesses (10 nm, 30 nm, and 50 nm) and measurement of size of microbubbles MB under irradiation with light, the size of microbubble MB formed when the thickness was set to 10 nm was greatest.

Microbubbles MB are different in diameter depending on samples, even when magnification of objective lens 22 is the same. The diameter of microbubble MB is smaller in the order of the third sample, the second sample, and the first sample. In particular, the diameter of microbubble MB in the third sample is smaller than the diameter of microbubbles MB in the first and second samples. This may be because an assembly formed from a plurality of types of beads (the assembly in the third sample) is higher in structure stability than an assembly formed from a single type of beads (the assembly in the first or second sample), which tends to suppress growth of microbubble MB. This can be seen also from the fact that a diameter of microbubble MB produced in a sample not containing beads (ultrapure water) is greatest.

<Influence of Ratio of the Number of Beads on Structure of Assembly>

An influence of a ratio between the number of beads 1 and the number of beads 2 on a structure of an assembly will now be described. A structure of an assembly formed when a ratio between the number of beads 1 and the number of beads 2 is N1:N2 = 1:43 (the case of the third' sample) and a structure of an assembly formed in the case of N1:N2 = 1:9 (the case of the third" sample) are compared with each other below.

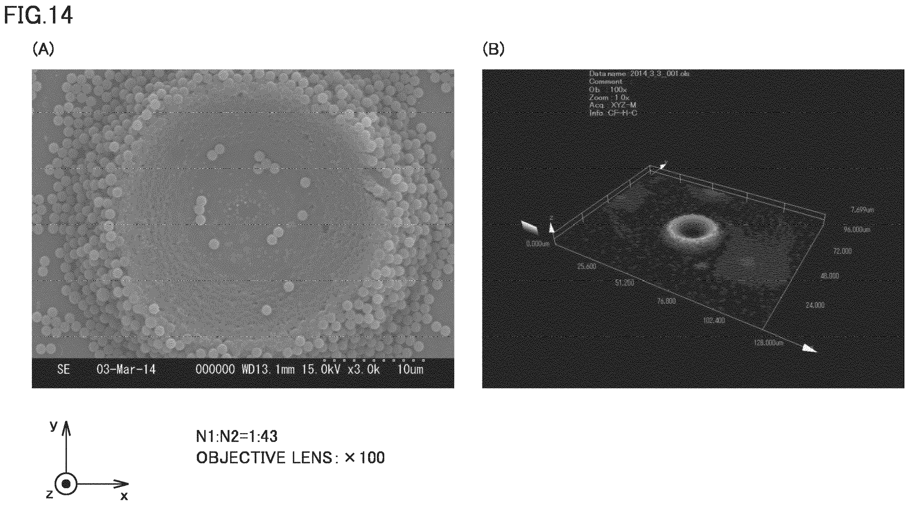

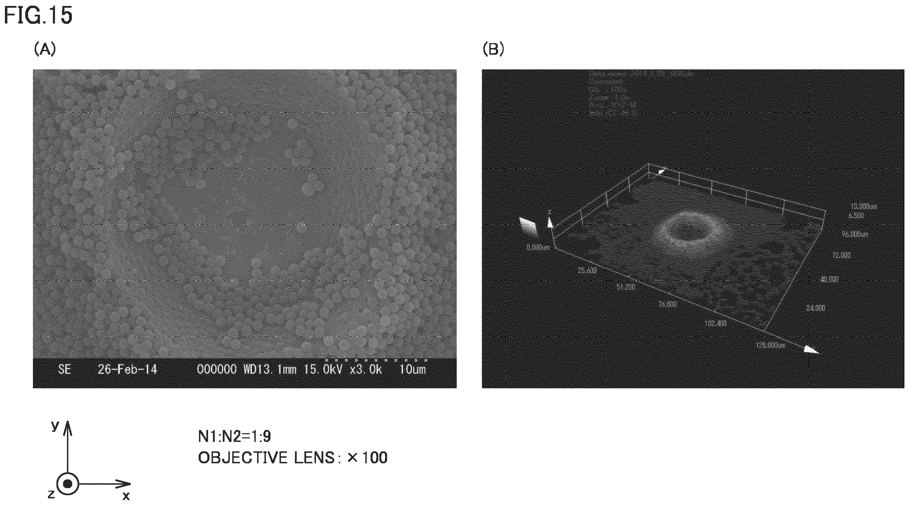

Fig. 14 is a diagram showing an SEM image of an assembly formed with the 100x lens and a three-dimensional shape of the assembly when a ratio between the number of beads 1 and the number of beads 2 is N1: N2 = 1:43. Fig. 15 is a diagram showing an SEM image of an assembly formed with the 100x lens and a three-dimensional shape of the assembly when a ratio between the number of beads 1 and the number of beads 2 is N1: N2 = 1:9. Figs. 14 (A) and 15 (A) show SEM images of the respective assemblies. Figs. 14 (B) and 15 (B) show results of determination of three-dimensional shapes of the respective assemblies.

Referring to Figs. 14 and 15, a diameter of the assembly is smaller whereas a height of the assembly is greater when a ratio between the number of beads 1 and the number of beads 2 is N1: N2 = 1:43 (see Fig. 14) than when N1:N2 = 1:9 (see Fig. 15).

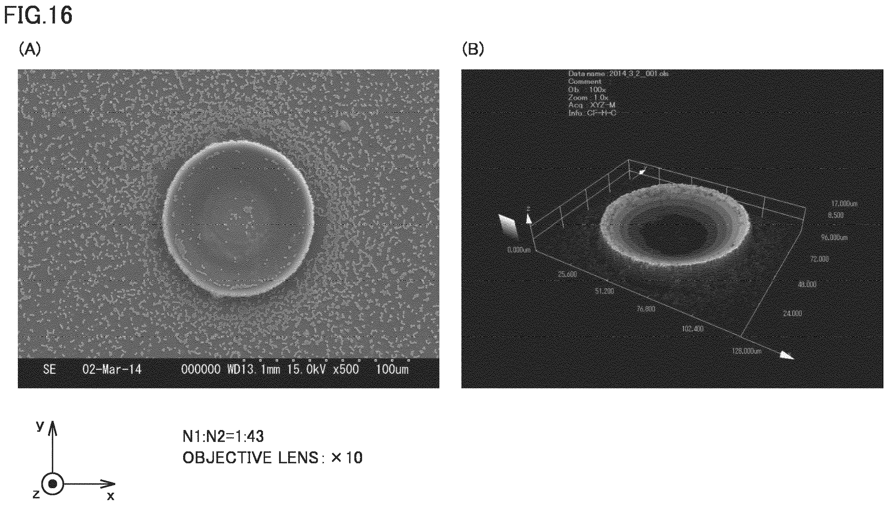

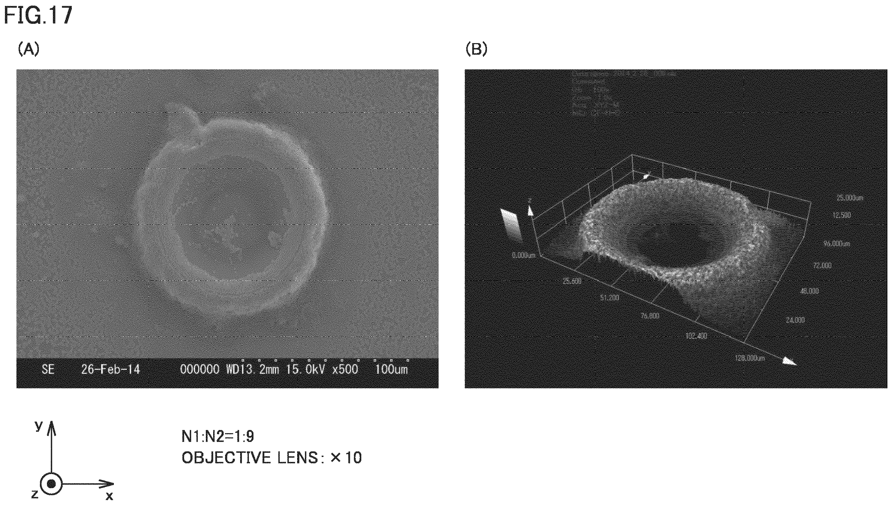

Fig. 16 is a diagram showing an SEM image of an assembly formed with the 10x lens and a three-dimensional shape of the assembly when a ratio between the number of beads 1 and the number of beads 2 is N1: N2 = 1:43. Fig. 17 is a diagram showing an SEM image of an assembly formed with the 10x lens and a three-dimensional shape of the assembly when a ratio between the number of beads 1 and the number of beads 2 is N1: N2 =1:9.

Referring to Figs. 16 and 17, as in Figs. 14 and 15, a diameter of the assembly is smaller whereas a height of the assembly is greater when a ratio between the number of beads 1 and the number of beads 2 is N1: N2 =1:43 (see Fig. 16) than when N1:N2 = 1:9 (see Fig. 17).

A ring shape of the assembly is confirmed more noticeably when a ratio between the number of beads 1 and the number of beads 2 is N1:N2 = 1:43 than in the case of N1:N2 = 1:9. This result shows that structure stability of the assembly is higher and the ring shape of the assembly tends to be maintained in the process of vaporization of sample 13 when a ratio between the number of beads 1 and the number of beads 2 is N1: N2 =1:43.

In the third' and third" samples, as described above, the hexagonal closest packing structure of beads 1 is formed when microbubble MB is produced. Then, beads 2 enter a gap between beads 1. Here, the number of beads 2 which enter a gap between beads 1 is greater when a total volume of beads 2 (a volume of each bead 2 $\times$ the number N2 of beads 2) is closer to a total volume of beads 1 (a volume of each bead 1 $\times$ the number N1 of beads 1). Structure stability of the assembly is thus enhanced.

In the examples shown in Figs. 14 to 17, since a ratio of a diameter between beads 1 (having a diameter of 1.0 $\mu$m) and beads 2 (having a diameter of 0.2 $\mu$m) is 5:1, a ratio of volume between beads 1 and beads 2 is 125:1. When a ratio between the number of beads 1 and the number of beads 2 is N1:N2 = 1:43, the total volume ratio is calculated as $(125\times1):(1\times43)$ = 2.9:1. When the ratio of the number is N1:N2 = 1:9, the total volume ratio is calculated as $(125\times1):(1\times9)$ = 14:1. Since the total volume of beads 2 is closer to the total volume of beads 1 when the ratio between the number of beads 1 and the number of beads 2 is N1:N2 = 1:43 than in the case of N1:N2 = 1:9, an assembly higher in structure stability is formed.

Thus, according to the present embodiment, a liquid in which a microscopic object is dispersed is prepared. The microscopic object can rapidly be assembled with a very simplified operation of irradiation of a substrate holding the liquid with light. In the sample shown in the present embodiment, assembly is achieved in a short period of time only approximately from several seconds to several ten seconds.

Since a microscopic object should only be dispersed in a liquid, a treatment for fixing a microscopic object onto a substrate as in PTD 1 is not necessary. Since a preparatory step before irradiation with light is reduced, cost can be reduced.

Though an example in which a microscopic object is dispersed in a liquid has been described in the present embodiment, a "space" according to the present invention is not limited to a space filled with a liquid. Even when a microscopic object is dispersed in a gas, the microscopic object can be assembled by producing a temperature difference in a gas through irradiation with light and by using convection resulting therefrom.

[Modification of First Embodiment]

Laser beams are absorbed by fluorescent beads in a modification of the first embodiment. In the present modification, a gas-liquid interface between a liquid representing a sample and a gas around the same is irradiated with laser beams.

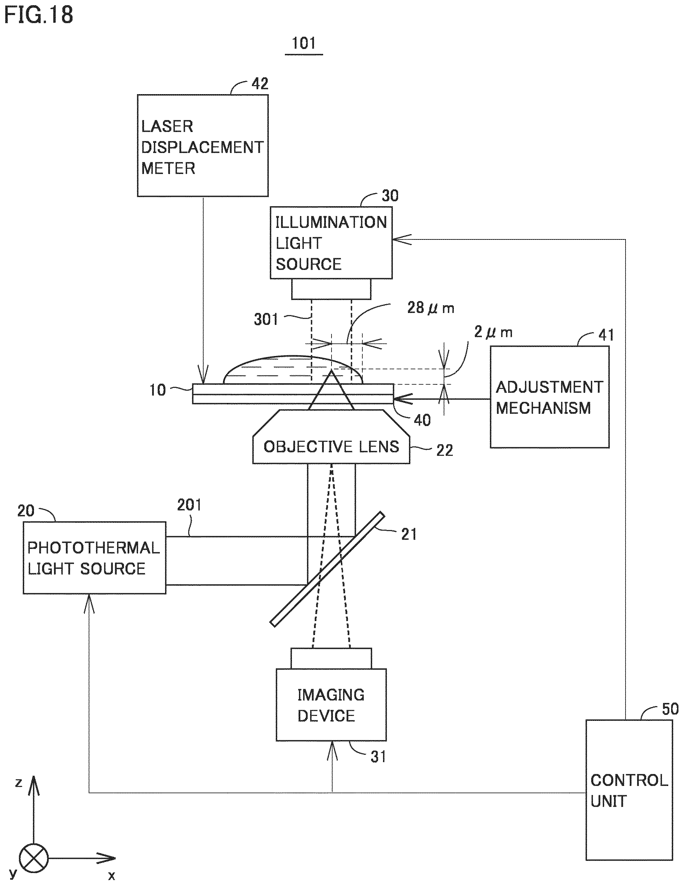

Fig. 18 is a diagram showing a schematic construction of an assembling apparatus according to the modification of the first embodiment of the present invention. Referring to Fig. 18, an assembling apparatus 101 is different from assembling apparatus 100 shown in Fig. 1 in further including an adjustment mechanism 41 and a laser displacement meter 42. Thin film 12 (see Fig. 2) is not formed on substrate 10.

In the construction shown in Fig. 18, a position of objective lens 22 is fixed. Adjustment mechanism 41 adjusts a position of XYZ-axis stage 40 on which substrate 10 is mounted, in the x direction, the y direction, and the z direction. For example, a drive mechanism such as a servo motor and a focusing knob equipped in a microscope can be employed as adjustment mechanism 41. A specific construction of adjustment mechanism 41 is not particularly limited. Adjustment mechanism 41 may adjust a position of objective lens 22 relative to fixed substrate 10.

Laser displacement meter 42 measures a distance in a vertical direction between laser displacement meter 42 and substrate 10 and measures displacement of substrate 10 in a horizontal direction. By using a result of measurement by laser displacement meter 42, a position of a laser spot of laser beams 201 can be adjusted. For example, adjustment mechanism 41 may adjust a position of XYZ-axis stage 40 based on a result of measurement by laser displacement meter 42. Laser displacement meter 42 is not an essential feature.

In the present modification, a structure of an assembly formed from fluorescent beads exhibiting two-photon absorption with respect to laser beams 201 is compared. Beads employed in the present modification are the same as beads 1 and 2 used in the first embodiment. Three types of samples (fourth to sixth samples) different in ratio between the number of beads 1 and the number of beads 2 are prepared. A volume of a liquid is set to 10 $\mu$L.

Beads 1 contain a bright blue fluorochrome (BB manufactured by Fluoresbrite). A maximum excitation wavelength of beads 1 is 365 nm.

Beads 2 contain a yellow orange fluorochrome (NYO manufactured by Fluoresbrite). A maximum excitation wavelength of beads 2 is 530 nm. Since a wavelength of photothermal light source 20 is 1064 nm as described above, it is approximately twice as long as the maximum excitation wavelength of beads 2. Therefore, in beads 2, a process of two-photon absorption may be brought about by laser beams 201. Though an example of the process of two-photon absorption is described in the present modification, a material bringing about a process of multiphoton absorption of 3 or more photons may be employed.

A ratio between the number of beads 1 and the number of beads 2 in the fourth sample is N1:N2 = $0.64\times10^7:3.3\times10^8$ = 1:51. A ratio between the number of beads 1 and the number of beads 2 in the fifth sample is N1:N2 = $1.2\text{x}10^7:2.9\times10^8$ = 1:25. A ratio between the number of beads 1 and the number of beads 2 in the sixth sample is N1:N2 = $2.3 \times 10^7 : 2.0 \times 10^8$ = 1:9.

A distance between objective lens 22 and the gas-liquid interface which is an interface between a liquid representing sample 13 and a gas around the liquid is adjusted such that a laser spot of laser beams 201 is located in the liquid and located in the vicinity of the gas-liquid interface. Furthermore, the laser spot of laser beams 201 is more preferably located in the liquid and in the vicinity of the gas-liquid interface. In an outer peripheral portion of a droplet, a thickness of the droplet is small and therefore evaporation of a dispersion medium is fast and a concentration of dispersed beads 1 and beads 2 is high. Since a probability of occurrence of the process of two-photon absorption is in proportion to a square of intensity of light, a probability of occurrence of the process of two-photon absorption is higher by introducing light high in optical intensity into the gas-liquid interface high in concentration where a large number of beads 2 are present.

In the present modification, a position of a laser spot of laser beams 201 is adjusted to 28 μm from the gas-liquid interface in the horizontal direction (the x direction and the y direction) by using 100x objective lens 22. In addition, in the height direction (the z direction), a position of the laser spot in the droplet is determined by adjusting a position of a beam waist at a height not greater than 10 μm from an upper surface of the substrate by lowering XYZ-axis stage 40 by 2 μm from a position on substrate 10 where a focus is achieved. Power of laser beams 201 is set to 2 W which is higher than power in the first embodiment. Power of laser beams 201 after passage through objective lens 22 and substrate 10 is approximately 0.4 W in the absence of sample 13. Adjustment of a position of a laser spot is not essential and laser beams 201 should only pass through sample 13.

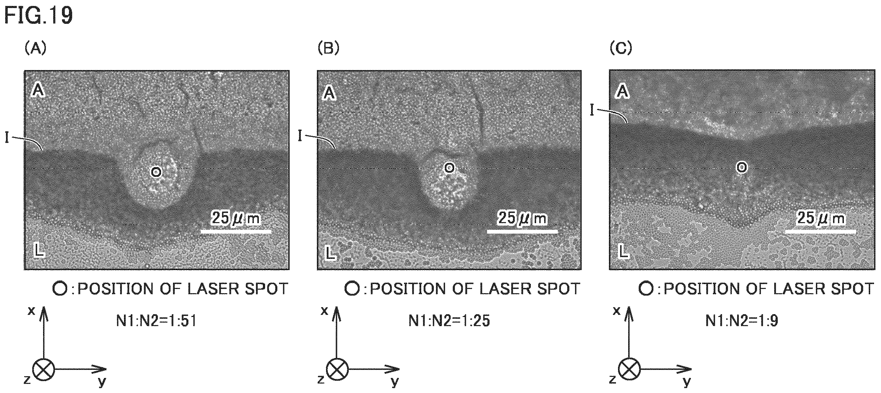

Fig. 19 shows optical photographs of assemblies at the gas-liquid interfaces of the respective fourth to sixth samples. Fig. 19(A) to (C) shows the assemblies of the respective fourth to sixth samples. An upper portion of an image (shown with A) represents a gas. A lower portion of the image (shown with L) represents a liquid. A dark portion (shown with I) between the liquid and the gas represents the gas-liquid interface.

Referring to Fig. 19 (A) to (C), the assembly shown in Fig. 19 (A) is greatest and the assembly shown in Fig. 19 (C) is smallest. As the number of beads 2 is larger or a ratio of the number of beads 2 to the number of beads 1 is higher, the assembly is greater. Since beads 2 absorb laser beams 201 in the process of two-photon absorption, an amount of absorption of light by beads 2 is greater with the larger number of beads 2 (or a higher ratio of the number of beads 2). Consequently, an amount of generation of heat by beads 2 increases. It is considered that as an amount of generation of heat is larger, convection is more likely to be produced and hence a large assembly is assembled.

In the liquid representing sample 13, a concentration of beads 1 and 2 is higher toward the gas-liquid interface from a bulk (a region around the center of the liquid distant from the gas-liquid interface). Therefore, a larger assembly can be formed by adjusting a position of a focal point of objective lens 22 at the gas-liquid interface.

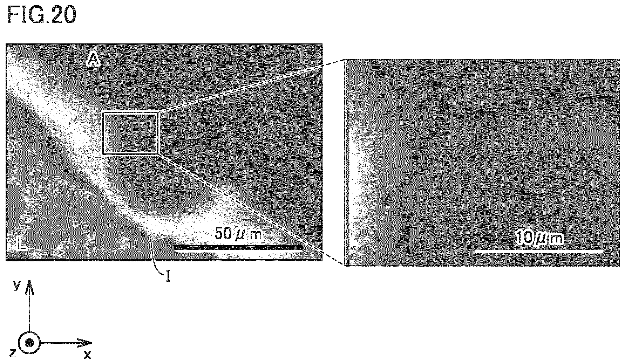

Fig. 20 shows an SEM image showing the assembly of the fourth sample as being enlarged. Referring to Fig. 20, bead 1 is shown to be buried in beads 2 at a position where a liquid has evaporated at the gas-liquid interface.

[Second Embodiment]

An apparatus for manufacturing a microscopic object assembly structure can be realized by using the assembling apparatus shown in the first embodiment. An example in which a two-component photonic structure formed from resin beads is manufactured will be described in a second embodiment. The apparatus for manufacturing a microscopic object assembly structure is similar in construction to assembling apparatus 100 shown in Figs. 1 and 2. A method for manufacturing a microscopic object assembly structure is similar to the operations in the flowchart shown in Fig. 4. Therefore, the construction of the apparatus for manufacturing a microscopic object assembly structure and a method for manufacturing the microscopic object assembly structure will not be repeated.

Referring back to Fig. 10 (A), as shown in an enlarged view in an upper right portion, when an assembly is formed with the 100x lens in the third sample, a structure in which beads 1 are periodically arranged is formed around a structure in which beads 1 are mainly assembled at high density. As a gap between bead 1 and another bead 1 closest to that bead 1 is filled with beads 2, beads 1 are periodically aligned. A period of a structure of beads 1 is several hundred nm, which is substantially the same as a wavelength of visible light. The apparatus for manufacturing a two-component photonic structure can thus be realized with the use of assembling apparatus 100. Formation of a similar two-component photonic structure can be confirmed also when an assembly is formed with the 10x lens as shown in an enlarged view in an upper right portion in Fig. 13 (A).

Thus, according to the second embodiment, a two-component photonic structure can be manufactured at a portion irradiated with laser beams. The photonic structure is a structure in which a dielectric having a size of the order of a wavelength of light is periodically disposed, and it has a property to intensely reflect or confine light at a specific wavelength based on variation in size, shape, index of refraction, and period of the dielectric employed as a constituent element. The two-component photonic structure means a photonic structure composed of two types of dielectrics different in size. In the present embodiment, as shown in Fig. 13, a two-component photonic structure like a concave lens is fabricated. Examples of an application of such a two-component photonic structure like a concave lens include a concave lens having wavelength selectivity. A wavelength-selective concave lens can be used, for example, as an optical communication element.

Since a size of a microbubble is varied by adjusting a material and a thickness of a thin film, a type of a liquid, a magnification of an objective lens, and intensity of laser beams, a size of a photonic structure can be controlled. A period of a photonic structure can be controlled by varying a type of beads (for example, a particle size or a shape). A photonic structure can thus be manufactured in accordance with various wavelengths.

[First Modification of Second Embodiment]

An example for forming a two-component photonic structure formed from resin beads has been described in the second embodiment. In the example described in the second embodiment, a sample is dried (naturally dried) until a liquid disappears as a result of natural evaporation after irradiation with light is stopped. In contrast, in a first modification, an example of vacuum drying a sample will be described. An apparatus for manufacturing a microscopic object assembly structure according to first and second modifications is similar in construction to assembling apparatus 100 shown in Figs. 1 and 2. A method for manufacturing a microscopic object assembly structure is similar to the operations in the flowchart shown in Fig. 4. Therefore, the construction of the apparatus for manufacturing a microscopic object assembly structure and a method for manufacturing a microscopic object assembly structure will not be repeated.

Referring again to Figs. 1 to 3, a sample containing both of beads 1 and 2 (the third sample) is employed in the present modification. A ratio between the number of beads 1 and the number of beads 2 is N1:N2 = 1:85 (see Fig. 7 (A)).

Power of laser beams 201 after passage through objective lens 22 (10x lens) and substrate 10 is 0.1 W in the absence of sample 13. Sample 13 is set in a not-shown dessicator after irradiation with light is stopped. A pressure in the dessicator is set to 0.02 MPa (= 152 Torr). After vacuum drying for approximately 40 minutes, sample 13 is taken out of the dessicator.

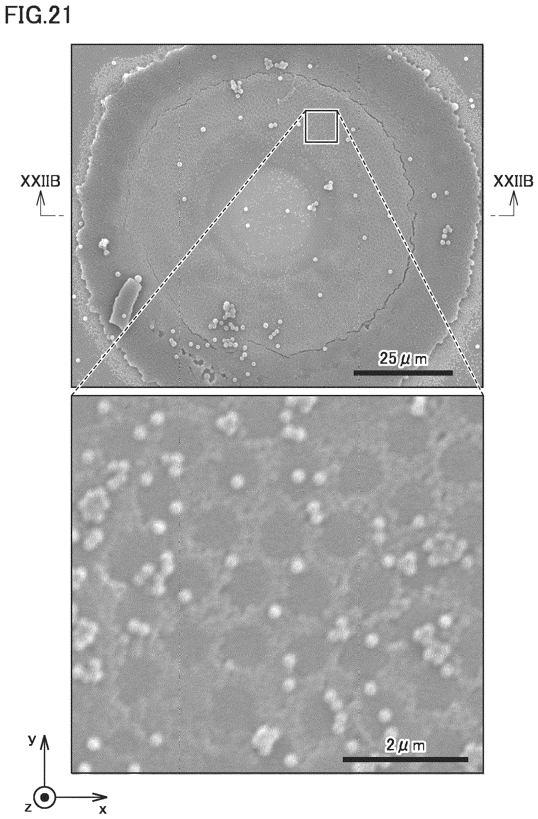

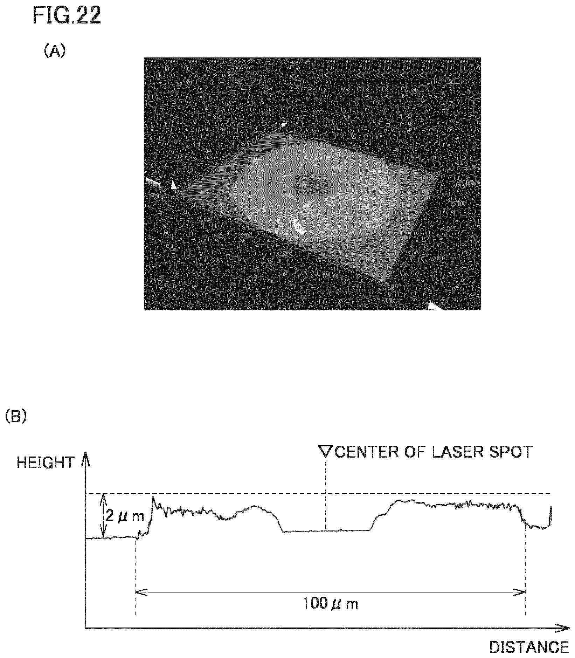

Fig. 21 shows an SEM image of an assembly formed when the third sample is vacuum dried. Fig. 22 is a diagram showing a three-dimensional shape and a cross-sectional shape of the assembly shown in Fig. 21. Fig. 22 (A) shows a result of determination of the three-dimensional shape of the assembly. Fig. 22 (B) shows a result of determination of the cross-sectional shape of the assembly along the line XXIIB-XXIIB in Fig. 21.

Referring to Figs. 21 and 22, when sample 13 is vacuum dried as well, an assembly in a ring shape is formed as in naturally drying of sample 13 (see Fig. 13). In this assembly, a gap between bead 1 and another bead 1 closest to that bead 1 is filled with beads 2, so that beads 1 are periodically aligned. A period of a structure of beads 1 is substantially the same as a wavelength of visible light (several hundred nm). Therefore, formation of a two-component photonic structure is confirmed. Furthermore, it can be seen from the result of determination of the cross-sectional shape that the assembly has a single-layered structure.

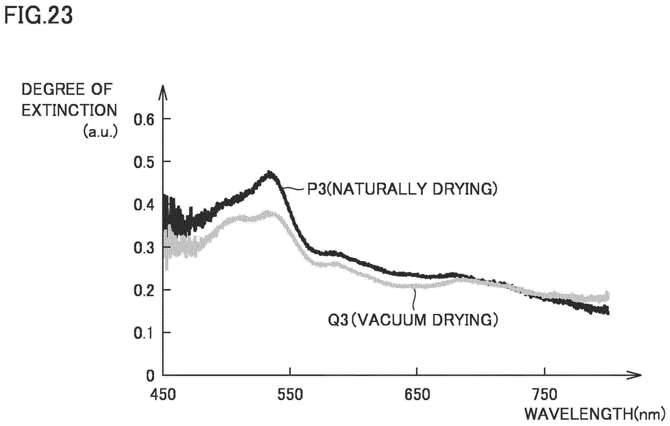

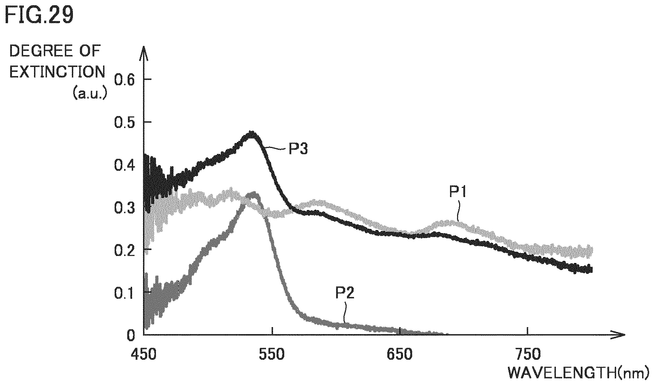

Fig. 23 is a diagram showing an extinction spectrum when the third sample is naturally dried without irradiation with light and an extinction spectrum when the third sample is vacuum dried after irradiation with light. In Fig. 23 and Fig. 29 which will be described later, the abscissa represents a wavelength and the ordinate represents a degree of extinction. A curve P3 represents an extinction spectrum after naturally drying. A curve Q3 represents an extinction spectrum after vacuum drying.

Referring to Fig. 23, the extinction spectrum after naturally drying and the extinction spectrum after vacuum drying each have a peak in a wavelength region from blue to green. Therefore, each of sample 13 after naturally drying and sample 13 after vacuum drying exhibits a structural color, because only light having a specific wavelength dependent on the photonic structure is reflected. It can thus also be confirmed that the assembly shown in Figs. 21 and 22 has the photonic structure.

When the third sample is naturally dried, the assembly has a diameter of approximately 70 $\mu$m and a height of approximately 10 $\mu$m (see Fig. 13 (B)). In contrast, when the third sample is vacuum dried, the assembly has a diameter of approximately 100 $\mu$m and a height of approximately 2 $\mu$m. The assembly is larger in diameter and smaller in height when sample 13 is vacuum dried than when sample 13 is naturally dried. Consequently, the assembly has a single-layered structure. This mechanism will be described.

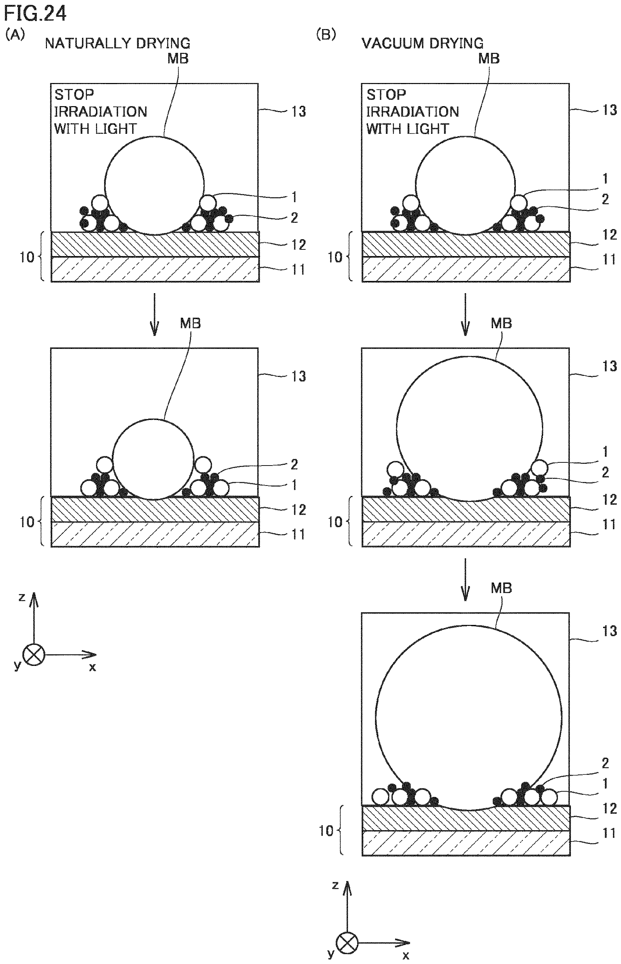

Fig. 24 is a schematic diagram for comparison between a mechanism in naturally drying after irradiation with light and a mechanism in vacuum drying after irradiation with light. As shown in Fig. 24 (A), when sample 13 is naturally dried, a diameter of microbubble MB does not much change after irradiation with light is stopped.

As shown in Fig. 24 (B), when sample 13 is vacuum dried, a diameter of microbubble MB is greater than a diameter before start of vacuum drying. This is because a pressure around sample 13 is reduced as a result of vacuum drying as compared with atmospheric pressure (that is, a pressure before start of vacuum drying).

This fact can be confirmed, for example, as follows. Sample 13 is irradiated with laser beams 201 for 1 minute. This sample 13 is set in the dessicator. When a time period for vacuum drying (a time period for drying) in the dessicator is relatively short, a liquid in sample 13 does not completely evaporate and microbubble MB can be observed in the liquid. Therefore, a diameter of microbubble MB in accordance with a duration of drying can be measured by obtaining an image of sample 13 with an optical microscope.

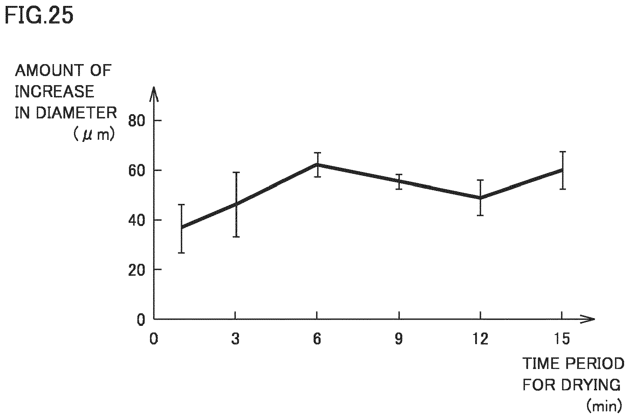

Fig. 25 is a diagram showing a result of measurement of a diameter of microbubble MB In Fig. 25, the abscissa represents a time period for drying sample 13. The ordinate represents an amount of increase in diameter with a diameter of microbubble MB at the time of stop of irradiation with light being defined as the reference.

Referring to Figs. 24 and 25, in the case of the third sample, as described in the first embodiment, a maximum value for a diameter of microbubble MB during irradiation with laser beams 201 is 61.0 $\mu$m. In contrast, microbubble MB during vacuum drying has a diameter approximately from 150 $\mu$m to 200 $\mu$m. It is thus confirmed that a diameter of microbubble MB increases as a result of vacuum drying.

As described above, during irradiation with laser beams 201, beads 1 and 2 are assembled in a region between microbubble MB and substrate 10 so that an assembly in a ring shape is formed. This assembly collapses toward a radius direction of the ring shape as a diameter of microbubble MB is greater as a result of vacuum drying. A height of the assembly thus becomes smaller. Consequently, the assembly is considered to have a single-layered structure.

In the example shown in Fig. 25, it can be seen that a diameter of microbubble MB increases with lapse of time for approximately 6 minutes since start of drying. Therefore, a size (a diameter) and a height of the photonic structure can be controlled by varying a time period for drying.

[Second Modification of Second Embodiment]

An assembly has been described to be formed at a position of irradiation with laser beams and a portion around the same in the second embodiment and the first modification thereof. When a droplet of a sample is dropped onto a substrate, however, an assembly can be formed also in a region at an edge of the droplet. In a second modification, a construction in which an assembly is formed at an edge of a droplet when the first to third samples (see Fig. 7 (A)) are employed will be described.

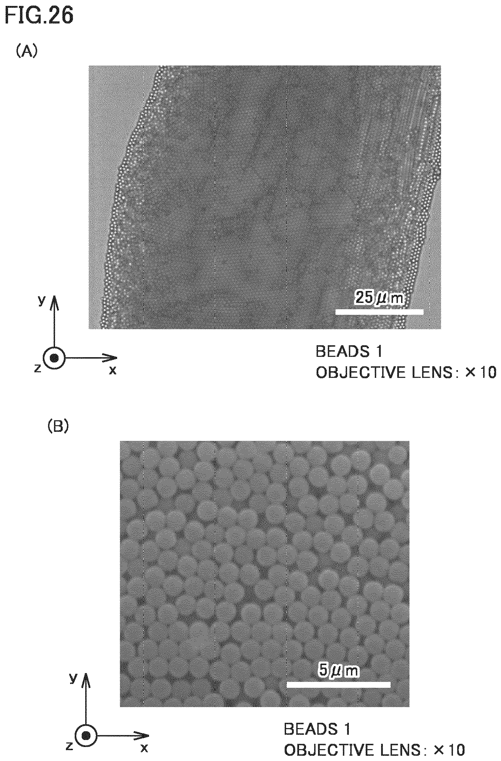

Figs. 26 to 28 show SEM images of assemblies formed in regions at edges of droplets in the first to third samples, respectively. Though an example in which each sample is naturally dried is shown below, the sample may be vacuum dried.

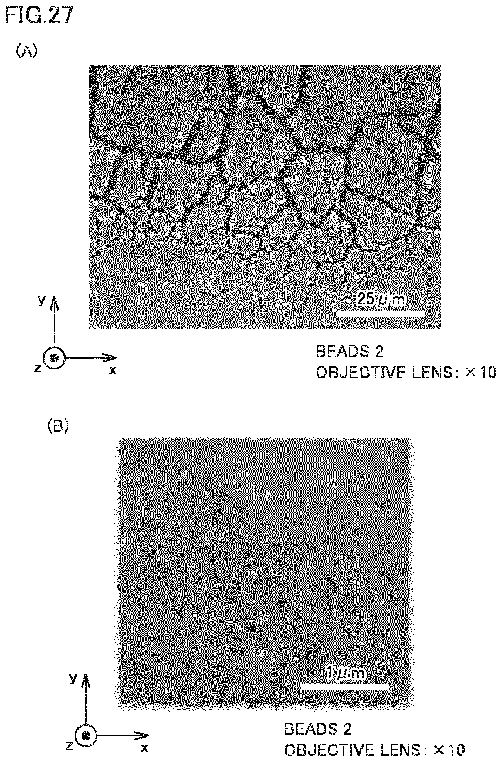

Referring to Figs. 26 and 27, in the first sample, an assembly formed from beads 1 is observed also at the edge of the liquid. In the second sample as well, an assembly formed from beads 2 is observed similarly to the first sample. The assembly formed from beads 1 and the assembly formed from beads 2 both have a hexagonal closest packing structure.

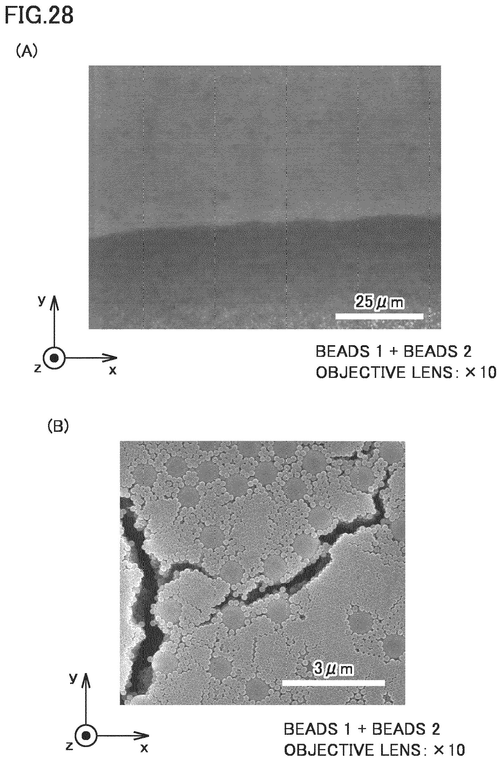

Referring to Fig. 28, in the third sample, a gap between bead 1 and another bead 1 closest to that bead 1 is filled with beads 2, so that a structure in which beads 1 are periodically arranged is formed. A period of the structure of beads 1 is substantially the same as a wavelength of visible light. Therefore, it can be seen that an assembly formed at the edge of the liquid when the third sample is employed also has a two-component photonic structure.

Fig. 29 is a diagram showing respective extinction spectra of the first to third samples. Curves P1 to P3 represent respective extinction spectra of the first to third samples. Referring to Fig. 29, it is confirmed that an assembly formed in the region at the edge of the droplet after naturally drying has the photonic structure.

Thus, when the sample containing both of beads 1 and 2 (the third sample) is irradiated with laser beams 201, a photonic structure is formed in a region in the vicinity of a laser spot (see Fig. 13 (A)). In addition, by naturally drying (or vacuum drying, not shown here) a sample, a photonic structure can be formed also in a region at the edge of the droplet of the sample.

[Third Embodiment]

A microscopic object may be derived from a living body. In a third embodiment, bacteria are assembled as one exemplary form of a microscopic object derived from a living body. Specifically, Pseudomonas aeruginosa representing bacteria in a rod shape is assembled. Since an assembling apparatus according to the third embodiment is similar in construction to assembling apparatus 100 shown in Figs. 1 and 2, description will not be repeated.

In the third embodiment, instead of beads 1 in the first embodiment, bacteria 3 (Pseudomonas aeruginosa) are employed. Pseudomonas aeruginosa used in the present embodiment has a width of $0.599\pm0.09$ $\mu$m. Pseudomonas aeruginosa has a length of $1.72\pm0.36$ $\mu$m. A volume ratio between Pseudomonas aeruginosa and bead 1 (having a diameter of 1.0 $\mu$m) is estimated as $(0.599\times0.599\times1.72):(1.0\times1.0\times1.0) = 0.617:1$. Pseudomonas aeruginosa is substantially the same in size as bead 1.

Sample 13 in which bacteria 3 and beads 2 (having a diameter of 0.2 $\mu$m) are dispersed is prepared as a seventh sample. The number N3 of bacteria 3 is $1.2\times10^6$. The number N2 of beads 2 is $1.0\times10^8$. A ratio between the number of bacteria 3 and beads 2 is N3 :N2 = 1:82. A volume of a liquid is set to 10 $\mu$L.

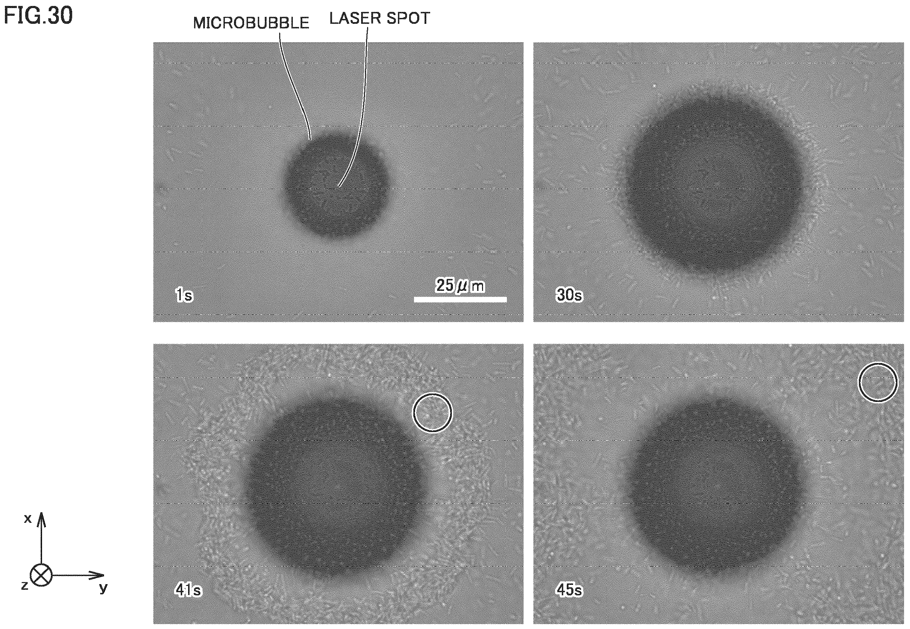

Fig. 30 shows successive photographs showing change over time of a region in the vicinity of a laser spot after start of irradiation with light in the seventh sample. Fig. 30 is compared with the third sample (see Fig. 5) containing beads 1 and 2. As in the case of the third sample, assembly of bacteria 3 in the vicinity of a laser spot is shown. A magnification of objective lens 22 is 100x.

When irradiation with laser beams 201 is stopped 41 seconds after start of irradiation with light, bacteria 3 (shown with a circle) move away from microbubble MB from after 41 seconds to after 45 seconds.

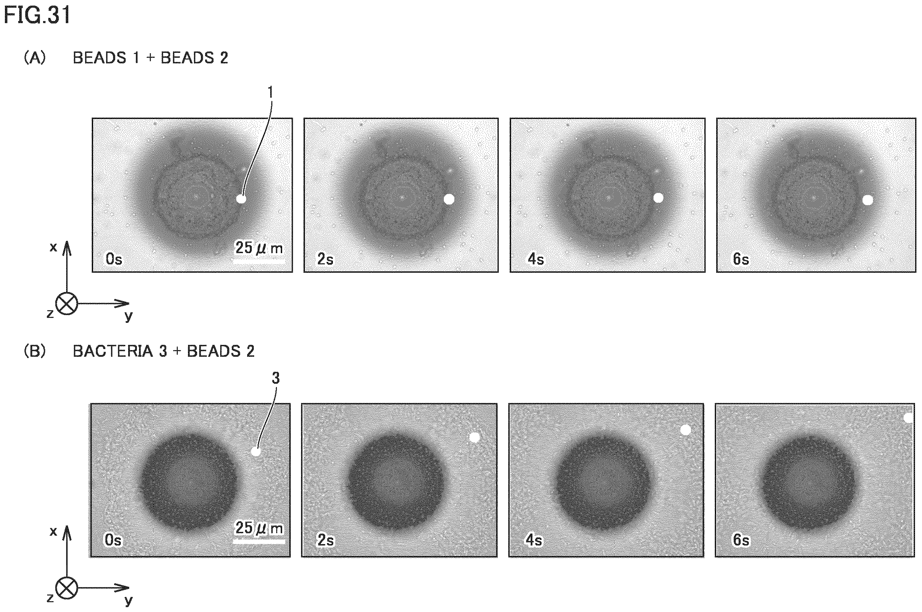

Fig. 31 shows successive photographs for comparison of change after irradiation with light is stopped, between the third sample and the seventh sample. Referring to Fig. 31, time shown in each photograph (such as 0 s or 2 s) represents lapse of time since stop of irradiation with laser beams 201 (unit: second).

Fig. 31 (A) shows a state of the third sample containing beads 1 and 2. A position of bead 1 does not substantially change even after irradiation of thin film 12 with light ends. With specifically noted bead 1 being shown with a circle, that bead 1 merely moves irregularly owing to Brownian movement. A distance of travel of bead 1 in 6 seconds shown in Fig. 31 (A) is approximately several μm.

In contrast, Fig. 31 (B) shows a state of the seventh sample containing Pseudomonas aeruginosa and beads 2. Diffusion of a population of bacteria 3 in a direction away from microbubble MB after irradiation of thin film 12 with light is stopped is shown. With specifically noted bacteria 3 being shown with a circle, rapid movement of bacteria 3 in a direction away from microbubble MB is shown. A distance of travel of bacteria 3 in 6 seconds shown in Fig. 31 (B) is approximately several ten μm. Such rapid collective diffusion of bacteria 3 is evidence which shows that at least some of assembled bacteria 3 are living.

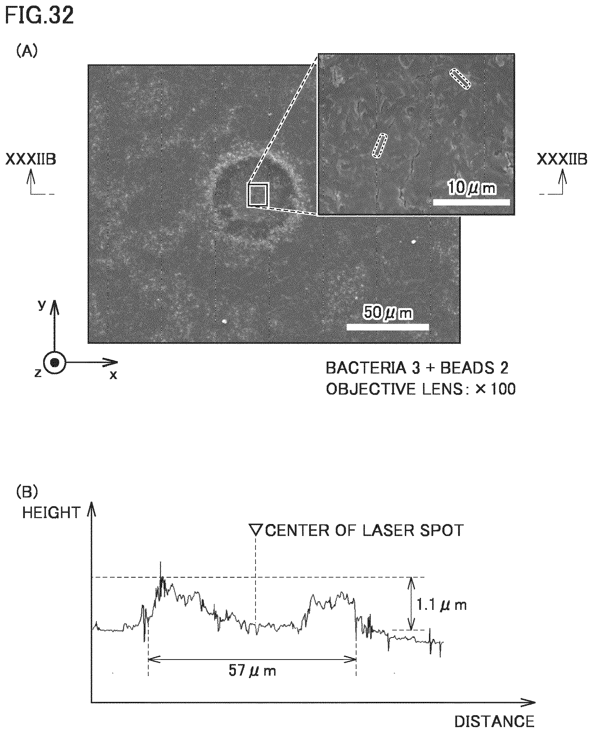

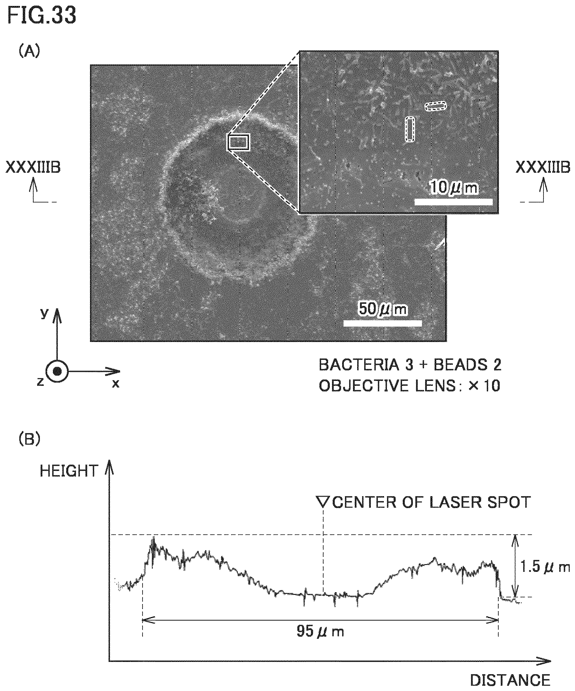

Fig. 32 is a diagram showing an SEM image of an assembly formed with the 100x lens and a result of determination of a cross-sectional shape of the assembly in the seventh sample. Fig. 33 is a diagram showing an SEM image of an assembly formed with the 10x lens and a result of determination of a cross-sectional shape of the assembly in the seventh sample. Figs. 32 and 33 are compared with Figs. 10 and 13, respectively.

Referring to Figs. 32 and 33, it is confirmed that an assembly in a ring shape is formed when bacteria 3 and beads 2 are assembled, as in assembly of beads 1 and beads 2. As shown with a dashed line in an enlarged view in Figs. 32 and 33, it can be seen that bacteria 3 are assembled such that a direction of a long axis of the rod shape is in parallel to a main surface of substrate 10 (a surface in parallel to the x direction and the y direction).

Pseudomonas aeruginosa is a bacterium harmful for humans which may be a cause for healthcare-acquired infection. Bacteria useful for humans, however, can also be assembled with a similar method.

For example, ultraoligotrophic bacteria (Rhodococcus erythropolis) which convert carbon dioxide into such a useful substance as methanol can be assembled. As described above, the ultraoligotrophic bacteria are assembled while they are living such that a direction of a long axis of the rod shape is in parallel to a main surface of substrate 10. Thus, a surface area of a population of bacteria (an area of a surface in parallel to the main surface of substrate 10) is larger than in assembly of the ultraoligotrophic bacteria while the direction of long axis of the rod shape of the ultraoligotrophic bacteria is perpendicular to the main surface of substrate 10. Therefore, when carbon dioxide flows over substrate 10, carbon dioxide can highly efficiently be fixed.

[Fourth Embodiment]

A microorganism harmful to humans can be removed by using the assembling apparatus described in the third embodiment.

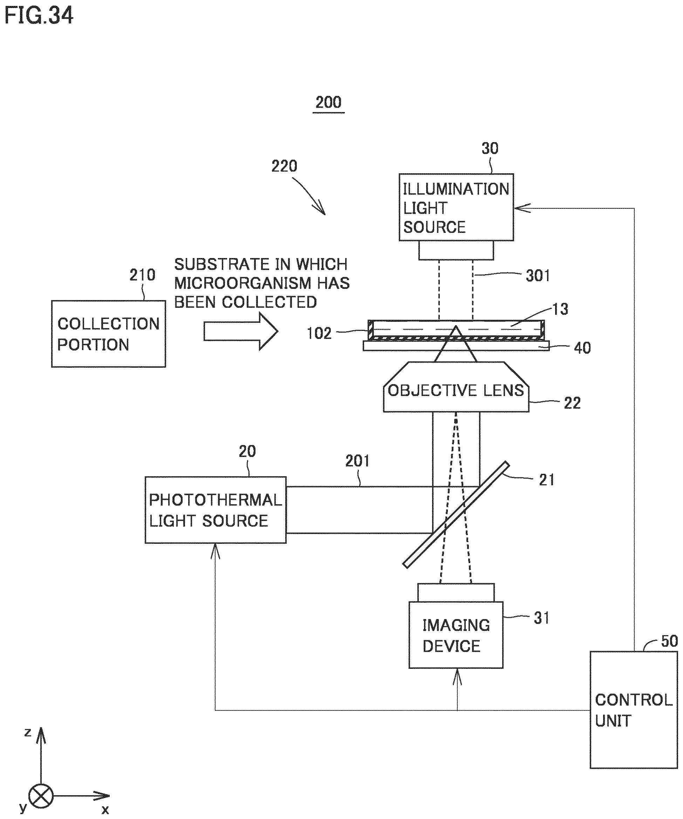

Fig. 34 is a diagram showing a schematic construction of an apparatus for assembling and removing a microorganism according to a fourth embodiment of the present invention. Referring to Fig. 34, an apparatus 200 for assembling and removing a microorganism includes a collection portion 210 and a removal portion 220.

Collection portion 210 collects a microorganism floating in air. Removal portion 220 assembles the microorganism collected by collection portion 210 through irradiation with light and removes the microorganism. Since removal portion 220 is similar in construction to assembling apparatus 100 shown in Fig. 1, detailed description will not be repeated.

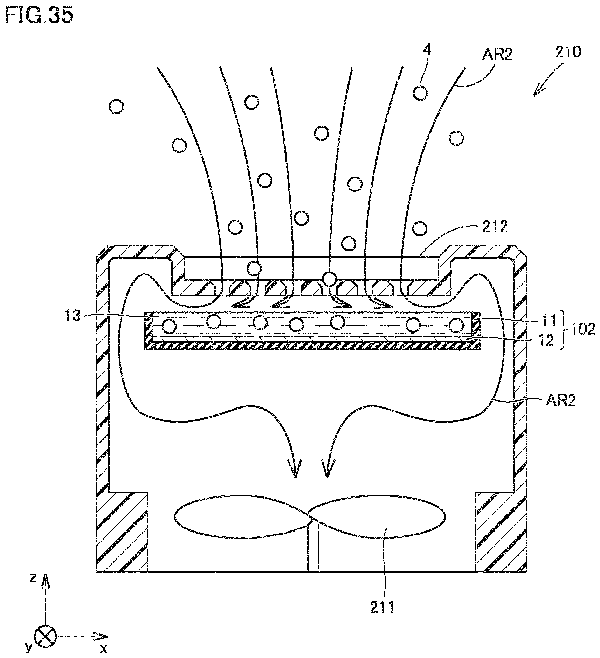

Fig. 35 is a diagram showing one example of a construction of collection portion 210 shown in Fig. 34. Referring to Figs. 34 and 35, as a fan 211 is rotated by a not-shown motor, a certain amount of air is suctioned through a suction port 212 of collection portion 210. A microorganism 4 floating in air is suctioned together with air and blown to a substrate 102 set in collection portion 210. Substrate 102 holds a liquid representing sample 13. Microorganism 4 floating in air is thus collected in the liquid representing sample 13. A method of collecting microorganism 4 is not limited to the technique above, and various known techniques can be adopted.

When microorganism 4 is collected in the liquid representing sample 13, substrate 102 is set at a prescribed position on XYZ-axis stage 40 of removal portion 220. Removal portion 220 irradiates substrate 102 with laser beams 201. The liquid in the vicinity of a laser spot reaches a high temperature. More specifically, it is confirmed that albumin is solidified by irradiation with light when albumin is dispersed in a liquid (water in the present embodiment). Since a solidification temperature of albumin is from 75 to 78°C, it can be seen that some of the liquid in the vicinity of the laser spot is approximately at 80°C or higher. Thus, an effect of removal of a microorganism through a high-temperature treatment in the vicinity of the laser spot can be obtained while microorganism 4 is assembled in the vicinity of surfaces of air bubbles through irradiation with light.

[Fifth Embodiment]

PM 2.5 representing environment contamination particles may be inhaled deeply into lungs when it is suctioned in a human body. Therefore, influence of PM 2.5 on a respiratory system and a circulatory system is concerned. In order to know a state of diffusion of PM 2.5, a technology to quickly measure a mass concentration (unit: $\mu$g/m$^3$) of PM 2.5 in atmosphere in a simplified manner has been demanded. A detection apparatus which detects PM 2.5 with the assembling apparatus in the first embodiment will be described in a fifth embodiment.

Air may contain not only PM 2.5 but also PM different in particle size (for example, PM 10). Therefore, a general apparatus for detecting PM 2.5 includes a size classifier for separating PM 2.5 from PM different in particle size. For example, in a detection apparatus including a cyclone type size classifier, an air suction port should be set at a height approximately several meters from a surface where the apparatus is set. Therefore, it may be difficult to secure a location for setting the apparatus.

The detection apparatus shown in the present embodiment can assemble PM 10 and PM 2.5 as being separated from each other as a result of irradiation with light. A mass concentration of PM 2.5 can thus be calculated without providing a size classifier. Therefore, the detection apparatus can be reduced in size.

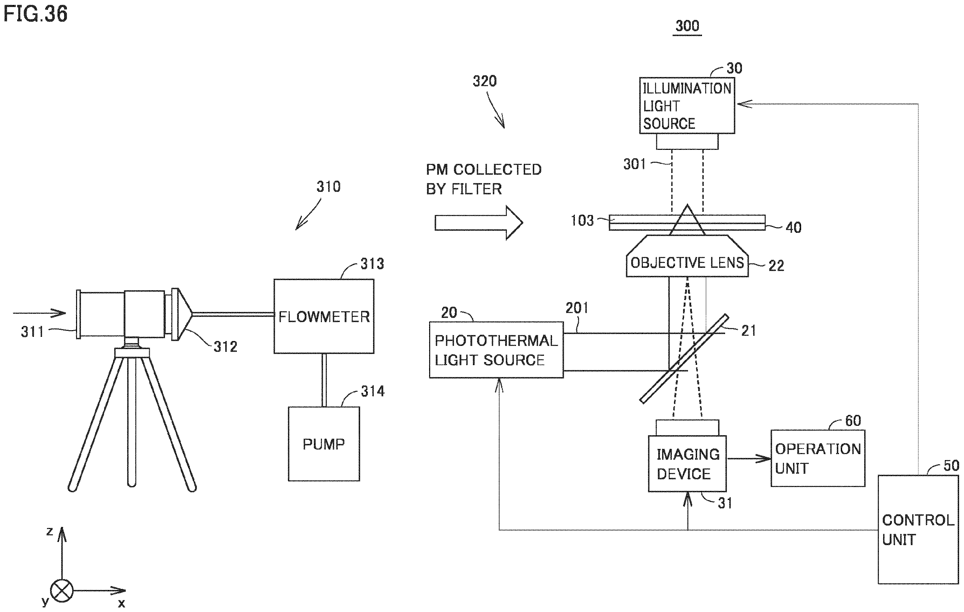

Fig. 36 is a diagram showing a schematic construction of the detection apparatus according to the fifth embodiment of the present invention. Referring to Fig. 36, a detection apparatus 300 includes an air sampler 310 and a detection portion 320.

Air sampler 310 includes a suction port 311, a filter 312, a flowmeter 313, and a pump 314. When pump 314 is activated, air is suctioned through suction port 311. Flowmeter 313 measures an amount of suctioned air. Filter 312 is provided in a flow path from suction port 311 to pump 314. Filter 312 collects PM contained in suctioned air. PM collected by filter 312 is dispersed in a liquid on a substrate 103 of detection portion 320. Since detection portion 320 is similar in construction to assembling apparatus 100 shown in Fig. 1, detailed description will not be repeated.

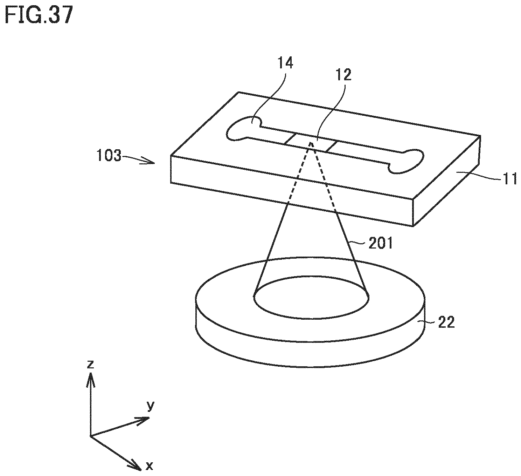

Fig. 37 is an enlarged perspective view of a construction of detection apparatus 300 shown in Fig. 36 around substrate 103. Referring to Fig. 37, a micro flow path 14 is formed in a surface of substrate 103. For example, S-BIO SUMILON BS-21102 manufactured by Sumitomo Bakelite Co., Ltd. can be employed as substrate 103. Thin film 12 is formed in at least a part of micro flow path 14. Laser beams 201 gathered by objective lens 22 are emitted to thin film 12 formed in micro flow path 14.

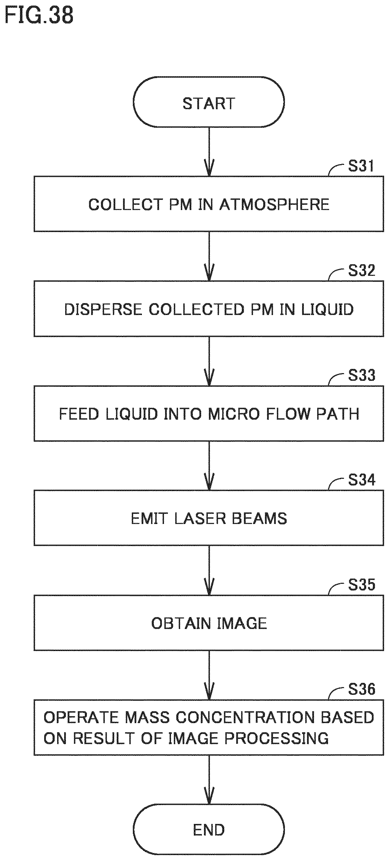

Fig. 38 is a flowchart for illustrating a method of detecting PM 2.5 according to the fifth embodiment of the present invention. Referring to Figs. 36 to 38, in step S31, air sampler 310 suctions, for example, approximately several liters (1 L to 10 L) of air. PM contained in suctioned air is thus collected by filter 312. PM collected by filter 312 is separated from filter 312 and dispersed in a liquid (step S32). Various known extraction methods may be used as a technique to separate PM from filter 312, or PM may be shaken off by vibrating filter 312.

In step S33, a dispersion liquid in which PM is dispersed is fed to micro flow path 14. When thin film 12 formed in micro flow path 14 is irradiated with laser beams 201, microbubbles MB are produced as a result of the photothermal effect. An assembly of PM is thus formed (step S34).

When a width of micro flow path 14 is greater than a size (a diameter) of a laser spot, PM dispersed in the liquid may flow through micro flow path 14 without passing through the laser spot. Therefore, relation in terms of a size between a width of micro flow path 14 and a diameter of a laser spot is preferably determined such that the width of micro flow path 14 is smaller than a size of a laser spot. Since PM which flows through micro flow path 14 without passing through a laser spot thus decreases, PM can highly efficiently be assembled.

Then, control unit 50 controls shooting instrument 31 so as to shoot an assembly of PM (step S35). An image shot by shooting instrument 31 is output to an operation unit 60. Operation unit 60 is implemented, for example, by a microcomputer. Operation unit 60 operates a mass concentration of PM in atmosphere based on a result of processing of images shot by shooting instrument 31 (step S36). During shooting by shooting instrument 31, irradiation with laser beams 201 may be continued or shooting may be carried out after irradiation with laser beams 201 is stopped.

An example of operation of a mass concentration of PM by operation unit 60 will be described. For example, in the image shown in Fig. 10, beads 2 small in size (having a diameter of 0.2 $\mu$m) are mainly assembled in the central portion in the vicinity of the laser spot and beads 1 large in size (having a diameter of 1.0 $\mu$m) are mainly assembled around the same. When PM is assembled through irradiation with light as well, as in the case of beads, a region of PM 2.5 is formed in the central portion of the laser spot and a region of PM 10 is formed to surround the central portion. By thus subjecting an image of an assembly in which PM 2.5 and PM 10 are separate from each other to image processing, the region of PM 2.5 is identified. For example, a boundary between these regions can be extracted based on a difference in brightness between the region of PM 2.5 and the region of PM 10.

As an amount of assembly of PM 2.5 is greater, transmitted light of white light 301 for illumination decreases and a color of the image is darker. A memory (not shown) of operation unit 60 holds in advance correspondence between brightness of images and the number of PM 2.5. Therefore, the number of PM 2.5 contained in each small region resulting from division of the region of PM 2.5 can be calculated for each such region based on brightness of the image in that region. By integrating the number of PM 2.5 in each small region, the total number of PM 2.5 in the entire region is calculated. Since an average mass of PM 2.5 has already been known, the total mass (unit: $\mu$g) of PM 2.5 is found by multiplying the total number of PM 2.5 by the average mass. Then, a mass concentration (unit: $\mu$g/m$^3$) of PM 2.5 can be calculated by dividing the total mass of PM 2.5 by an amount of suctioned air (unit: m$^3$).

Thus, according to the present embodiment, PM dispersed in a liquid is assembled through irradiation with light. In this assembly, PM 2.5 and PM 10 are separate from each other. Therefore, since it is not necessary to separate PM 2.5 with the use of a cyclone type size classifier, a detection apparatus can significantly be reduced in size. Since a time period for separating PM 2.5 with a size classifier is obviated, a time period for detection can be shortened.

[Sixth Embodiment]

In a sixth embodiment, the assembling apparatus is applied to a separation apparatus which separates a separation target substance. A flow cytometer will be described here as one exemplary form of the separation apparatus. Cells are adopted as a microscopic object in the present embodiment.

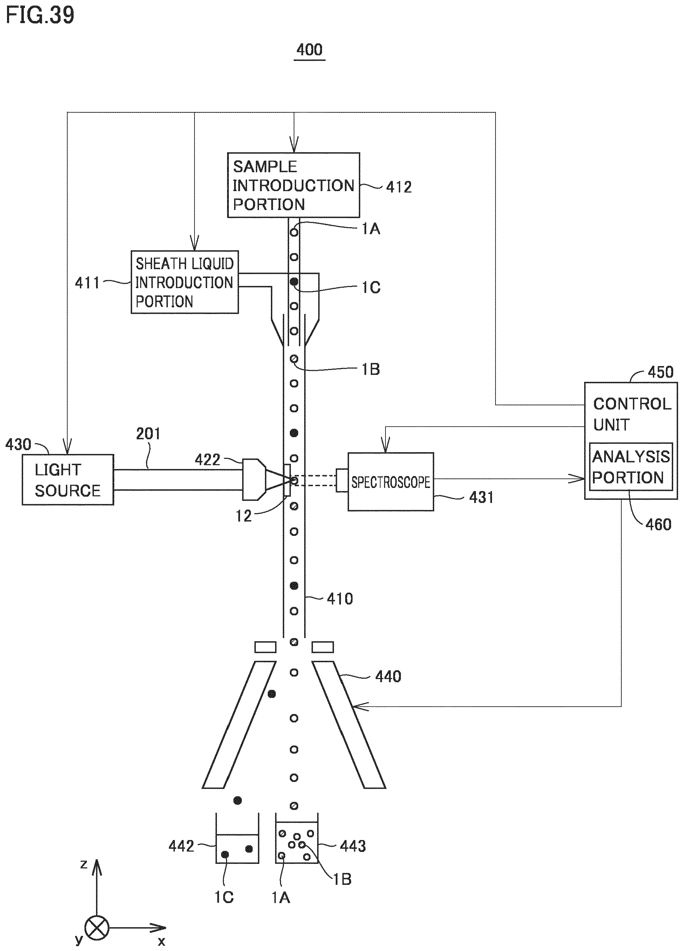

Fig. 39 is a diagram showing a schematic construction of a flow cytometer according to the sixth embodiment of the present invention. Referring to Fig. 39, a flow cytometer 400 includes a flow cell 410, a sheath liquid introduction portion 411, a sample introduction portion 412, an objective lens 422, a light source 430, a spectroscope 431, a cell sorter 440, and a control unit 450.

Light source 430 generates, for example, red laser beams 201 (for example, at a wavelength of 635 nm). Light source 430 serves as a photothermal light source and a spectroscopic light source. Light source 430 corresponds to both of the "light source" and the "spectroscopic light source" according to the present invention. The flow cytometer may include a photothermal light source and a spectroscopic light source separately from each other.

Sheath liquid introduction portion 411 introduces a sheath liquid into flow cell 410. A flow of the sheath liquid is controlled so as to form a laminar flow kept at a prescribed current velocity. Sample introduction portion 412 introduces into flow cell 410, a sample liquid in which cells 1A, 1B, and 1C are dispersed. Cell 1C is defined here as a separation target substance.

Objective lens 422 is arranged in a flow path in flow cell 410. Objective lens 422 gathers laser beams 201 from light source 430. Thin film 12 is formed in a part of a side surface of the flow path in flow cell 410. Light gathered by objective lens 422 is emitted to thin film 12 and the sample liquid. Emitted light assembles cells in the sample liquid and is incident on spectroscope 431 as transmitted light or scattered light as shown with a dashed line.

Cell sorter 440 for separating a separation target substance is provided at an exit end of flow cell 410. Cell sorter 440 charges a droplet dropped from the exit end of flow cell 410. Control unit 450 controls sheath liquid introduction portion 411, sample introduction portion 412, light source 430, spectroscope 431, and cell sorter 440.

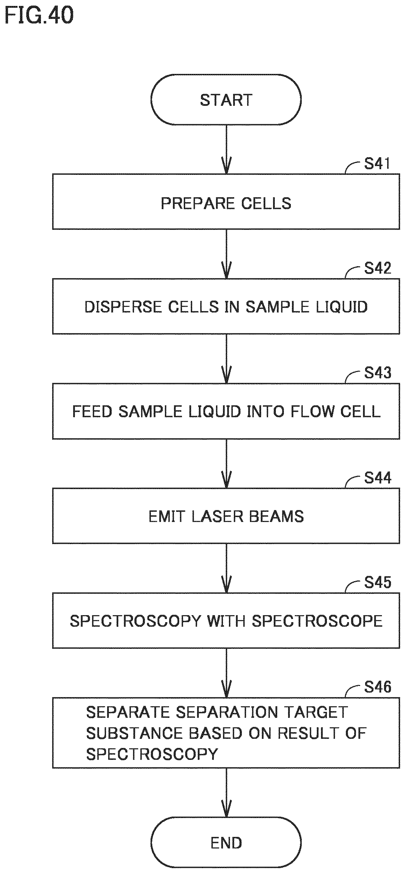

Fig. 40 is a flowchart for illustrating a method of separating cells representing a separation target substance according to the sixth embodiment of the present invention. Referring to Figs. 39 and 40, initially, cells 1A, 1B, and 1C are prepared (step S41). Then, cells 1A, 1B, and 1C are dispersed in the sample liquid (step S42). Cells 1A, 1B, and 1C in the sample liquid are successively fed through flow cell 410 in which a laminar flow is formed (step S43).

As laser beams 201 gathered by objective lens 422 are introduced in the sample liquid which flows through thin film 12 and the flow path in flow cell 410, cells in the sample liquid (any one type of cells among cells 1A, 1B, and 1C) are assembled to form an assembly of cells (step S44).

Spectroscope 431 subjects transmitted light or scattered light from the assembly to spectroscopy and outputs a result of spectroscopy to an analysis portion 460 in control unit 450 (step S45). A memory (not shown) of analysis portion 460 holds in advance spectroscopy data (spectra) for each of cells 1A, 1B, and 1C. Analysis portion 460 analyzes whether or not the assembly is composed of a separation target substance (cells 1C) by comparing the result of spectroscopy from spectroscope 431 with spectroscopy data held in the memory.

When a result of analysis that the assembly is composed of the separation target substance is obtained, control unit 450 controls cell sorter 440 such that electric field is applied to a droplet containing the assembly when the droplet falls. A direction of drop of the droplet is thus changed so that the droplet falls into a container 442. Therefore, the assembly composed of cells 1C is sorted into container 442.

When a result of analysis that the assembly is not composed of the separation target substance is obtained, control unit 450 controls cell sorter 440 such that electric field is not applied to the droplet containing that assembly. Since the droplet thus freely falls into a container 443, the assembly composed of cells 1A or cells 1B is collected in container 443 (step S46).

By thus applying the assembling apparatus according to the first embodiment to the flow cytometer, cells which flow through a flow cell can be assembled through irradiation with light. As cells are assembled and thus a target of spectroscopy is greater in size, accuracy in analysis of a result of spectroscopy is improved. Therefore, accuracy in separation of a separation target substance can be improved.

[Seventh Embodiment]

In the first to sixth embodiments, a liquid is described to be heated by making use of the photothermal effect of thin film 12 (thin gold film) formed on cover glass 11 (see Fig. 2). The technique for heating a liquid is not limited as such. In a seventh embodiment, a liquid is heated by making use of the photothermal effect of metallic nanoparticles dispersed in the liquid.

In the present embodiment, gold nanoparticles are adopted as metallic nanoparticles. The metallic nanoparticles may have an average particle size from the sub nanometer order to the nanometer order (approximately from 2 nm to 1000 nm), for example, from 2 nm to 500 nm, preferably from 2 nm to 100 nm, and more preferably from 5 nm to 50 nm. In the present embodiment, the gold nanoparticles have a particle size of 30 nm.

Particles for heating a liquid based on the photothermal effect (photothermal conversion particles) are not limited to gold nanoparticles. Examples of other metallic nanoparticles include silver nanoparticles and copper nanoparticles. The photothermal conversion particles are not limited to metallic nanoparticles, and may be composed, for example, of carbon black.

In the first embodiment, beads 1 and 2 which move over convection have been described to stay in the vicinity of the stagnation region provided by microbubble MB In the seventh embodiment, as will be described in detail below, a plurality of beads 1 are captured by light-induced force resulting from laser beams 201. Captured beads 1 provide a stagnation region.

<Optical Trapping of Beads and Gold Nanoparticles>

Optical trapping by using light-induced force resulting from laser beams 201 will be described. In the present invention and embodiments thereof, "light-induced force" is used as a generic denotation of dissipative force, gradient force, and light-induced force between substances. Dissipative force refers to force produced by providing a momentum of light to a substance in a dissipative process such as light scattering or light absorption. Gradient force refers to force which moves a substance to a stability point of an electromagnetic potential when a substance in which light-induced polarization is caused is placed in non-uniform electromagnetic field. Light-induced force between substances refers to the sum of force resulting from longitudinal electric field and transverse electric field (radiation field) produced by induced polarization in a plurality of substances excited by light.

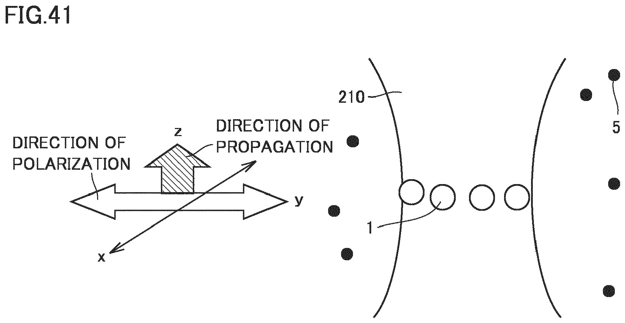

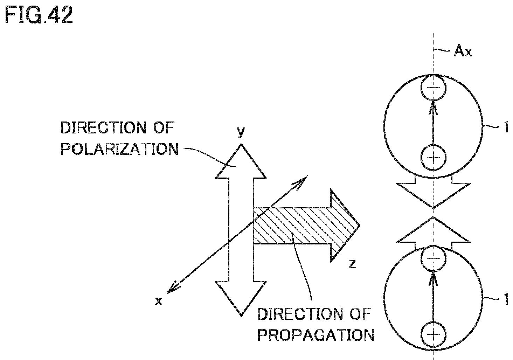

Fig. 41 is a schematic diagram for illustrating a mechanism of capture of a plurality of beads 1. Fig. 42 is a diagram for illustrating a 2-particle model of beads 1. Referring to Figs 41 and 42, a plurality of beads 1 are dispersed in a liquid.

Each of the plurality of beads 1 receives laser beams 201. A direction of polarization of laser beams 201 is in the y direction. The direction of polarization of laser beams 201 is in a direction substantially in parallel to an axis Ax connecting centers of beads 1. In this case, electric polarization is produced in each of beads 1 along a direction in parallel to the direction of polarization of laser beams 201. Thus, each bead 1 is captured in the vicinity of a laser spot of laser beams 201, which is a stability point of an electromagnetic potential. Orientations of electric polarization in all beads 1 are the same. Therefore, as shown in Fig. 42, attraction force is generated between beads 1 proximate to each other.

Since beads 1 are spherical, response optical electric field can be expressed as an integrated form of the Maxwell's equations. Response electric field $E_i$ is expressed in accordance with an expression (1) below:

$$\mathbf{E}_i = \mathbf{E}_i^0 + \sum_{\substack{j=1 \\ j \neq i}}^{N_p} \mathbf{G}_{\mathbf{r}, \mathbf{r}', \omega}(i - j) \cdot \mathbf{P}_j V_j + \mathbf{M}_i \cdot \mathbf{P}_i - \frac{\mathbf{L} \cdot \mathbf{P}_i}{k^2} \qquad \dots (1)$$

where i and j represent particle numbers of spherical cells. $V_j = V$ represents a volume of a spherical cell. M and L each represent an amount relating to self-interaction. Susceptibility and distribution of electric field in an individual bead are assumed to be flat. Induced polarization $P_i$ is expressed in accordance with an expression (2) below.

$$\mathbf{P}_i = \chi_i(\omega) \mathbf{E}_i \qquad \dots (2)$$

The Drude model is applied to susceptibility $\chi$. Susceptibility $\chi$ is expressed in accordance with an expression

(3) below.

$$\chi = \chi_b - \frac{\omega_p^2}{\omega^2 + i\omega(\gamma + v_f / a)} \qquad \ldots (3)$$

$\chi_b$ represents susceptibility of a background, $\omega_p$ represents plasma energy, $\gamma$ represents a non-radiant relaxation constant, $V_f$ represents an electron speed on a Fermi surface, and a represents a radius of a spherical cell. Non-radiant relaxation constant $\gamma$ is a value indicating relaxation from electrons excited by light to a state other than light (for example, a state converted to heat by means of phonons).

Light-induced force is generally expressed in an expression (4) below (T. Iida and H. Ishihara, Phys. Rev. B77, 245319 (2008)).

$$\langle \mathbf{F} \rangle = \frac{1}{2}\mathrm{Re}\left\{ \sum_\omega \int_V dr \left[ \nabla \mathbf{E}(\mathbf{r}, \omega)^* \right] \cdot \mathbf{P}(\mathbf{r}, \omega) \right\} \qquad \ldots (4)$$

Induced polarization $P_i$ and response electric field $E_i$ represented in accordance with the expression (2) and the expression (3), respectively, are substituted into the expression (4). Light-induced force is thus expressed in accordance with an expression (5) below.

$$\langle \mathbf{F}_i \rangle = \frac{1}{2}\mathrm{Re}\left\{ \left[ \nabla \mathbf{E}^{(0)}(\mathbf{r}_i, \omega)^* \right] \cdot \mathbf{P}(\mathbf{r}_i, \omega) \cdot V \right\}$$

$$+ \frac{1}{2}\mathrm{Re}\left\{ \left[ \nabla_{ij} \mathbf{G}(\mathbf{r}_i)^* \cdot \mathbf{P}_j^* \cdot V \right] \cdot \mathbf{P}_i \cdot V \right\} \qquad \ldots (5)$$

$E^{(0)}$ represents electric field of incident light and G represents the Green's function. The first term in the right side of the expression (5) represents the sum of dissipative force and gradient force of incident light and the second term represents light-induced force between substances. Since gradient force of incident light is in proportion to light intensity gradient $\nabla|E^{(0)}|^2$, such a situation that gradient force exceeds dissipative force by intense gathering of incident light can be produced and optical trapping is enabled. Light-induced force between substances is in proportion to light intensity. Therefore, gradient force and light-induced force between substances can be adjusted by controlling a light intensity gradient and light intensity of incident light.

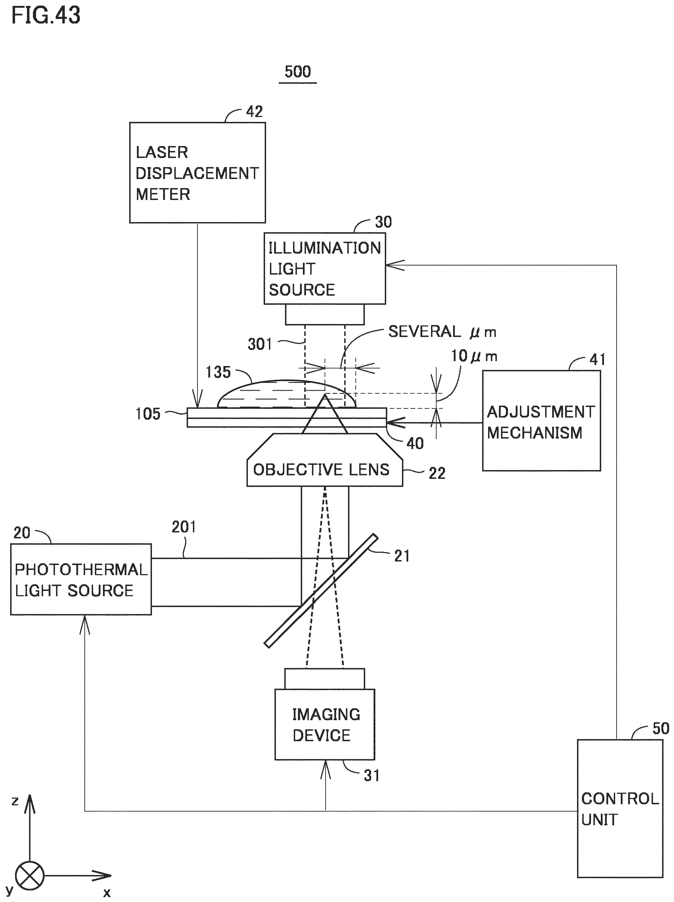

Fig. 43 is a diagram showing a schematic construction of an assembling apparatus according to the seventh embodiment of the present invention. Referring to Fig. 43, an assembling apparatus 500 is different from assembling apparatus 100 shown in Fig. 1 in including a substrate 105 instead of substrate 10. Power of laser beams 201 emitted from photothermal light source 20 is 0.1 W. Power of laser beams 201 after passage through objective lens 22 is 0.019 W. A 100x lens is employed as objective lens 22. Since the construction is otherwise similar to the corresponding construction of assembling apparatus 100, detailed description will not be repeated. An assembling method is similar to the operations in the flowchart shown in Fig. 4.

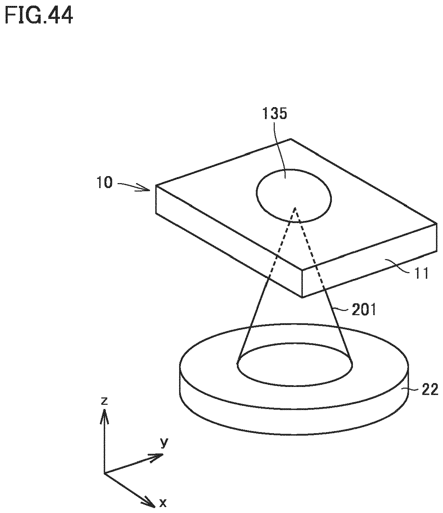

Fig. 44 is an enlarged perspective view of a construction of assembling apparatus 500 shown in Fig. 43 around substrate 105. Referring to Fig. 44, substrate 105 is different from substrate 10 shown in Fig. 2 in that thin film 12 (thin gold film) is not formed on cover glass 11.

Substrate 105 holds a sample 135. Sample 135 is a liquid in which beads 1 and gold nanoparticles 5 are dispersed. Though a type of a liquid (a dispersion medium) is not particularly limited, water (ultrapure water) is employed in the present embodiment. In the present embodiment, four types of samples (eighth to eleventh samples) are prepared as sample 135. A volume of each liquid is set to 10 µL. A region in the vicinity of an edge of a droplet is irradiated with laser beams.

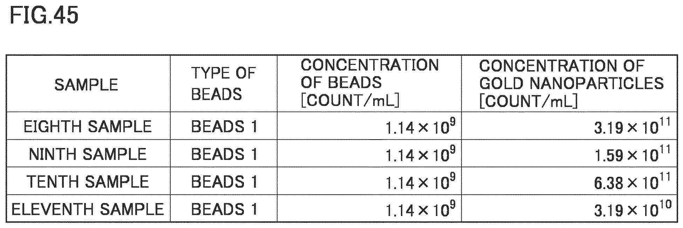

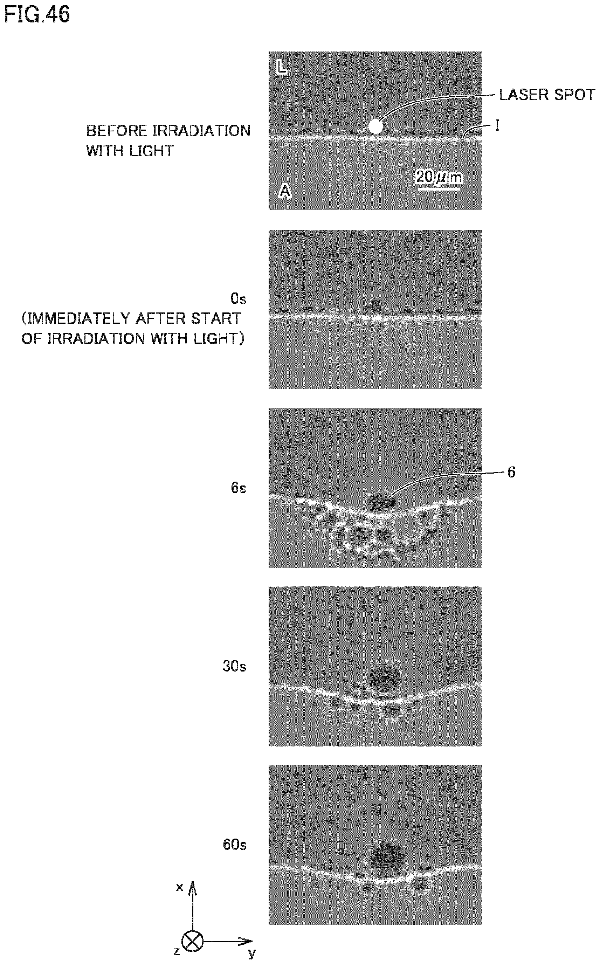

Fig. 45 is a diagram for illustrating samples 135 used in Figs. 46 to 49. Referring to Fig. 45, the eighth to eleventh samples are common in concentration of beads 1, which is $1.14 \times 10^9$ (counts/mL). A concentration of gold nanoparticles 5 (hereinafter also simply denoted as a "gold nanoparticle concentration") is lower in the order from the eight to eleventh samples.

<Assembly of Beads and Mechanism of Assembly>

Fig. 46 shows successive photographs showing change over time of a region in the vicinity of a laser spot resulting from irradiation with light in the eighth sample. Referring to Figs. 43 and 46, a state of the gas-liquid interface shot with shooting instrument 31 is shown, with the time of start of irradiation with laser beams 201 being defined as 0 second. A dark color portion (shown with L) on an upper side of each photograph represents a liquid, although it is shown only in a photograph before start of irradiation with light for avoiding being complicated. A light color portion (shown with A) on a lower side of each photograph represents a gas. A white portion (shown with I) between the liquid and the gas represents the gas-liquid interface.

A laser spot is adjusted in the vertically upward direction (the z direction) by 10 $\mu$m from a surface of substrate 105 in the vicinity of the gas-liquid interface of the liquid (for example, within several $\mu$m from the gas-liquid interface) (see Fig. 43). Irradiation with light is stopped at the time point of lapse of 110 seconds since start of irradiation with light.

In the example shown in Fig. 46, formation of an assembly 6 around the laser spot is clearly observed 6 seconds after the time of start of irradiation with light. With further continued irradiation with light, growth of assembly 6 is observed with lapse of time. When approximately 40 seconds elapse after start of irradiation with light, a speed of growth of assembly 6 becomes gradual. On the lower side of each photograph, annular portions dark in color observed on a side of the gas relative to the gas-liquid interface represent bubbles.

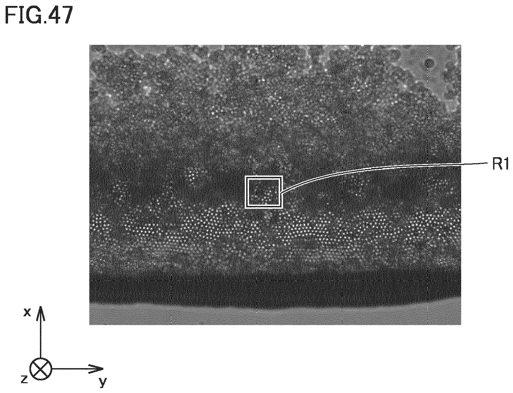

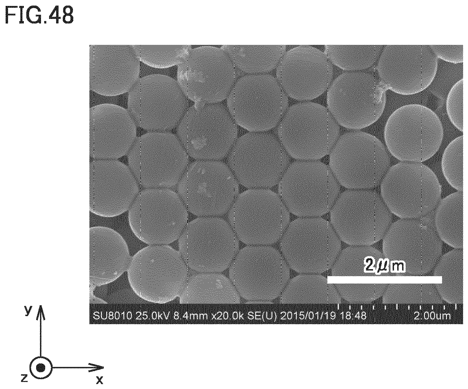

Fig. 47 shows an optical photograph of assembly 6 formed in a tenth sample which is naturally dried after irradiation with light is stopped. Fig. 48 shows an SEM image of a region R1 (a region in the vicinity of a laser spot) shown in Fig. 47. It can be seen with reference to Figs. 47 and 48 that beads 1 are assembled in the vicinity of the laser spot to thereby form a hexagonal closest packing structure. Deposition of gold nanoparticles 5 in a gap between beads 1 is observed.

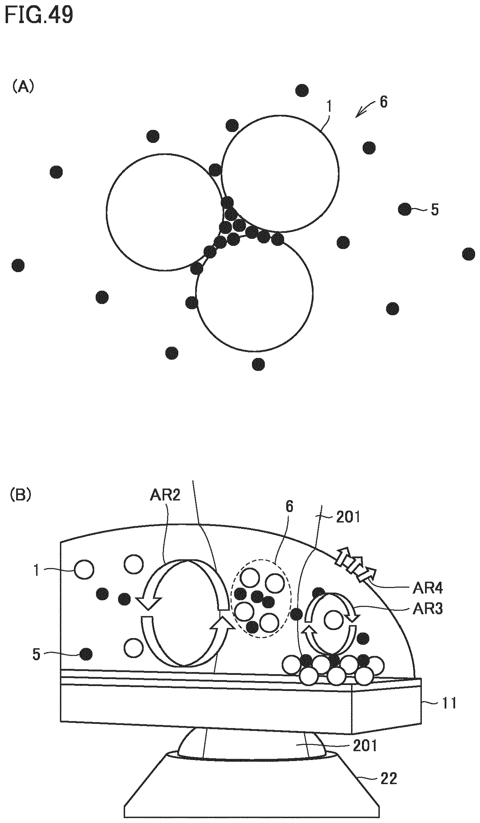

Fig. 49 is a schematic diagram for illustrating a mechanism of formation of assembly 6 in the seventh embodiment. Fig. 49 (A) shows a state in the vicinity of a laser spot of laser beams 201 and Fig. 49 (B) shows a state of the entire liquid.

Referring to Fig. 49 (A), beads 1 are captured at a position of the laser spot as a result of irradiation with laser beams 201. Assembly 6 grows, with captured beads 1 serving as "nucleus" so to speak.

A factor for growth of assembly 6 may be beads 1 being hydrophobic particles. Hydrophobic particles are smaller in surface area of contact with water while the hydrophobic particles are in contact with each other in water than while the hydrophobic particles are dispersed in water, and hence they are stable. Therefore, since beads 1 which have once come into contact with assembly 6 are less likely to move away from assembly 6, assembly 6 tends to grow.

As a particle size is smaller, it becomes more difficult to capture particles with the use of light-induced force. Beads 1 have a particle size of 1.0 $\mu$m, whereas gold nanoparticles 5 have a particle size of 30 nm. Therefore, gold nanoparticles 5 are more difficult to capture than beads 1. According to the present embodiment, however, gold nanoparticles 5 enter a gap in assembly 6 formed from captured beads 1. Gold nanoparticles 5 can thus be captured in a stable manner.

Gold nanoparticles 5 thus captured generate heat as a result of the photothermal effect, so that a liquid at the laser spot and around the same is heated. The photothermal effect of assembly 6 will be described in further detail. Polarization is produced as a result of oscillation of free electrons at the surface of each of the plurality of gold nanoparticles 5 by laser beams 201. Since the plurality of gold nanoparticles 5 are proximate to each other down to a scale not longer than a wavelength (1064 nm) of laser beams 201, a peak wavelength of an absorption spectrum of localized surface plasmon resonance is shifted. Therefore, even laser beams 201 in a wavelength region not in resonance with localized surface plasmon of single gold nanoparticle 5 can highly efficiently be absorbed. Since strong absorption of laser beams 201 is consequently achieved, heat can highly efficiently be generated in assembly 6. With highly efficient conversion of laser beams 201 into heat by assembly 6, a surrounding liquid is locally heated. Since a temperature difference (or a temperature gradient) is thus produced in a liquid, convection is produced in a direction toward assembly 6 owing to the temperature difference.

Referring to Fig. 49 (B), as shown with arrows AR2 and AR3, convection is once directed to assembly 6 and thereafter moves away from assembly 6. Therefore, a convection stagnation region is produced in the vicinity of assembly 6. As beads 1 and gold nanoparticles 5 move over convection toward the stagnation region, growth of assembly 6 is promoted. Assembly 6 most of which volume is occupied by beads 1 thus functions also as a micrometer-order fluid stopper which stops convection controllable by irradiation with light.

In the present embodiment, a portion in the vicinity of the gas-liquid interface is irradiated with laser beams 201. A temperature of a liquid tends to be non-uniform between a region on a side of a bulk relative to the laser spot and a region on a side of the gas-liquid interface relative to the laser spot, due to spatial asymmetry derived from a surface shape of a droplet. Therefore, convection is likely.

Since a portion in the vicinity of the gas-liquid interface is heated, a liquid is more likely to evaporate from the gas-liquid interface than when the bulk is heated (see an arrow AR4). With evaporation of the liquid from the droplet, the liquid in the droplet flows. With such an effect of flow, beads 1 and gold nanoparticles 5 move toward assembly 6.

<Influence of Concentration of Gold Nanoparticles on Structure of Assembly>

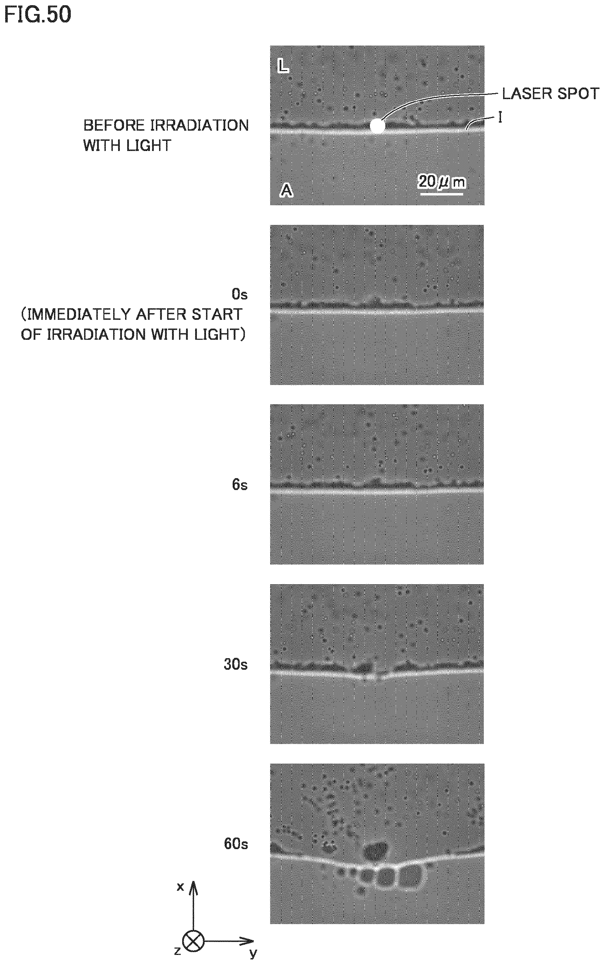

Fig. 50 shows successive photographs showing change over time of a portion in the vicinity of a laser spot resulting from irradiation with light in the tenth sample. Referring to Fig. 50, in the tenth sample, as in the eighth sample (see Fig. 46), growth of assembly 6 over time after start of irradiation with light is observed. It can be seen that a size of assembly 6 (hereinafter simply denoted as an "assembly size") in the tenth sample at each time is smaller than a size of an assembly in the eighth sample.

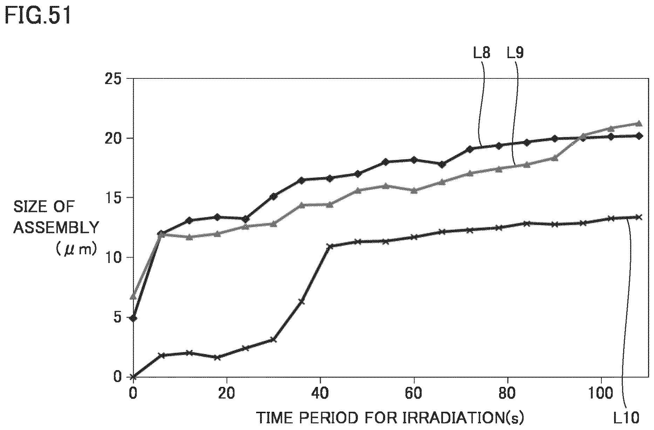

Fig. 51 is a diagram for comparison of change over time in size of an assembly after start of irradiation with light, among the eighth to tenth samples. In Fig. 10, the abscissa represents lapse of time (a time period for irradiation) since start of irradiation with light. The ordinate represents a size of an assembly. Curves L8 to L10 represent change over time in size of assemblies in the eighth to tenth samples, respectively. A size of an assembly is calculated from a diameter of a circumcircle (not shown) of assembly 6 in the successive photographs (see Figs. 46 and 50).

It is confirmed with reference to Fig. 51 that assembly 6 grows and increases in size over time. It can be seen that assembly 6 increases in size in a shorter period of time as a concentration of gold nanoparticles is high.

As shown in Fig. 46, convection is observed immediately after start of irradiation with light in the eighth sample. This is also the case in the ninth sample, although it is not shown. This may be because, in the eighth and ninth samples, an amount of assembly of gold nanoparticles 5 in assembly 6 formed at the laser spot is large to some extent even immediately after irradiation with light, and hence heat sufficient for producing convection is generated.

In the tenth sample, convection is hardly observed immediately after start of irradiation with light, and convection is observed to become stronger after lapse of approximately 40 seconds since start of irradiation with light. This may be because a concentration of gold nanoparticles in the tenth sample is lower than concentrations of gold nanoparticles in the eighth and ninth samples and hence an amount of heat generation from gold nanoparticles 5 in assembly 6 is relatively small immediately after start of irradiation with light. With continued irradiation with light, an amount of assembly of gold nanoparticles 5 increases over time. When an amount of assembly of gold nanoparticles 5 reaches a certain value, a sufficient amount of heat generation is obtained and convection is produced.

It can be seen that, when assembly 6 grows to a size to some extent (approximately 12 $\mu$m in the example shown in Fig. 51), growth of assembly 6 slows down as compared with an initial stage of formation of assembly 6, which may be because of two reasons below.

Firstly, since a system is close to a thermal equilibrium state with continued irradiation with light, a temperature of assembly 6 is close to a temperature of a dispersion medium around assembly 6. Therefore, since a temperature difference in the liquid becomes relatively small, convection is less likely. Consequently, a size of assembly 6 is saturated at a certain size.

Secondly, since an amount of heat generation per unit time is greater with a greater amount of gold nanoparticles 5 in a liquid, assembly 6 rapidly grows. Since a sufficient amount of gold nanoparticles 5 is contained in the liquid in any of the eighth to tenth samples, growth of assembly 6 is less likely to slow down. In the tenth sample, however, an amount of gold nanoparticles 5 is smaller than in the eighth and ninth samples, and hence formation of assembly 6 takes time and a size of assembly 6 is saturated with the size remaining small.

In the present embodiment, a portion in the vicinity of the gas-liquid interface (a region at the edge of the droplet) is irradiated with laser beams 201. Gold nanoparticles 5 tend to gather in the vicinity of the gas-liquid interface even before start of irradiation with light, which will be described below based on a result of measurement of extinction spectra in the eighth and eleventh samples before irradiation with light. A concentration of beads 1 is common between the eighth and eleventh samples. A concentration of gold nanoparticles in the eleventh sample is one tenth of a concentration of gold nanoparticles in the eighth sample (see Fig. 45).

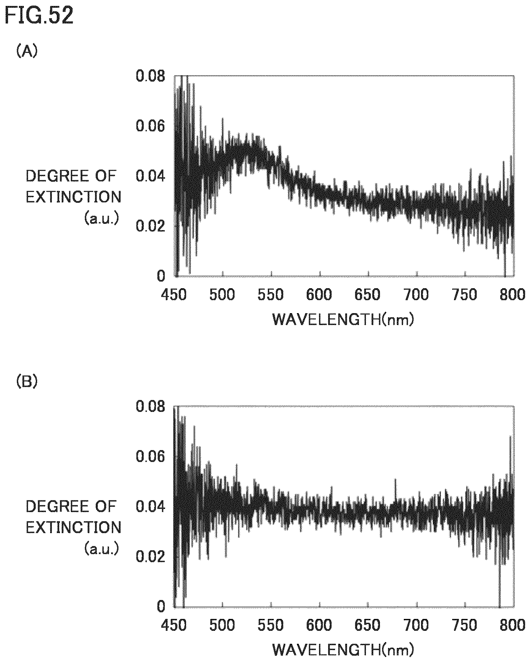

Fig. 52 is a diagram showing extinction spectra in the vicinity of the gas-liquid interface before start of irradiation with light, of the eighths and eleventh samples. Fig. 52 (A) shows an extinction spectrum of the eighth sample and Fig. 52 (B) shows an extinction spectrum of the eleventh sample. The abscissa represents a wavelength and the ordinate represents a degree of extinction.

Referring to Fig. 52 (A) and (B), a peak is observed around 530 nm in the eighth sample, whereas such a peak is not observed in the eleventh sample. It can be seen based on this result that gold nanoparticles 5 are present at high density in the vicinity of the gas-liquid interface even before start of irradiation with light, when a concentration of the gold nanoparticles is relatively high. Therefore, when a portion in the vicinity of the gas-liquid interface is irradiated with laser beams 201, an amount of gold nanoparticles 5 which enter a gap in assembly 6 formed from beads 1 is greater than when the bulk is irradiated with laser beams 201. Since an amount of heat generation from gold nanoparticles 5 is thus increased, a temperature difference can be produced in the liquid in a shorter period of time and convection can be produced.

As set forth above, according to the seventh embodiment, beads 1 and gold nanoparticles 5 can be assembled by making use of the photothermal effect of gold nanoparticles 5. In the present embodiment, it is not necessary to form thin film 12 on cover glass 11 through vapor deposition or sputtering, and hence a more simplified assembling method can be realized.

In the present embodiment, any one of gold nanoparticles 5 and beads 1 corresponds to the "first particles" according to the present invention and the other of them corresponds to the "second particles" according to the present invention. Gold nanoparticles 5 correspond to the "photothermal conversion particles" according to the present invention. Gold nanoparticles 5 and beads 1 are different from each other, for example, in mechanical characteristics (such as a size, a shape, or a density), optical characteristics (such as a resonance absorption wavelength), thermal characteristics (such as a specific heat, a thermal conductivity, a coefficient of thermal expansion, or resistance to heat), and affinity to water (a degree of hydrophilicity or hydrophobicity).

In the present embodiment, an example in which a sample containing metallic nanoparticles as photothermal conversion particles and resin beads greater in particle size than the photothermal conversion particles is employed has been described. Relation between the particle size of the photothermal conversion particles and the particle size of the resin beads may be reverse. By way of example, a sample containing carbon-based particles or organic particles (for example, particles having a diameter of approximately several hundred nm) as the photothermal conversion particles and resin beads smaller in particle size than the photothermal conversion particles (for example, polystyrene beads having a diameter of several ten nm) may be prepared. When the photothermal conversion particles are relatively greater, the photothermal conversion particles are preferably particles not intensely reflecting light.

When such a sample is irradiated with laser beams, photothermal conversion particles relatively greater in size are optically trapped at a position of a laser spot and convection is produced as a result of the photothermal effect of the photothermal conversion particles. The resin beads move over convection toward the optically trapped photothermal conversion particles and captured in a gap between the photothermal conversion particles. Thus, the photothermal conversion particles function as a heat source bringing about the photothermal effect and also functions as a fluid stopper from the nanometer order to the micrometer order.

For example, a combination of two types of metallic nanoparticles different from each other in particle size or a combination between metallic nanoparticles and carbon-based particles (such as carbon black) may be adopted as the "first and second particles" according to the present invention. In this case, both of the particles correspond to the "photothermal conversion particles" according to the present invention.

When a content and a concentration of particles other than photothermal conversion particles (a light absorptive microscopic object) in a sample are the same, a size of a finally formed assembly will be different depending of a content of the photothermal conversion particles. Therefore, by carrying out irradiation with light for a certain period of time under the same condition with a content and a concentration of particles other than the photothermal conversion particles being determined in advance, a content of the photothermal conversion particles can be estimated (or whether or not photothermal conversion particles are contained can be detected) based on a size of the assembly.

[Modification of Seventh Embodiment]

An example in which a sample containing beads 1 having a diameter of 1.0 $\mu$m and gold nanoparticles 5 is employed has been described in the seventh embodiment. In a modification of the seventh embodiment, a sample containing beads 2 having a diameter of 0.2 $\mu$m and gold nanoparticles 5 (a twelfth sample) is adopted.

A concentration of beads 2 is set to $1.42 \times 10^{11}$ (counts/mL). A concentration of gold nanoparticles is set to $3.19 \times 10^{11}$ (counts/mL). Since an assembling apparatus employed in the modification is similar to assembling apparatus 500 (see Fig. 43), description will not be repeated. Power of laser beams 201 emitted from photothermal light source 20 is 0.8 W. Power of laser beams 201 after passage through objective lens 22 is 0.16 W. A 100x lens is employed as objective lens 22.

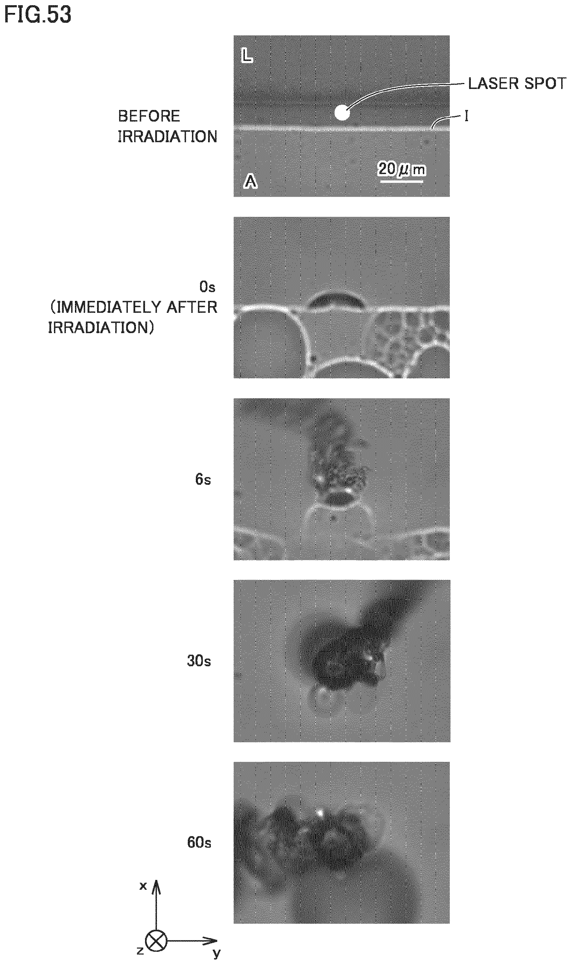

Fig. 53 shows successive photographs showing change over time of a portion in the vicinity of a laser spot resulting from irradiation with light in the twelfth sample. Referring to Fig. 53, production of a substance from a laser spot as if it were springing as a result of irradiation with light is observed. This substance is a melt of beads 2 owing to heat generation resulting from irradiation with light.

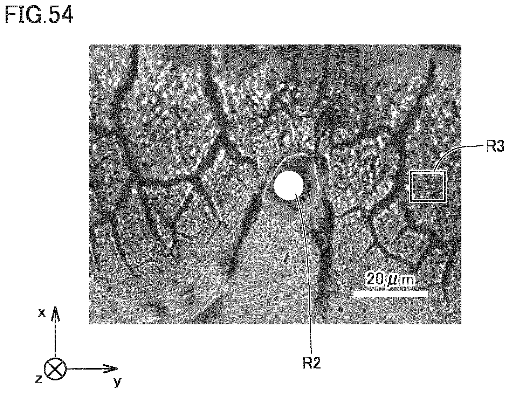

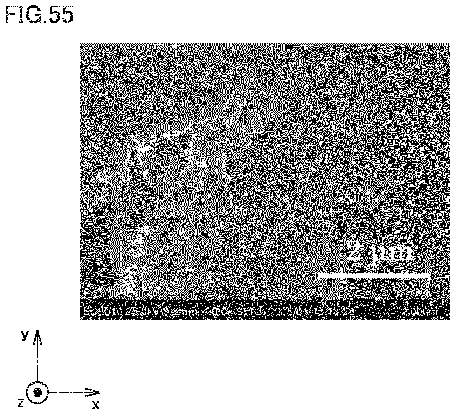

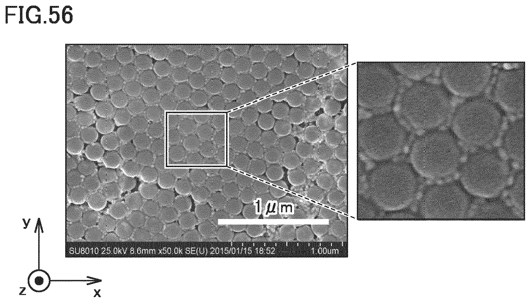

Fig. 54 shows an optical photograph of assembly 6 formed in the twelfth sample which is naturally dried after irradiation with light. Fig. 55 shows an SEM image of a region of a laser spot (a region R2 shown in Fig. 53) in the twelfth sample. Fig. 56 shows an SEM photograph of a region (a region R3 shown in Fig. 53) distant from the laser spot in the twelfth sample. A time period for irradiation with laser beams is set to 110 seconds.

Referring to Fig. 55, it is observed that some of a plurality of beads 2 are molten and integrated in region R2 of the laser spot. It can be seen that a temperature of assembly 6 is higher than 240°C at the time of irradiation with light, because polystyrene beads employed as beads 2 have a melting point of 240°C. It is expected that gold nanoparticles 5 are not molten because gold nanoparticles 5 have a melting point not lower than 1000°C.

Referring to Fig. 56, a hexagonal closest packing structure is formed by beads 2 in region R3 distant from the laser spot. As shown in an enlarged view on the right, it can be seen that gold nanoparticles 5 enter a gap between adjacent beads 2.

As set forth above, according to the present modification, beads 2 are assembled over convection originating from heat generation from gold nanoparticles 5 and assembled beads 2 are increased in temperature and molten. According to such an assembly technique, for example, by preparing a small die (what is called a mold) and assembling beads 2 in the die, beads 2 can be molded into a shape of the die. A micromolding process can be realized.

[Eighth Embodiment]

Cells have been known to have a function for selective bonding as a result of molecular recognition of a substance present around the cells at surfaces thereof or for uptake of a substance present around the cells into themselves (an uptake function). In an eighth embodiment, instead of resin beads, cells and a substance which can be introduced into cells (an introduction target substance) owing to the uptake function of the cells are applied. Examples of the uptake function include an internalizing function such as endocytosis by cells, passing of ions via membrane channel protein (channel protein located across a cell membrane), or direct permeability through a cell membrane.

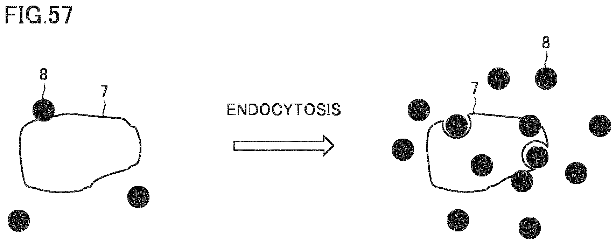

Fig. 57 is a conceptual diagram for illustrating introduction of peptides 8 as a result of endocytosis by cells 7. Referring to Fig. 57, when a sugar chain (not shown) at a surface of cells 7 and a type of a peptide present around cells 7 correspond to each other, the peptide is taken into the cells owing to endocytosis. In general, when a concentration of peptides 8 in a liquid (hereinafter simply also denoted as a "peptide concentration") is uniform, a peptide concentration not lower than 10 $\mu$M is required to cause endocytosis. With a higher peptide concentration, an amount of introduction of peptides 8 into cells 7 increases. When cells 7 and peptides 8 are assembled through irradiation with light, a peptide concentration around cells 7 is locally high. Introduction of peptides 8 into cells 7 through endocytosis can thus be promoted.

An apparatus for introducing peptides according to the eighth embodiment is different in construction from assembling apparatus 100 shown in Fig. 1 in including a substrate 106 instead of substrate 10. Since a construction of the apparatus for introducing peptides is otherwise similar to the corresponding construction of assembling apparatus 100, detailed description will not be repeated.

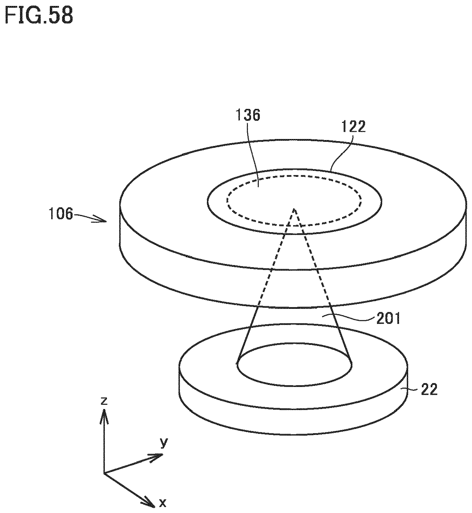

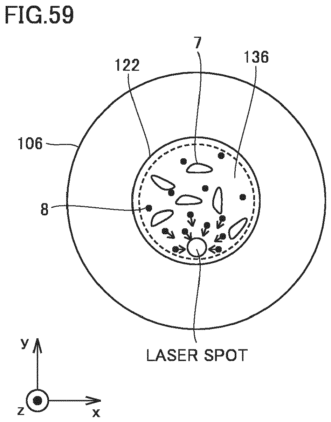

Fig. 58 is an enlarged perspective view of a construction around substrate 106. Fig. 59 is a top view of substrate 106.

Referring to Figs. 58 and 59, for example, a glass bottom dish can be employed as substrate 106. A membrane (hereinafter also referred to as a "self-assembled membrane") 122 is formed through self-assembly of gold nanoparticles 5 on a bottom surface of substrate 106, instead of a thin gold film formed through sputtering. A known technique can be adopted as a technique for forming self-assembled membrane 122. In the present embodiment, a self-assembled membrane is formed specifically in a procedure below. A dispersion liquid of gold nanoparticles 5 at a concentration of $6.38 \times 10^{11}$ (counts/mL) is prepared. After 15 $\mu$L of this dispersion liquid is dropped onto the bottom surface of substrate 106, the dispersion liquid is vacuum dried.

A 10x lens is employed as objective lens 22. Self-assembled membrane 122 of gold nanoparticles 5 is irradiated with laser beams 201. Self-assembled membrane 122 is varied in density. In the present embodiment, a position of a laser spot is adjusted to a portion in self-assembled membrane 122 where gold nanoparticles 5 are at high density. Power of laser beams 201 emitted from photothermal light source 20 is 0.1W. Power of laser beams 201 after passage through objective lens 22 is 0.068 W. A laser spot has a diameter of approximately 10 $\mu$m.

Substrate 106 holds a sample 136. Sample 136 contains cells 7 and peptides 8. In the present embodiment, HeLa cells derived from human cervical cancer are employed as cells 7.

A technique for preparing sample 136 will be described. $\alpha$-MEM (+) is employed as a culture medium for culture of cells 7. $\alpha$-MEM (+) is obtained by adding fetal bovine serum (10% FBS) (Gibco manufactured by Life Technologies Corporation) to $\alpha$-MEM (-).

In general, in culture of HeLa cells, HeLa cells in a state floating in the culture medium (a floating state) are transferred to a culture dish (not shown) and thereafter cultured in a state adhering to a bottom surface of the culture dish (an adhering state). Therefore, in order to irradiate substrate 106 with light with many of cells 7 again being in a floating state, cells 7 should be peeled off and recovered from the culture dish. In many cases, all cells 7 are not necessarily peeled off from the culture dish, and cells in the floating state (hereinafter also referred to as "floating cells") and cells in the adhering state (hereinafter also referred to as "adhering cells") are present as being mixed. In the present embodiment, initially, cells 7 in the culture dish are washed with Dulbecco's phosphate buffered saline (D-PBS) (manufactured by Nacalai Tesque, Inc.).

Furthermore, a trypsin solution (a mixture solution of 0.5 g/L of trypsin and 0.53 mM of ethylenediaminetetraacetic acid (EDTA)) is added to the culture dish. Thereafter, the culture dish is rested for 5 minutes in an incubator set to 37°C, so that HeLa cells are peeled off from the bottom surface of the culture dish.

Then, a solution obtained by diluting a solution containing an antibiotic (a penicillin-streptomycin solution) (manufactured by Sigma-Aldrich Co. LLC.) with α-MEM (+) at a dilution factor of 50 is used to recover cells 7 in the culture dish. The antibiotic is added for prevention of contamination.

A culture medium containing cells 7 thus recovered from the culture dish is dropped onto substrate 106. Sample 136 contains approximately 50,000 cells 7. This numeric value is found by counting the number of cells with a counting chamber. Cells 7 are cultured by resting substrate 106 in an incubator at 37°C overnight.

Thereafter, prior to irradiation with light, the culture medium is replaced from α-MEM (+) to α-MEM (-). When peptide α-MEM (+) is employed, fetal bovine serum contained therein inhibits introduction of peptides 8 into cells 7. Peptides 8 are added to the culture medium.

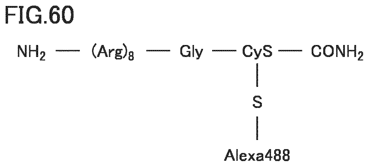

Fig. 60 is a diagram showing peptide 8 employed in the eighth embodiment. Referring to Fig. 60, peptide 8 is peptide octa-arginine. Arginine peptide has been known to be introduced into cells mainly by endocytosis (more specifically, macropinocytosis).

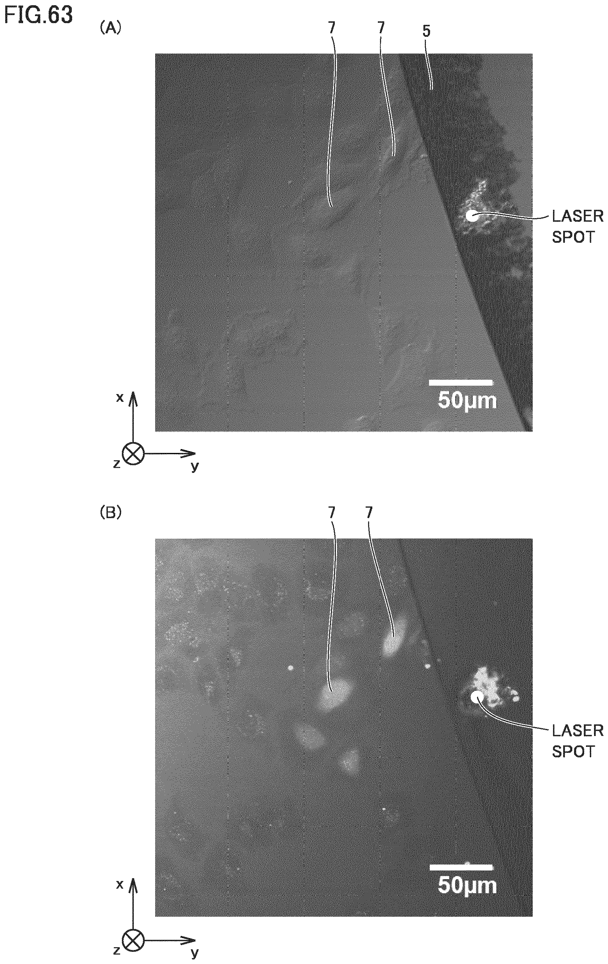

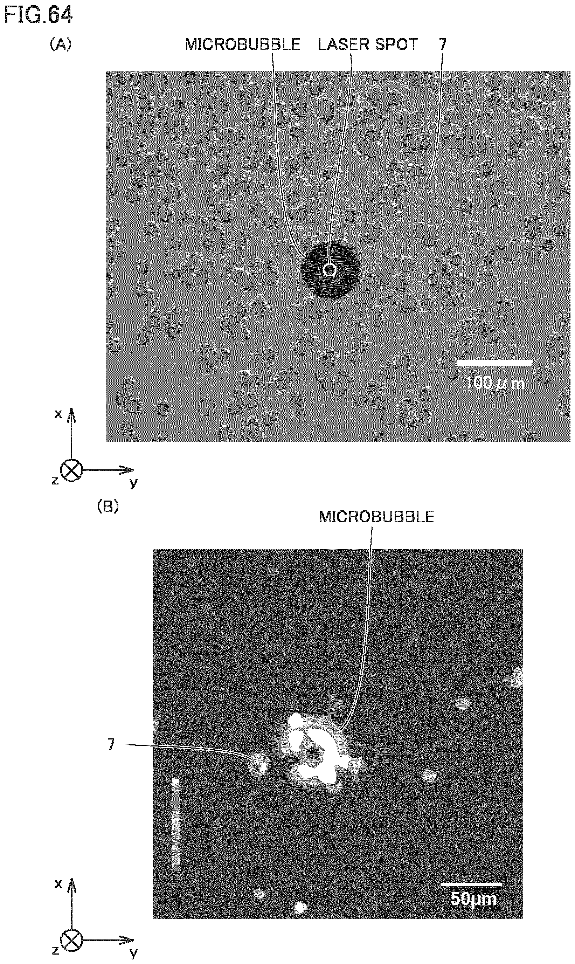

A cysteine residue (shown as Cys) is bonded to a C terminal of peptide 8. A fluorochrome is bonded to a cysteine lateral chain. Alexa 488 C5-maleimide (Invitrogen manufactured by Life Technologies Corporation) is employed as the fluorochrome. The fluorochrome has an excitation wavelength of 488 nm and a maximum fluorescence wavelength of 519 nm. Bonding of a fluorochrome to peptide 8 is for measurement of fluorescence (see Figs. 63 and 64) which will be described later and not essential.

In the present embodiment, a thirteenth sample is prepared as sample 136. A peptide concentration in the thirteenth sample is 1 μM, which is lower than a peptide concentration (not lower than 10 μM) at which endocytosis is generally tends to occur.

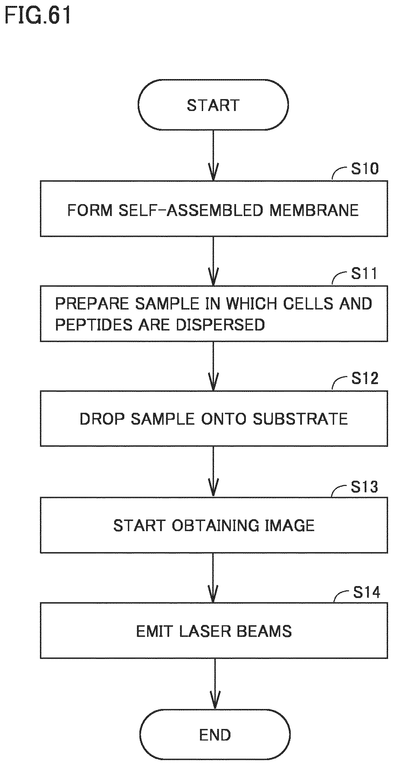

Fig. 61 is a flowchart for illustrating a method of introducing peptides 8 according to the eighth embodiment of the present invention. Referring to Fig. 61, a method of introducing peptides further includes step S10 of forming a self-assembled membrane of gold nanoparticles 5 prior to step S11 of dispersing cells 7 and peptides 8 in sample 136. The method of introducing peptides does not have to include step S15 of drying sample 136. An operation is otherwise similar to the corresponding operation in the flowchart shown in Fig. 4.

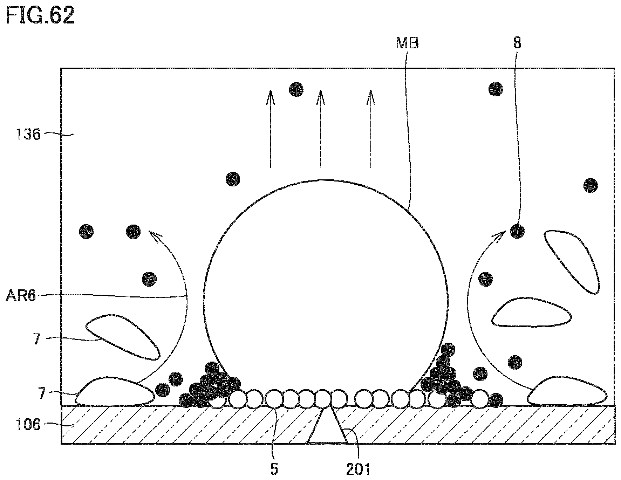

Fig. 62 is a schematic diagram for illustrating a mechanism of assembly of peptides 8 in the eighth embodiment. The present embodiment is different from the first embodiment in use of a self-assembled membrane instead of a thin gold film and use of cells 7 and peptides 8 instead of respective beads 1 and 2. The assembly mechanism in the present embodiment is basically similar to the mechanism (see Fig. 6) described in the first embodiment.

Referring to Fig. 62, when the self-assembled membrane of gold nanoparticles 5 is irradiated with laser beams 201, a liquid around the self-assembled membrane (a culture medium) is locally heated as a result of the photothermal effect of gold nanoparticles 5. With vaporization of the culture medium or thermal expansion of a gas (such as air) dissolved in the culture medium, microbubble MB is produced. Furthermore, since a temperature difference is produced in the culture medium through heating, convection (shown with an arrow AR6) is produced. Since floating cells of cells 7 and peptides 8 are carried over convection toward microbubble MB, a concentration of peptides 8 is relatively high in the vicinity of the laser spot. Consequently, endocytosis into cells 7 in the vicinity of the laser spot can be promoted.

As described above, the fluorochrome is bonded to each of peptides 8. Therefore, a distribution of peptides in sample 136 can be determined by determining fluorescence emitted from the fluorochrome. In the present embodiment, a transmission image and a fluorescent image of each sample are obtained with the use of a confocal microscope.

Fig. 63 shows a confocal micrograph after the thirteenth sample is irradiated with light. Fig. 63 (A) shows a transmission image and Fig. 63 (B) shows a fluorescent image.

As shown in the transmission image in Fig. 63 (A), each cell 7 is in a shape of a substantially oval sphere. A boundary between a nucleus and a cytoplasm is not clear in a living cell, whereas shrinkage of a nucleus is clearly observed in a killed cell. Therefore, a living cell and a killed cell can be distinguished from each other. It can be seen in Fig. 62(A) that most of cells 7 are living even after irradiation with light.

Referring next to Fig. 63 (B), a region brightly shown in the figure results from fluorescence emitted from the fluorochrome. Fluorescence from the inside of cells 7 is observed. Therefore, it can be seen that peptides 8 are introduced into cells 7 by endocytosis. More specifically, fluorescence is observed in the cytoplasm and nuclei in cells 7. Therefore, it is expected that peptide 8 escaped from endosome before fusion with lysosome in a process of endocytosis and reached the cytoplasm and the nucleus.

Fluorescence from cells 7 is brighter toward the laser spot. It can thus been seen that peptides 8 are assembled at higher density toward the laser spot. Since brightness is highest at the position of the laser spot, an amount of assembly of peptides 8 is considered to be largest at the position of the laser spot.

Thus, according to the present embodiment, endocytosis can positively be caused even at a low peptide concentration (lower than 10 μM) at which endocytosis is generally less likely. Endocytosis is confirmed to efficiently occur even when a peptide concentration is 50 nM, although it is not shown. This may be because, as a result of assembly of peptides 8 in a region in the vicinity of the laser spot owing to convection caused by irradiation with light as described with reference to Fig. 62, a peptide concentration around cells 7 is locally higher than 10 μM.

As described above, some of cells 7 adhere to the bottom surface of substrate 106 and the remainder floats in the culture medium (see Fig. 62). When the laser spot is equidistant from cells 7, floating cells are observed more brightly than adhering cells. An amount of introduction of peptides 8 into the floating cells is greater than an amount of introduction of peptides 8 in the adhering cells, for reasons below.

The floating cells move through a liquid together with peptides 8, over convection caused by irradiation with light. The adhering cells are fixed to the bottom surface of substrate 106 even when convection is produced. Therefore, a relative speed between the floating cells and peptides 8 is lower than a relative speed between the adhering cells and peptides 8. Therefore, since a period during which peptides 8 are present around the floating cells is relatively longer, an amount of introduction of peptides 8 is larger.

In order to have peptides 8 assembled in the present embodiment, power of laser beams 201 should be set to be high to some extent. When power of laser beams 201 is set to excessively be high, however, cells 7 will be killed in heat generation and endocytosis does not occur. Therefore, in order to promote endocytosis with irradiation with light, power of laser beams 201 is preferably appropriately set so as to suppress excessive heat generation while assembly of peptides 8 is being allowed.

From another point of view, in the present embodiment, a position of a laser spot is adjusted to a portion in a self-assembled membrane where gold nanoparticles 5 are at high density. Therefore, an amount of heat generation is larger than in irradiation with laser beams 201, of a portion where gold nanoparticles 5 are at low density. Thus, an amount of heat generation may vary depending on a position of a laser spot. Therefore, a position of a laser spot (in other words, a thickness of a self-assembled membrane at a position of a laser spot) is preferably determined based on power of laser and light absorbency of gold nanoparticles 5.

In the eighth embodiment, cells (more specifically, floating cells) and peptides 8 correspond to the "microscopic object" according to the present invention. Cells 7 and peptides 8 are different from each other in mechanical characteristics (such as a size, a shape, an internal structure, or a density). Peptides 8 correspond to an "introduction target substance" according to the present invention.

Though a construction for promoting introduction of peptides into cells by endocytosis has been described in the present embodiment, a function which can be promoted by assembly of an introduction target substance is not limited to introduction into cells. In a stage preceding endocytosis, selective bonding of an introduction target substance to a surface of cells based on molecular recognition takes place. There may be also a case, however, where selective bonding merely takes place and an introduction target substance is not taken into cells. According to the present embodiment, a substance which can bring about interaction for molecular recognition with a surface of a cell membrane can also be assembled on a surface of the cell membrane, so that molecular recognition (or selective bonding) at the surface of the cells is promoted.

[Modification of Eighth Embodiment]

An example in which floating cells and adhering cells are present as being mixed in a culture medium has been described in the eighth embodiment. In the present modification, an example in which most of cells 7 in a culture medium are floating cells will be described.

In a fourteenth sample employed in the modification, a thin gold film is formed on a substrate (a not-shown glass bottom dish) through sputtering instead of a self-assembled membrane of gold nanoparticles 5. Since a technique for forming a thin gold film is similar to the technique in the first embodiment, description will not be repeated. The thin gold film has a thickness of 10 nm. Power of laser beams 201 output from photothermal light source 20 is 0.1 W.

A technique for recovering cells 7 cultured in a culture dish is substantially similar to the technique in the eighth embodiment. In the present modification, however, laser beams are emitted immediately after drop of a culture medium containing cells 7 recovered from the culture dish onto substrate 106. Almost all cells 7 can thus be floating cells. A peptide concentration in the fourteenth sample is 1 $\mu$M.

Fig. 64 shows an optical photograph and a fluorescent image in the vicinity of a laser spot resulting from irradiation with light in the fourteenth sample. Fig. 64 (A) shows an optical photograph. Fig. 64 (B) shows a fluorescent image obtained with a confocal microscope.

Referring to Fig. 64 (A), it is confirmed that microbubble MB is produced at a position of a laser spot. Many cells 7 are present around microbubble MB

Referring to Fig. 64 (B), a peptide concentration at a position of microbubble MB is higher than a peptide concentration around microbubble MB It is thus confirmed that peptides 8 are assembled as a result of irradiation with light. Furthermore, fluorescence is observed also from the inside of cells 7 (floating cells) in the vicinity of microbubble MB Therefore, it can be seen that peptides 8 are introduced into cells 7.

When power of laser beams 201 is set to excessively be high, cells 7 may be damaged by heat generation. It is confirmed, however, that cells 7 around microbubble MB are in a cell 7 living state even 1 day to 2 days after irradiation with light. Therefore, according to the present modification, it can be seen that peptides 8 can be introduced into cells 7 without damaging cells 7.

It should be understood that the embodiments disclosed herein are illustrative and non-restrictive in every respect. The scope of the present invention is defined by the terms of the claims, rather than the description above, and is intended to include any modifications within the scope and meaning equivalent to the terms of the claims.

INDUSTRIAL APPLICABILITY

The present invention can be made use of as an assembling apparatus which assembles a microscopic object. For example, bacteria (ultraoligotrophic bacteria) useful for humans can be assembled.

The present invention can be made use of as an apparatus for manufacturing a microscopic object assembly structure which manufactures a microstructure. Thus, for example, a two-component photonic structure is manufactured.

The present invention can mold a microscopic object in a shape of a die by preparing a small die and assembling a microscopic object in the die. Thus, a micromolding process can be realized.

The present invention can estimate a content (or a concentration) of a light absorptive microscopic object (a detection target substance) contained in a sample by making use of a difference in size of a finally formed assembly depending on a content of a light absorptive microscopic object (photothermal conversion particles).

The present invention can be made use of as an apparatus for detecting a microscopic object. For example, it can detect a mass concentration of PM (such as PM 2.5) in atmosphere. Since PM 2.5 and PM 10 are separated in accordance with a size, a separation mechanism does not have to separately be provided. Therefore, a detection apparatus can be reduced in size.

The present invention can be realized as an apparatus for removing a microorganism harmful for a human body, because a microorganism can be assembled and the assembled microorganism can be removed through a high-temperature treatment making use of the photothermal effect.

The present invention can separate a separation target substance from among a plurality of microscopic objects. For example, when the present invention is applied to flow cytometry, sensitivity in measurement can be improved by assembling cells representing a separation target substance at high density.

The present invention can promote introduction of an introduction target substance into cells and assembly of an introduction target substance in a cell membrane. For example, in research and development of a new drug, influence of the new drug on cells (or a tissue constituted of the cells) may be evaluated in a stage preceding clinical studies. In such a case, according to the present invention, an amount of a new drug required for evaluation can be reduced.

REFERENCE SIGNS LIST

1, 2 beads; 1A, 1B, 1C, 7 cell; 3 bacteria; 4 microorganism; 5 gold nanoparticle; 6 assembly; 8 peptide; 10, 102, 103, 105, 106 substrate; 11 cover glass; 12 thin film; 122 self-assembled membrane; 13, 135, 136 sample; 14 micro flow path; 20 photothermal light source; 21 dichroic mirror; 22, 422 objective lens; 30 illumination light source; 31 shooting instrument; 40 XYZ-axis stage; 50, 450 control unit; 60 operation unit; 460 analysis portion; 100, 101, 500 assembling apparatus; 200 apparatus for assembling and removing microorganism; 210 collection portion; 211 fan; 212, 311 suction port; 220 removal portion; 300 detection apparatus; 310 air sampler; 312 filter; 313 flowmeter; 314 pump; 400 flow cytometer; 410 flow cell; 411 sheath liquid introduction portion; 412 sample introduction portion; 430 light source; 431 spectroscope; 440 cell sorter; 442, 443 container; and MB microbubble.

**Claims**

1.    An assembling apparatus which assembles a plurality of types of microscopic objects different in at least one of physical characteristics, chemical characteristics, and biological characteristics, the assembling apparatus comprising:

    a holding member configured to hold a space in which the plurality of types of microscopic objects are dispersed; and
    a light source configured to irradiate the holding member or the space with light and produce a temperature difference in the space.

2.    The assembling apparatus according to claim 1, wherein
    the space contains a liquid in which the plurality of types of microscopic objects are dispersible, and
    the holding member includes a substrate configured to hold the liquid in which the plurality of types of microscopic objects are dispersed.

**3.** The assembling apparatus according to claim 2, wherein
the substrate includes a holding region configured to hold the liquid, and
the holding region is formed of a material which converts light from the light source into heat.

**4.** The assembling apparatus according to claim 3, wherein
the light source emits laser beams at a wavelength included in a light absorption region of the material.

**5.** The assembling apparatus according to claim 4, wherein
the holding region has a thickness along a direction of emission of the laser beams, and
the thickness is determined based on intensity of the laser beams and light absorbency of the material.

**6.** The assembling apparatus according to any one of claims 3 to 5, wherein the light source heats the holding region by irradiating the holding region with light so as to produce convection in the vicinity of the holding region.

**7.** The assembling apparatus according to claim 6, wherein
the light source heats the holding region by irradiating the holding region with light so as to further produce a bubble in the vicinity of the holding region.

**8.** The assembling apparatus according to claim 2, wherein
the plurality of types of microscopic objects include a plurality of first particles and a plurality of second particles different in particle size from each other,
at least one of the plurality of first particles and the plurality of second particles includes a plurality of photothermal conversion particles each of which converts light from the light source into heat, and
the light source assembles the plurality of first particles and the plurality of second particles at a position of irradiation with light in the liquid by irradiating the liquid with light so as to have the plurality of photothermal conversion particles generate heat at the position of irradiation, produce the temperature difference and convection in the liquid, and move the plurality of first particles and the plurality of second particles over the convection.

**9.** The assembling apparatus according to any one of claims 2 to 8, further comprising an objective lens which gathers light from the light source and introduces gathered light into the holding member or the liquid.

**10.** The assembling apparatus according to claim 9, further comprising an adjustment mechanism configured to adjust a distance between the objective lens and a gas-liquid interface such that a focal point of the objective lens is located in the liquid and located in the vicinity of the gas-liquid interface, the gas-liquid interface being an interface between a gas around the liquid and the liquid.

**11.** The assembling apparatus according to any one of claims 1 to 10, wherein the plurality of types of microscopic objects include two or more types of microscopic objects different in at least one of a particle size and a shape, the particle size and the shape representing the physical characteristics.

**12.** An assembling method for assembling a plurality of types of microscopic objects different in at least one of physical characteristics, chemical characteristics, and biological characteristics, the assembling method comprising:

dispersing the plurality of types of microscopic objects in a space defined by a holding member; and
irradiating the holding member or the space with light for producing temperature difference in the space.

**13.** The assembling method according to claim 12, wherein
the space contains a liquid in which the plurality of types of microscopic objects are dispersible,
the holding member includes a substrate configured to hold the liquid, and
the dispersing the plurality of types of microscopic objects includes preparing the liquid in which the plurality of types of microscopic objects are dispersed prior to the irradiating the holding member or the space.

**14.** The assembling method according to claim 13, wherein
the substrate includes a holding region in which the liquid is held, and
in the irradiating the holding member or the space, the light with which the holding region is irradiated is converted to heat.

**15.** The assembling method according to claim 14, wherein

in the irradiating the holding member or the space, laser beams are emitted, the laser beams having a wavelength included in a light absorption region of a material for the holding region.

16. The assembling method according to claim 14 or 15, wherein
in the irradiating the holding member or the space, the holding region is heated by irradiation of the holding region with light such that convection is produced in the vicinity of the holding region.

17. The assembling method according to claim 16, wherein
in the irradiating the holding member or the space, the holding region is heated by irradiation of the holding region with light such that a bubble is further produced in the vicinity of the holding region.

18. The assembling method according to claim 16 or 17, the assembling method further comprising vacuum drying the holding region after heating the holding region.

19. The assembling method according to claim 13, wherein
the plurality of types of microscopic objects include a plurality of first particles and a plurality of second particles different in particle size from each other,
at least one of the plurality of first particles and the plurality of second particles includes a plurality of photothermal conversion particles each of which converts light from the light source into heat, and
in the irradiating the holding member or the space,

the plurality of photothermal conversion particles produce convection in the liquid by generating heat at a position of irradiation with light in the liquid so as to produce the temperature difference in the liquid, and
the plurality of first particles and the plurality of second particles are assembled at the position of irradiation by being moved over the convection.

20. The assembling method according to any one of claims 12 to 19, wherein the plurality of types of microscopic objects include two or more types of microscopic objects different in at least one of a particle size and a shape representing the physical characteristics.

21. An apparatus for manufacturing a microscopic object assembly structure, the apparatus comprising the assembling apparatus according to any one of claims 1 to 11.

22. An apparatus for assembling and removing a microorganism, the apparatus comprising the assembling apparatus according to any one of claims 1 to 11,
the plurality of types of microscopic objects including a microorganism, and the light source heating the holding member by irradiating the holding member or the space with light so as to remove the microorganism in the space.

23. A detection apparatus for detecting a detection target substance which is included in a plurality of types of microscopic objects, the detection apparatus comprising:

the assembling apparatus according to any one of claims 1 to 11;
an imaging device configured to obtain an image of the space irradiated with light from the light source; and
a detector configured to detect the detection target substance based on an image obtained by the imaging device.

24. A separation apparatus for separating a separation target substance which is included in a plurality of types of microscopic objects from the plurality of types of microscopic objects, the separation apparatus comprising:

the assembling apparatus according to any one of claims 1 to 11 which assembles the plurality of types of microscopic objects so as to form an assembly;
a spectroscopic light source configured to emit light for measuring a spectrum of the assembly assembled by the assembling apparatus;
a spectroscope configured to measure a spectrum of the assembly; and
a separation portion configured to separate the separation target substance based on a result of measurement of the spectrum by the spectroscope.

25. An apparatus for introducing an introduction target substance, the apparatus comprising the assembling apparatus according to any one of claims 1 to 11,

the plurality of types of microscopic objects including cells and an introduction target substance to be introduced into the cells by an uptake action of the cells, and

the light source promoting introduction of the introduction target substance into the cells by irradiating the holding member or the space with light so as to heat the holding member and assemble the introduction target substance.

26. The apparatus for introducing an introduction target substance according to claim 25, the apparatus further comprising cells adhering to the holding member.

FIG.1

FIG.2

FIG.3

(A)                         (B)                              (C)

FIRST SAMPLE            SECOND SAMPLE                      THIRD SAMPLE

$4.6 \times 10^6$ COUNTS    $3.9 \times 10^8$ COUNTS    $4.6 \times 10^6$ COUNTS    $3.9 \times 10^8$ COUNTS

EP 3 141 300 A1

FIG.4

```
          ┌─────────────┐
          │    START    │
          └──────┬──────┘
                 │
                 ▼              ⌐ S11
    ┌────────────────────────────┐
    │   PREPARE SAMPLE IN WHICH   │
    │   BEADS ARE DISPERSED       │
    └─────────────┬──────────────┘
                  │
                  ▼             ⌐ S12
    ┌────────────────────────────┐
    │   DROP SAMPLE ONTO SUBSTRATE │
    └─────────────┬──────────────┘
                  │
                  ▼             ⌐ S13
    ┌────────────────────────────┐
    │    START OBTAINING IMAGE    │
    └─────────────┬──────────────┘
                  │
                  ▼             ⌐ S14
    ┌────────────────────────────┐
    │      EMIT LASER BEAMS       │
    └─────────────┬──────────────┘
                  │
                  ▼             ⌐ S15
    ┌────────────────────────────┐
    │ DRY SAMPLE AND OBTAIN ASSEMBLY │
    └─────────────┬──────────────┘
                  │
                  ▼
          ┌─────────────┐
          │     END     │
          └─────────────┘
```

FIG.5

MICROBUBBLE
LASER SPOT                    1        2

1s    25μm    5s    10s    15s

20s    25s    30s    35s

x
z⊗→y

FIG.6

# FIG.7

(A)

| FIGURE NUMBER | SAMPLE | THE NUMBER N1 OF BEADS 1 | THE NUMBER N2 OF BEADS 2 | MAGNIFICATION OF OBJECTIVE LENS |
|---|---|---|---|---|
| FIG. 8 | FIRST SAMPLE | $4.6 \times 10^6$ | 0 | $\times 100$ |
| FIG. 9 | SECOND SAMPLE | 0 | $3.9 \times 10^8$ | $\times 100$ |
| FIG. 10 | THIRD SAMPLE | $4.6 \times 10^6$ | $3.9 \times 10^8$ | $\times 100$ |
| FIG. 11 | FIRST SAMPLE | $4.6 \times 10^6$ | 0 | $\times 10$ |
| FIG. 12 | SECOND SAMPLE | 0 | $3.9 \times 10^8$ | $\times 10$ |
| FIG. 13 | THIRD SAMPLE | $4.6 \times 10^6$ | $3.9 \times 10^8$ | $\times 10$ |

(B)

| FIGURE NUMBER | SAMPLE | RATIO N1:N2 BETWEEN THE NUMBER OF BEADS 1 AND THE NUMBER OF BEADS 2 | MAGNIFICATION OF OBJECTIVE LENS |
|---|---|---|---|
| FIG. 14 | THIRD' SAMPLE | 1 : 43 | $\times 100$ |
| FIG. 15 | THIRD" SAMPLE | 1 : 9 | $\times 100$ |
| FIG. 16 | THIRD' SAMPLE | 1 : 43 | $\times 10$ |
| FIG. 17 | THIRD" SAMPLE | 1 : 9 | $\times 10$ |

## FIG.8

(A)

ONLY BEADS 1
OBJECTIVE LENS : ×100

(B)

# FIG.9

(A)

IXB

IXB

5μm

25μm

ONLY BEADS 2
OBJECTIVE LENS: ×100

(B)

∇CENTER OF LASER SPOT

HEIGHT

2μm

38μm

DISTANCE

## FIG.10

(A)

BEADS 1 + BEADS 2
OBJECTIVE LENS: ×100

(B)

## FIG.11

(A)

XIB

y
z  x

5μm

XIB

50μm

ONLY BEADS 1
OBJECTIVE LENS : ×10

(B)

▽CENTER OF LASER SPOT

HEIGHT

10μm

75μm

DISTANCE

## FIG.12

(A)

XIIB

XIIB

ONLY BEADS 2
OBJECTIVE LENS: ×10

(B)

∇CENTER OF LASER SPOT

HEIGHT

2 μm

100 μm

DISTANCE

# FIG.13

(A)

BEADS 1 + BEADS 2
OBJECTIVE LENS: ×10

(B)

# FIG.14

(A)

SE    03-Mar-14    000000 WD13.1mm 15.0kV x3.0k    10um

(B)

N1:N2=1:43
OBJECTIVE LENS: ×100

# FIG.15

(A)

(B)

SE    26-Feb-14    000000 WD13.1mm 15.0kV x3.0k    10um

N1:N2=1:9
OBJECTIVE LENS: ×100

EP 3 141 300 A1

## FIG.16

(A)

SE    02-Mar-14    000000  WD13. 1mm  15. 0kV  x500    100um

(B)

N1:N2=1:43
OBJECTIVE LENS : ×10

# FIG.17

(A)

SE    26-Feb-14    000000  WD13.2mm  15.0kV  x500    100μm

(B)

N1:N2=1:9
OBJECTIVE LENS: ×10

# FIG.18

101

LASER DISPLACEMENT METER 42

ILLUMINATION LIGHT SOURCE 30

301

28 μm

2 μm

41 ADJUSTMENT MECHANISM

10

40

OBJECTIVE LENS 22

20 PHOTOTHERMAL LIGHT SOURCE

201

21

IMAGING DEVICE 31

50 CONTROL UNIT

z
y
x

FIG.19

(A)

O:POSITION OF LASER SPOT

N1:N2=1:51

(B)

O:POSITION OF LASER SPOT

N1:N2=1:25

(C)

O:POSITION OF LASER SPOT

N1:N2=1:9

FIG.20

FIG.21

# FIG.22

(A)

(B)

FIG.23

# FIG.24

(A) NATURALLY DRYING

(B) VACUUM DRYING

FIG.25

AMOUNT OF
INCREASE
IN DIAMETER
($\mu$m)

TIME PERIOD
FOR DRYING
(min)

FIG.26

(A)

BEADS 1
OBJECTIVE LENS: ×10

(B)

BEADS 1
OBJECTIVE LENS: ×10

FIG.27

(A)

BEADS 2
OBJECTIVE LENS: ×10

(B)

BEADS 2
OBJECTIVE LENS: ×10

FIG.28

(A)

BEADS 1 + BEADS 2
OBJECTIVE LENS : ×10

(B)

BEADS 1 + BEADS 2
OBJECTIVE LENS : ×10

FIG.29

FIG.30

## FIG.31

(A)    BEADS 1 + BEADS 2

(B)    BACTERIA 3 + BEADS 2

# FIG.32

(A)

XXXIIB

XXXIIB

BACTERIA 3 + BEADS 2
OBJECTIVE LENS: × 100

(B)

HEIGHT

▽CENTER OF LASER SPOT

1.1 $\mu$ m

57 $\mu$ m

DISTANCE

## FIG.33

(A)

XXXIIIB

XXXIIIB

10 $\mu$m

50 $\mu$m

y

z · x

BACTERIA 3 + BEADS 2
OBJECTIVE LENS: ×10

(B)

HEIGHT

$\nabla$CENTER OF LASER SPOT

1.5 $\mu$m

95 $\mu$m

DISTANCE

FIG.34

FIG.35

FIG.36

FIG.37

# FIG.38

```
        ┌───────────────┐
        │     START     │
        └───────┬───────┘
                │          ┌─ S31
        ┌───────▼────────────────┐
        │ COLLECT PM IN ATMOSPHERE│
        └───────┬─────────────────┘
                │          ┌─ S32
        ┌───────▼──────────────────────┐
        │ DISPERSE COLLECTED PM IN LIQUID│
        └───────┬────────────────────────┘
                │          ┌─ S33
        ┌───────▼──────────────────────┐
        │ FEED LIQUID INTO MICRO FLOW PATH│
        └───────┬────────────────────────┘
                │          ┌─ S34
        ┌───────▼────────────────┐
        │    EMIT LASER BEAMS     │
        └───────┬─────────────────┘
                │          ┌─ S35
        ┌───────▼────────────────┐
        │      OBTAIN IMAGE       │
        └───────┬─────────────────┘
                │          ┌─ S36
        ┌───────▼────────────────────────────┐
        │ OPERATE MASS CONCENTRATION BASED    │
        │ ON RESULT OF IMAGE PROCESSING       │
        └───────┬─────────────────────────────┘
                │
        ┌───────▼───────┐
        │      END      │
        └───────────────┘
```

FIG.39

FIG.40

```
                    ┌─────────────┐
                    │    START    │
                    └──────┬──────┘
                           │
                           ▼           ⌒S41
            ┌──────────────────────────────┐
            │        PREPARE CELLS          │
            └──────────────┬───────────────┘
                           │
                           ▼           ⌒S42
            ┌──────────────────────────────┐
            │  DISPERSE CELLS IN SAMPLE LIQUID │
            └──────────────┬───────────────┘
                           │
                           ▼           ⌒S43
            ┌──────────────────────────────┐
            │  FEED SAMPLE LIQUID INTO FLOW CELL │
            └──────────────┬───────────────┘
                           │
                           ▼           ⌒S44
            ┌──────────────────────────────┐
            │        EMIT LASER BEAMS        │
            └──────────────┬───────────────┘
                           │
                           ▼           ⌒S45
            ┌──────────────────────────────┐
            │  SPECTROSCOPY WITH SPECTROSCOPE │
            └──────────────┬───────────────┘
                           │
                           ▼           ⌒S46
            ┌──────────────────────────────┐
            │  SEPARATE SEPARATION TARGET    │
            │  SUBSTANCE BASED ON RESULT OF  │
            │  SPECTROSCOPY                  │
            └──────────────┬───────────────┘
                           │
                           ▼
                    ┌─────────────┐
                    │     END     │
                    └─────────────┘
```

FIG.41

DIRECTION OF
POLARIZATION

z

DIRECTION OF
PROPAGATION

y

x

210

1

5

FIG.42

DIRECTION OF
POLARIZATION

DIRECTION OF
PROPAGATION

y

z

x

Ax

1

1

## FIG.43

FIG.44

# FIG.45

| SAMPLE | TYPE OF BEADS | CONCENTRATION OF BEADS [COUNT/mL] | CONCENTRATION OF GOLD NANOPARTICLES [COUNT/mL] |
|---|---|---|---|
| EIGHTH SAMPLE | BEADS 1 | $1.14 \times 10^9$ | $3.19 \times 10^{11}$ |
| NINTH SAMPLE | BEADS 1 | $1.14 \times 10^9$ | $1.59 \times 10^{11}$ |
| TENTH SAMPLE | BEADS 1 | $1.14 \times 10^9$ | $6.38 \times 10^{11}$ |
| ELEVENTH SAMPLE | BEADS 1 | $1.14 \times 10^9$ | $3.19 \times 10^{10}$ |

# FIG.46

BEFORE IRRADIATION WITH LIGHT

0s
(IMMEDIATELY AFTER START OF IRRADIATION WITH LIGHT)

6s

30s

60s

FIG.47

FIG.48

FIG.49

(A)

(B)

FIG.50

BEFORE IRRADIATION
WITH LIGHT

0s
(IMMEDIATELY AFTER START
OF IRRADIATION WITH LIGHT)

6s

30s

60s

FIG.51

FIG.52

(A)

(B)

# FIG.53

BEFORE
IRRADIATION

LASER SPOT

I

20μm

0s
(IMMEDIATELY AFTER
IRRADIATION)

6s

30s

60s

FIG.54

R3

20 μm

x

z ⊗ → y

R2

FIG.55

FIG.56

FIG.57

ENDOCYTOSIS

FIG.58

FIG.59

LASER SPOT

FIG.60

$$\text{NH}_2 \text{—— (Arg)}_8 \text{—— Gly —— CyS —— CONH}_2$$
$$|$$
$$\text{S}$$
$$|$$
$$\text{Alexa488}$$

# FIG.61

```
        START
          |
          v                    S10
  FORM SELF-ASSEMBLED MEMBRANE
          |
          v                    S11
  PREPARE SAMPLE IN WHICH CELLS AND
  PEPTIDES ARE DISPERSED
          |
          v                    S12
  DROP SAMPLE ONTO SUBSTRATE
          |
          v                    S13
  START OBTAINING IMAGE
          |
          v                    S14
  EMIT LASER BEAMS
          |
          v
         END
```

FIG.62

FIG.63 (A)

(B)

# FIG.64

(A)

MICROBUBBLE   LASER SPOT   7

100 μm

x
z ⊗ y

(B)

MICROBUBBLE

7

50μm

x
z ⊗ y

**INTERNATIONAL SEARCH REPORT**

International application No.

PCT/JP2015/063364

| A. CLASSIFICATION OF SUBJECT MATTER |
|---|
| *B01J19/00*(2006.01)i, *C12M1/26*(2006.01)i, *C12M1/34*(2006.01)i, *G01N15/00* (2006.01)i, *G01N15/14*(2006.01)i |

According to International Patent Classification (IPC) or to both national classification and IPC

| B. FIELDS SEARCHED |
|---|
| Minimum documentation searched (classification system followed by classification symbols) |
| B01J10/00-12/02, B01J14/00-19/32, C12M1/00-3/10, G02B19/00-21/00, G02B21/06-21/36, G01N1/00-1/34, G01N15/00-15/14, G01N21/00-21/01, G01N21/17-21/61, G01N35/00-37/00, B81B1/00-7/04, B81C1/00-99/00 |

Documentation searched other than minimum documentation to the extent that such documents are included in the fields searched
  Jitsuyo Shinan Koho          1922–1996   Jitsuyo Shinan Toroku Koho   1996–2015
  Kokai Jitsuyo Shinan Koho    1971–2015   Toroku Jitsuyo Shinan Koho   1994–2015

Electronic data base consulted during the international search (name of data base and, where practicable, search terms used)

| C. DOCUMENTS CONSIDERED TO BE RELEVANT |
|---|

| Category* | Citation of document, with indication, where appropriate, of the relevant passages | Relevant to claim No. |
|---|---|---|
| X<br>Y | JP 2013-254940 A (Osaka Prefecture University), 19 December 2013 (19.12.2013), claim 1; paragraphs [0159] to [0167]; fig. 35 to 40 (Family: none) | 1-2,8,11-13, 19-22,25<br>3-7,9-10, 14-18,23-24, 26 |
| Y | JP 2009-214044 A (Canon Inc.), 24 September 2009 (24.09.2009), paragraph [0028]; fig. 3 to 4 (Family: none) | 3-7,9-10, 14-18,23-24, 26 |
| Y | JP 2009-103624 A (Sony Corp.), 14 May 2009 (14.05.2009), paragraphs [0001], [0023], [0043] to [0045], [0054] to [0055]; fig. 5, 7 (Family: none) | 3-7,9-10, 14-18,23-24, 26 |

[X] Further documents are listed in the continuation of Box C.    [ ] See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>  28 July 2015 (28.07.15) | Date of mailing of the international search report<br>  11 August 2015 (11.08.15) |
|---|---|
| Name and mailing address of the ISA/<br>  Japan Patent Office<br>  3-4-3,Kasumigaseki,Chiyoda-ku,<br>  Tokyo 100-8915,Japan | Authorized officer<br><br>Telephone No. |

Form PCT/ISA/210 (second sheet) (July 2009)

**INTERNATIONAL SEARCH REPORT**

International application No.

PCT/JP2015/063364

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 4-066873 A  (Canon Inc.),<br>03 March 1992 (03.03.1992),<br>claims; page 2, upper left column, lines 8 to 11; page 2, lower left column, line 20 to page 4, lower left column, line 3; fig. 1 to 2<br>& US 5198369 A          & EP 0455125 A2 | 9-10,23-24, 26 |
| Y | JP 2012-013714 A  (Arryx, Inc.),<br>19 January 2012 (19.01.2012),<br>paragraphs [0001], [0024] to [0025]; fig. 2<br>& JP 2008-530985 A      & US 2006/0170912 A1<br>& US 2008/0144037 A1     & WO 2006/083907 A2<br>& EP 1846748 A2          & EP 2166340 A1<br>& EP 2410314 A2          & EP 2801813 A1 | 24,26 |
| A | JP 2009-162558 A  (Yugen Kaisha Bio Device Technology),<br>23 July 2009 (23.07.2009),<br>claims 1 to 13; paragraphs [0029] to [0031]; fig. 1<br>(Family: none) | 1-26 |
| A | JP 2012-130920 A  (Monash University),<br>12 July 2012 (12.07.2012),<br>claims 1 to 13; paragraphs [0001] to [0002]; fig. 1 to 5<br>& JP 2009-535203 A       & US 2009/0206171 A1<br>& WO 2007/128046 A1      & EP 2013604 A1 | 1-26 |
| A | JP 2006-000738 A  (Ricoh Co., Ltd.),<br>05 January 2006 (05.01.2006),<br>claims 1 to 6; paragraph [0001]; fig. 1 to 9<br>(Family: none) | 1-26 |
| A | JP 8-280375 A  (Moritex Corp.),<br>29 October 1996 (29.10.1996),<br>claims 1 to 2; paragraphs [0001] to [0010]; fig. 1 to 2<br>(Family: none) | 1-26 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006104048 A

**Non-patent literature cited in the description**

- **T. IIDA ; H. ISHIHARA.** *Phys. Rev. B,* 2008, vol. 77, 245319